# EUROPÄISCHE PATENTSCHRIFT

(11) **EP 2 296 473 B1**
(45) Veröffentlichungstag und Bekanntmachung des Hinweises auf die Patenterteilung: **11.07.2012**
(21) Anmeldenummer: 09761369.9
(22) Anmeldetag: 15.05.2009
(51) Int. Cl.: A01N 43/56, C07C 317/14, C07D 231/20

(54) **HERBIZID WIRKSAME 4- (3-ALKYLSULFINYLBENZOYL) PYRAZOLE**
HERBICIDALLY EFFECTIVE 4- (3-ALKYL SULFINYL BENZOYL) PYRAZOLES
4-(3-ALKYLSULFINYLBENZOYL)PYRAZOLES A ACTION HERBICIDE

(30) Priorität: 29.05.2008 EP 08009758
(43) Veröffentlichungstag der Anmeldung: 23.03.2011
(73) Patentinhaber: Bayer CropScience AG, 40789 Monheim (DE)
(72) Erfinder: VAN ALMSICK, Andreas, 61184 Karben (DE); AHRENS, Hartmut, 63329 Egelsbach (DE); DITTGEN, Jan, 60316 Frankfurt (DE); DÖRNER-RIEPING, Simon, 61267 Neu-Anspach (DE); FEUCHT, Dieter, 65760 Eschborn (DE); HÄUSER-HAHN, Isolde, 51375 Leverkusen (DE); LEHR, Stefan, 65835 Liederbach (DE); ROSINGER, Christopher, Hugh, 65719 Hofheim (DE)
(86) Internationale Anmeldenummer: PCT/EP2009/003474
(87) Internationale Veröffentlichungsnummer: WO 2009/149806

(56) Entgegenhaltungen:
- EP-A- 0 961 774
- WO-A-00/34270
- WO-A-2007/069771
- JP-A- 1 052 759
- US-A- 4 885 022

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte 4-Benzoylpyrazole, die in 3-Position des Phenylrings durch Schwefel haltige Reste substituiert sind, herbizide Eigenschaften besitzen. So werden in EP 0 352 543 und WO 97/41106 Benzoylpyrazole genannt, die im Phenylring unter anderem durch einen Alkylsulfinylrest substituiert sein können. Aus WO 00/03993 und WO 2008/151719 sind unter anderem 3-Cyclopropyl-4-(3-alkylsulfinylbenzoyl)pyrazole bekannt. Außerdem aus JP 1 052759 ist bekannt, dass 4-(3-Alkylthioetherbenzoyl)pyrazole und 4-(3-Alkylsulfoxidebenzoyl)pyrazole herbizide Eigenschaften besitzen.

Die aus diesen Schriften bekannten Verbindungen zeigen jedoch häufig eine nicht ausreichende herbizide Wirksamkeit. Aufgabe der vorliegenden Erfindung ist daher die Bereitstellung alternativer herbizid wirksamer Verbindungen.

Es wurde nun gefunden, daß bestimmte 4-Benzoylpyrazole, deren Phenylring in 3-Position einen Alkylsulfinylrest trägt, als Herbizide besonders gut geeignet sind.

Ein Gegenstand der vorliegenden Erfindung sind 4-(3-Alkylsulfinylbenzoyl)pyrazole der Formel (I) oder deren Salze 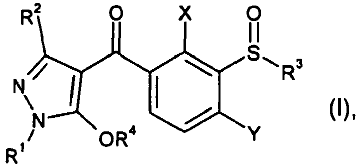 worin
R¹ bedeutet (C₁-C₄)-Alkyl,
R² bedeutet Wasserstoff oder (C₁-C₄)-Alkyl,
R³ bedeutet (C₁-C₆)-Alkyl,
R⁴ bedeutet Wasserstoff, (C₁-C₆)-Alkylsulfonyl, (C₁-C₄)-Alkoxy-(C₁-C₆)-alkylsulfonyl, oder jeweils durch m gleiche oder verschiedene Reste aus der Gruppe bestehend aus Halogen, (C₁-C₄)-Alkyl und (C₁-C₄)-Alkoxy substituiertes Phenylsulfonyl, Thiophenyl-2-sulfonyl, Benzoyl, Benzoyl-(C₁-C₆)-alkyl oder Benzyl,
X bedeutet Wasserstoff, Mercapto, Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, OR⁵, OCOR⁵, OSO₂R⁵, S(O)ₙR⁵, SO₂OR⁵, SO₂N(R⁵)₂, NR⁵SO₂R⁵, NR⁵COR⁵, (C₁-C₆)-Alkyl-S(O)ₙR⁵, (C₁-C₆)-Alkyl-OR⁵, (C₁-C₆)-Alkyl-OCOR⁵, (C₁-C₆)-Alkyl-OSO2R⁵, (C₁-C₆)-Alkyl-SO₂OR⁵, (C₁-C₆)-Alkyl-SO₂N(R⁵)₂ oder (C₁-C₆)-Alkyl-NR⁵COR⁵;
Y bedeutet Fluor, Chlor, Brom, Iod, Nitro oder die Gruppe SO₂R⁷,
R⁵ bedeutet Wasserstoff, (C₁-C₆)-Alkyl, (C₂-C₆)-Alkenyl, (C₂-C₆)-Alkinyl, (C₃-C₆)-Cycloalkyl, Phenyl oder Phenyl-(C₁-C₆)-alkyl, wobei die sechs letztgenannten Reste durch s Reste der Gruppe Hydroxy, Mercapto, Amino, Cyano, Nitro, Rhodano, OR⁶, SR₆, N(R⁶)₂, NOR⁶, OCOR⁶, SCOR⁶, NR⁶COR⁶, CO₂R⁶, COSR⁶, CON(R⁶)₂, (C₁-C₄)-Alkyliminooxy, (C₁-C₄)-Alkylcarbonyl, (C₁-C₄)-Alkoxy-(C₂-C₆)-alkoxycarbonyl und (C₁-C₄)-Alkylsulfonyl substituiert sind;
R⁶ bedeutet Wasserstoff, (C₁-C₆)-Alkyl, (C₂-C₆)-Alkenyl oder (C₂-C₆)-Alkinyl,
R⁷ bedeutet (C₁-C₄)-Alkyl,
m bedeutet 0, 1, 2, 3, 4 oder 5,
n bedeutet 0, 1 oder 2,
s bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) treten in Folge des Chiralitätszentrums der Sulfinylgruppe als Enantiomere auf. Darüberhinaus können je nach Art und Verknüpfung der Substituenten, beispielsweise im Falle von asymmetrisch substituierten Kohlenstoffatomen, weitere Stereoisomere vorliegen. Stereoisomere lassen sich, sofern sie nicht bereits bei der Synthese in reiner Form anfallen, aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Bevorzugt sind Verbindungen der allgemeinen Formel (I), worin
R¹ bedeutet Methyl, Ethyl, n-Propyl oder i-Propyl,
R² bedeutet Wasserstoff, Methyl, Ethyl, n-Propyl oder i-Propyl,
R³ bedeutet (C₁-C₄)-Alkyl,
R⁴ bedeutet Wasserstoff, (C₁-C₃)-Alkylsulfonyl, (C₁-C₂)-Alkoxy-(C₁-C₄)-alkylsulfonyl, oder jeweils durch m Methylgruppen substituiertes Phenylsulfonyl, Thiophenyl-2-sulfonyl, Benzoyl, Benzoyl-(C₁-C₆)-alkyl oder Benzyl,
X bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Fluor, Chlor, Brom, Iod oder die Gruppe SO₂R⁷,
R⁷ bedeutet Methyl, Ethyl, n-Propyl oder i-Propyl,
m bedeutet 0, 1, 2 oder 3.

Besonders bevorzugt sind Verbindungen der allgemeinen Formel (I), worin
R¹ bedeutet Methyl oder Ethyl,
R² bedeutet Wasserstoff, Methyl oder Ethyl,
R³ bedeutet Methyl, Ethyl, n-Propyl oder i-Propyl,
R⁴ bedeutet Wasserstoff,
X bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Fluor, Chlor, Brom, Iod oder die Gruppe SO₂R⁷,
R⁷ bedeutet Methyl oder Ethyl.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁴ für Wasserstoff steht, können beispielsweise nach dem in Schema 1 angegebenen und aus WO2005/122768 bekannten Verfahren durch Reaktion einer Benzoesäure der Formel (II) mit einem Pyrazol der Formel (III) in Anwesenheit eines Kondensationsreagenz, wie N-(3-Dimethylaminopropyl)-N'-ethylcarbodiimid-Hydrochlorid, und anschließender Cyanid katalysierter Umlagerung hergestellt werden.

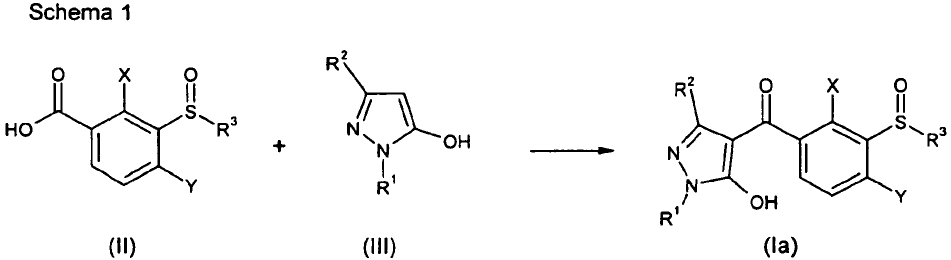

Verbindungen der Formel (I) können beispielsweise nach dem in Schema 2 angegebenen und in W02005/122768 beschriebenen Verfahren durch Reaktion einer Verbindung der Formel (Ia) mit Verbindungen der Formel (IV), in der E für eine nucleophile Abgangsgruppe steht, hergestellt werden.

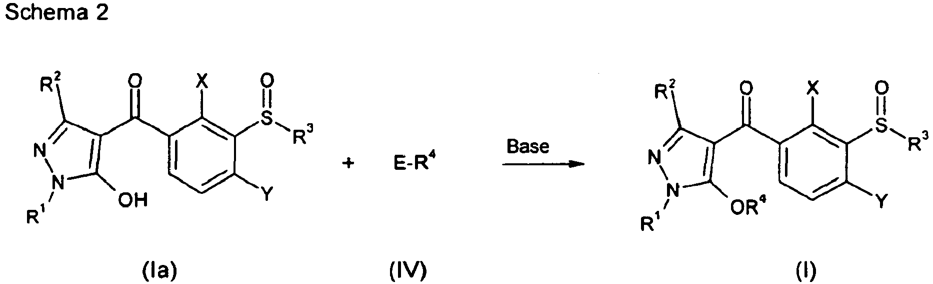

Erfindungsgemäße Verbindungen, in denen R⁴ für Wasserstoff steht, können beispielsweise auch nach dem in Schema 3 angegebenen Verfahren durch nucleophile aromatische Substitution aus Verbindungen der Formel (V), in der L² beispielsweise für ein Fluor- oder Chloratom steht, mit einem Thiolat (VI), worin M für ein Metallkation steht, und anschließende Oxidation hergestellt werden.

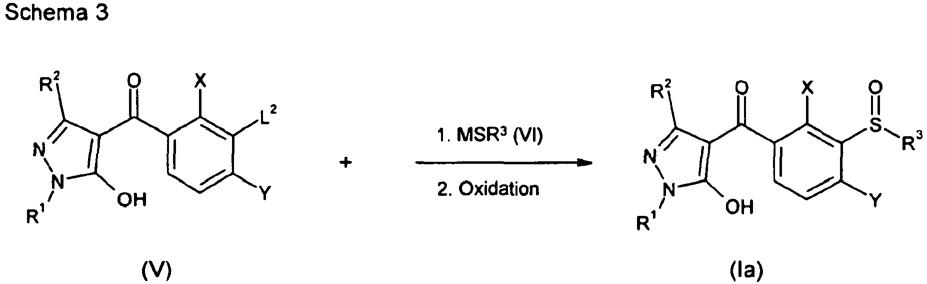

Verbindungen der Formel (II) können beispielsweise nach dem in Schema 4 angegebenen Verfahren aus Benzoesäuren der Formel (VII) durch Einführung des Substituenten X, anschließender nucleophiler aromatischer Substitution und nachfolgender Oxidation hergestellt werden. Geeignete Reaktionen zur Einführung des Substituenten X sind beispielsweise aus J. Chem. Soc. Perkin Trans. 1 1995, S. 1265 ff, J. Heterocyclic Chem. 1999, 36, S. 1453 ff., Angew. Chem. 2005, 117, 380 - 398, J. Org. Chem. 2003, 68, 2030 - 2033 und J. Org. Chem. 1994, 59, 4042 - 4044 bekannt.

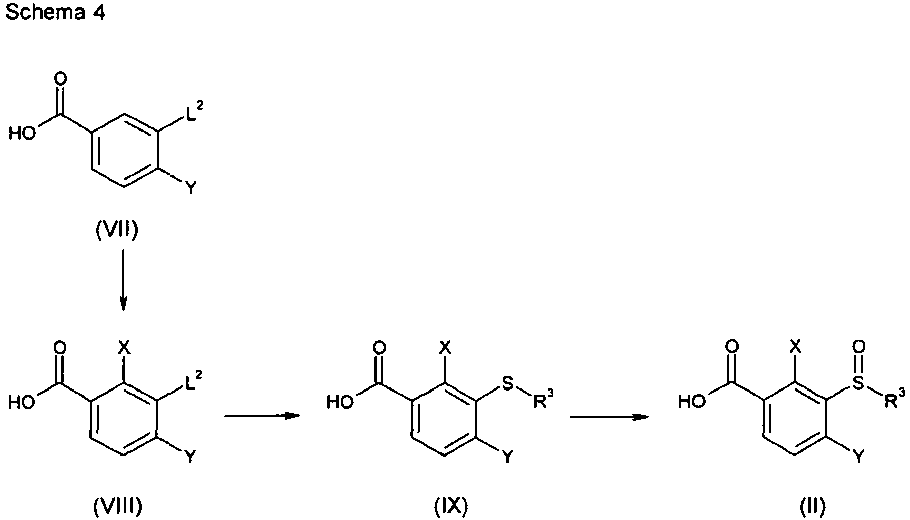

Verbindungen der Formel (II) sind neu und ebenfalls Gegenstand vorliegender Erfindung.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono-oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird. Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie ÖI-in-Wasser- und Wasser-in-ÖI-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. ÖI-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), Iodosulfuron, lodosulfuron-methyl-natrium, loxynil, Isocarbamid, Isopropalin, Isoproturon, Isouron, Isoxaben, Isoxachlortole, Isoxaflutole, Isoxapyrifop, KUH-043, KUH-071, Karbutilate, Ketospiradox, Lactofen, Lenacil, Linuron, Maleinsäurehydrazid, MCPA, MCPB, MCPB-methyl, -ethyl und - natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vernolate, ZJ-0166, ZJ-0270, ZJ-0543, ZJ-0862 sowie die folgenden Verbindungen

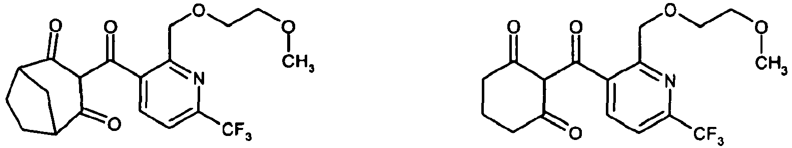

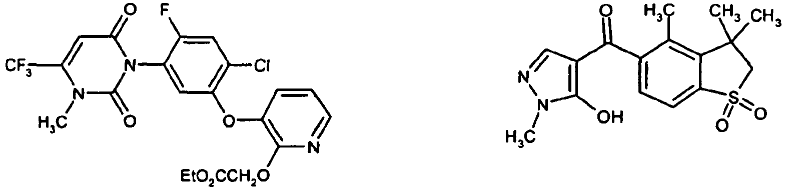

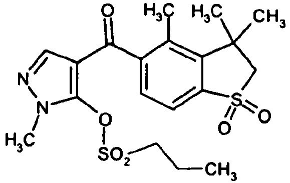

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Herstellung von 4-(4-Chlor-3-ethylsulfinyl-2-methylbenzoyl)-5-hydroxy-1,3-dimethylpyrazol (Nr. 3-238 der Tabelle 3)

### Schritt 1: Synthese von 4-Chlor-3-(dimethylaminothiocarbonyloxy)-2-methylbenzoesäuremethylester

11.0 g (54.8 mmol) 4-Chlor-3-hydroxy-2-methylbenzoesäuremethylester wurden unter Stickstoff-Atmosphäre in 200 ml N,N-Dimethylformamid mit 12.3 g (109.7 mmol) 1,4-Diazabicyclo[2.2.2]octan und anschließend mit 13.6 g (109.7 mmol) Dimethylaminothiocarbonylchlorid versetzt. Das Gemisch wurde 16 h bei Raumtemperatur (RT) gerührt, zur Aufarbeitung wurde es auf Eiswasser gegossen. Das Produkt fiel aus und wurde über eine Filtration abgetrennt. Der Rückstand wurde 1M HCl gewaschen. Es wurden 14.7 g sauberes Produkt erhalten.

### Schritt 2: Synthese von 4-Chlor-3-(dimethylaminocarbonylthio)-2-methylbenzoesäuremethylester

12.1 g (42.0 mmol) 4-Chlor-3-(dimethylaminothiocarbonyloxy)-2-methylbenzoesäuremethylester wurden unter Stickstoff-Atmosphäre in 30 ml 1,3-Dimethoxybenzol 6 h lang auf 220°C erhitzt. Zur Aufarbeitung wurde das Reaktionsgemisch abgekühlt und unter Vakuum eingeengt. Nach chromatographischer Reinigung des Rückstands wurden 5.2 g sauberes Produkt isoliert.

### Schritt 3: Synthese von 4-Chlor-3-mercapto-2-methylbenzoesäure

4.80 g (16.7 mmol) 4-Chlor-3-(dimethylaminocarbonylthio)-2-methylbenzoesäuremethylester wurden in 150 ml Methanol mit 6.61 g (85 Gew.-% Reinheit, 100.1 mmol) Kaliumhydroxid versetzt und unter Rückfluss zwei Tage gerührt. Das Reaktionsgemisch wurde vom Lösungsmittel befreit, und der Rückstand wurde mit Wasser versetzt, danach mit 1 M HCl angesäuert, und der Feststoff wurde über eine Filtration abgetrennt. Es wurden 3.2 g sauberes Produkt erhalten.

### Schritt 4: Synthese von 4-Chlor-3-ethylthio-2-methylbenzoesäure

1.10 g (5.42 mmol) 4-Chlor-3-mercapto-2-methylbenzoesäure wurden in 20 ml Acetonitril mit 3.71 g (11.3 mmol) Caesiumcarbonat versetzt. Das Reaktionsgemisch wurde 10 min. bei RT gerührt, anschließend wurden langsam 1.02 g (6.51 mmol) lodethan zugetropft. Das Reaktionsgemisch wurde danach 16 h bei RT gerührt. Anschließend wurde das Lösungsmittel abgetrennt, und der Rückstand wurde mit einem Gemisch von 20 ml Methanol und 2 ml 20 %-iger Natronlauge versetzt. Das Gemisch wurde zur Verseifung des entstandenen Ethylesters 16 h bei RT gerührt und danach von den Lösungsmitteln befreit. Der Rückstand wurde in Wasser aufgenommen, mit verdünnter HCl angesäuert, und nachdem das Gemisch 10 min. bei RT gerührt hatte wurde es filtriert. Der Rückstand wurde mit Wasser gewaschen und trocken gesaugt. Es wurden 1.15 g sauberes Produkt isoliert.

### Schritt 5: Synthese von 4-Chlor-3-ethylsulfinyl-2-methylbenzoesäure (Nr. 4-238)

1.15 g (4.98 mmol) 4-Chlor-3-ethylthio-2-methylbenzoesäure wurden in 10 ml Eisessig auf eine Temperatur von 50 °C - 60 °C erhitzt. Bei dieser Temperatur wurden 484 mg (35 %-ig, 4.98 mmol) einer wäßrigen Wasserstoffperoxid-Lösung tropfenweise zugegeben. Das Gemisch wurde 5 h bei dieser Temperatur gerührt. Das Reaktionsgemisch wurde abgekühlt, mit einer wässrigen gesättigten Natriumhydrogensulfit-Lösung gewaschen und nach dem analytischen Nachweis der Abwesenheit von Peroxiden von den Lösungsmitteln befreit. Der Rückstand wurde mit Wasser versetzt und mit 6M HCl angesäuert. Das Gemisch wurde mit Essigsäureethylester extrahiert, die vereinigten organischen Phasen wurden getrocknet, filtriert und unter Vakuum von dem Lösungsmittel befreit. 1.24 g des Produkts (Reinheit ca. 95 Gew.-%) wurden isoliert.

### Schritt 6: Synthese von 4-(4-Chlor-3-ethylsulfinyl-2-methylbenzoyl)-5-hydroxy-1,3-dimethylpyrazol

270 mg (95 Gew.-% Reinheit; 1.04 mmol) 4-Chlor-3-ethylsulfinyl-2-methylbenzoesäure wurden in 20 ml Dichlormethan (CH₂Cl₂) mit 128 mg (1.14 mmol) 5-Hydroxy-1,3-dimethylpyrazol versetzt. 239 mg (1.24 mmol) 1-(3'-Dimethylaminopropyl)-3-ethylcarbodiimidhydrochlorid wurden zugegeben, und das Gemisch wurde 16 h bei RT gerührt. Zur Aufarbeitung wurden 3ml 1M HCl zugegeben, und die organische Phase wurde vom Lösungsmittel befreit. Der Rückstand wurde in 20 ml Acetonitril mit 210 mg (2.07 mmol) Triethylamin, 10 Tropfen Acetoncyanhydrin sowie einer Spatelspitze Kaliumcyanid versetzt. Das Reaktionsgemisch wurde 16 h bei RT und anschließend vom Lösungsmittel befreit. Der Rückstand wurde mit 25 ml eines Gemisches aus wässriger gesättigter Natriumhydrogencarbonat-Lösung und Diethylether 10 min. lang bei RT gerührt. Die Phasen wurden getrennt, die wässrige Phase wurde mit verdünnter HCl angesäuert und anschließend mit CH₂Cl₂ extrahiert. Die organische Phase wurde vom Lösungsmittel befreit, und der Rückstand wurde danach chromatographisch gereinigt. Es wurden 100 mg sauberes Produkt erhalten.

### 2. Herstellung von 5-Hydroxy-1-methyl-4-(2-methyl-3-methylsulfinyl-4-methylsulfonylbenzoyl)pyrazol (Nr. 1-9 der Tabelle 1)

### Schritt 1: Synthese von 2-Methyl-3-methylsulfinyl-4-methylsulfonylbenzoesäure (Nr. 4-9)

900 mg (3.45 mmol) 2-Methyl-4-methylsulfonyl-3-methylthiobenzoesäure wurden in 10 ml Eisessig auf eine Temperatur von 50 °C - 60 °C erhitzt. Bei dieser Temperatur wurden 336 mg (35 %-ig, 3.45 mmol) einer wäßrigen Wasserstoffperoxid-Lösung tropfenweise zugegeben. Das Gemisch wurde 5 h bei 60 °C gerührt. Das Reaktionsgemisch wurde abgekühlt, mit einer wässrigen 10 %-igen Natriumhydrogensulfit-Lösung gewaschen und nach dem analytischen Nachweis der Abwesenheit von Peroxiden von den Lösungsmitteln befreit. Der Rückstand wurde mit Wasser versetzt, und die wässrige Phase wurde dreimal mit Essigsäureethylester extrahiert. Die vereinigten organischen Phasen wurden getrocknet, filtriert und unter Vakuum von dem Lösungsmittel befreit. 950 mg des sauberen Produkts wurden isoliert.

### Schritt 2: Synthese von 5-Hydroxy-1-methyl-4-(2-methyl-3-methylsulfinyl-4-methylsulfonylbenzoyl)pyrazol

230 mg (0.83 mmol) 2-Methyl-3-methylsulfinyl-4-methylsulfonylbenzoesäure wurden in 20 ml CH₂Cl₂ mit 90 mg (0.91 mmol) 5-Hydroxy-1-methylpyrazol und einer Spatelspitze 4-(Dimethylamino)pyridin versetzt. 191 mg (0.99 mmol) 1-(3'-Dimethylaminopropyl)-3-ethylcarbodiimidhydrochlorid wurden zugegeben, und das Gemisch wurde 90 min. bei RT gerührt. Dann wurden 3ml 1 M HCl zugegeben, und die organische Phase wurde vom Lösungsmittel befreit. Der Rückstand wurde in 15 ml Acetonitril mit 168 mg (1.66 mmol) Triethylamin, 10 Tropfen Acetoncyanhydrin sowie einer Spatelspitze Kaliumcyanid versetzt. Das Reaktionsgemisch wurde 16 h bei RT und anschließend vom Lösungsmittel befreit. Der Rückstand wurde mit 15 ml CH₂Cl₂ versetzt, anschließend wurden 2 ml 1M HCl zugegeben. Nach der Phasentrennung wurde die organische Phase vom Lösungsmittel befreit. Der Rückstand wurde mit 25 ml eines Gemisches aus wässriger gesättigter Natriumhydrogencarbonat-Lösung und Diethylether 10 min. lang bei RT gerührt. Die Phasen wurden getrennt, die wässrige Phase wurde mit verdünnter HCl angesäuert und anschließend dreimal mit CH₂Cl₂ extrahiert. Die vereinigten organischen Phasen wurden vom Lösungsmittel befreit. Es wurden 171 mg sauberes Produkt erhalten.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:

Et = Ethyl Me = Methyl Pr = Propyl Ph = Phenyl

**Tabelle 1: Erfindungsgemäße Verbindungen der allgemeinen Formel (I), worin R¹ für Methyl, R² und R⁴ jeweils für Wasserstoff stehen.**

| | | | | |
|---|---|---|---|---|
| 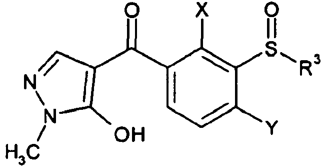 | | | | |

| Nr. | X | R³ | Y | Physikalische Daten: ¹H-NMR: δ [CDCl₃] |
|---|---|---|---|---|
| 1-1 | Cl | Me | SO₂Me | |
| 1-2 | Cl | Et | SO₂Me | 8.23 (d, 1H), 7.66 (d, 1H), 7.32 (s, 1 H), 3.92 (m, 1 H), 3.73 (s, 3H), 3.45 (s, 3H), 3.28 (m, 1H), 1.53 (t, 3H) |
| 1-3 | Cl | n-Pr | SO₂Me | |
| 1-4 | Cl | i-Pr | SO₂Me | |
| 1-5 | Br | Me | SO₂Me | |
| 1-6 | Br | Et | SO₂Me | |
| 1-7 | Br | n-Pr | SO₂Me | |
| 1-8 | Br | i-Pr | SO₂Me | |
| 1-9 | Me | Me | SO₂Me | 8.08 (d, 1H), 7.62 (d, 1H), 7.27 (s, 1H), 3.72 (s, 3H), 3.38 (s, 3H), 3.18 (s, 3H), 2.87 (s, 3H) |
| 1-10 | Me | Et | SO₂Me | 8.08 (d, 1H), 7.61 (d, 1H), 7.27 (s, 1H), 3.72 (s, 3H), 3.52 (m, 1H), 3.38 (s, 3H), 3.28 (m, 1H), 2.82 (s, 3H), 1.52 (t, 3H) |
| 1-11 | Me | n-Pr | SO₂Me | |
| 1-12 | Me | i-Pr | SO₂Me | |
| 1-13 | Et | Me | SO₂Me | |
| 1-14 | Et | Et | SO₂Me | |
| 1-15 | Et | n-Pr | SO₂Me | |
| 1-16 | Et | i-Pr | SO₂Me | |
| 1-17 | CF₃ | Me | SO₂Me | |
| 1-18 | CF₃ | Et | SO₂Me | |
| 1-19 | CF₃ | n-Pr | SO₂Me | |
| 1-20 | CF₃ | i-Pr | SO₂Me | |
| 1-21 | OMe | Me | SO₂Me | |
| 1-22 | OMe | Et | SO₂Me | |
| 1-23 | OMe | n-Pr | SO₂Me | |
| 1-24 | OMe | i-Pr | SO₂Me | |
| 1-25 | OEt | Me | SO₂Me | |
| 1-26 | OEt | Et | SO₂Me | |
| 1-27 | OEt | n-Pr | SO₂Me | |
| 1-28 | OEt | i-Pr | SO₂Me | |
| 1-29 | NO₂ | Me | SO₂Me | |
| 1-30 | NO₂ | Et | SO₂Me | |
| 1-31 | NO₂ | n-Pr | SO₂Me | |
| 1-32 | NO₂ | i-Pr | SO₂Me | |
| 1-33 | SO₂Me | Me | SO₂Me | |
| 1-34 | SO₂Me | Et | SO₂Me | |
| 1-35 | SO₂Me | n-Pr | SO₂Me | |
| 1-36 | SO₂Me | i-Pr | SO₂Me | |
| 1-37 | CH₂OMe | Me | SO₂Me | |
| 1-38 | CH₂OMe | Et | SO₂Me | |
| 1-39 | CH₂OMe | n-Pr | SO₂Me | |
| 1-40 | CH₂OMe | i-Pr | SO₂Me | |
| 1-41 | CH₂SO₂Me | Me | SO₂Me | |
| 1-42 | CH₂SO₂Me | Et | SO₂Me | |
| 1-43 | CH₂SO₂Me | n-Pr | SO₂Me | |
| 1-44 | CH₂SO₂Me | i-Pr | SO₂Me | |
| 1-45 | CH₂OCH₂CH₂OMe | Me | SO₂Me | 8.21 (d, 1H), 7.67 (d, 1H), 7.37 (s, 1H), 5.57 - 5.32 (br. Signal, 1H), 5.00 (d, 1H), 3,72 (s, 3H), 3.63 - 3.58 (m, 1H), 3.56 - 3.50 (m, 1H), 3,42 - 3,25 (m, 2H), 3.37 (s, 3H), 3.32 (s, 3H), 3.23 (s, 3H) |
| 1-46 | CH₂OCH₂CH₂OMe | Et | SO₂Me | |
| 1-47 | CH₂OCH₂CH₂OMe | n-Pr | SO₂Me | |
| 1-48 | CH₂OCH₂CH₂OMe | i-Pr | SO₂Me | |
| 1-49 | CH₂OCH₂CH₂OEt | Me | SO₂Me | |
| 1-50 | CH₂OCH₂CH₂OEt | Et | SO₂Me | |
| 1-51 | CH₂OCH₂CH₂OEt | n-Pr | SO₂Me | |
| 1-52 | CH₂OCH₂CH₂OEt | i-Pr | SO₂Me | |
| 1-53 | CH₂OCH₂CH₂CH₂OMe | Me | SO₂Me | |
| 1-54 | CH₂OCH₂CH₂CH₂OMe | Et | SO₂Me | |
| 1-55 | CH₂OCH₂CH₂CH₂OMe | n-Pr | SO₂Me | |
| 1-56 | CH₂OCH₂CH₂CH₂OMe | i-Pr | SO₂Me | |
| 1-57 | CH₂OCH₂OMe | Me | SO₂Me | |
| 1-58 | CH₂OCH₂OMe | Et | SO₂Me | |
| 1-59 | CH₂OCH₂OMe | n-Pr | SO₂Me | |
| 1-60 | CH₂OCH₂OMe | i-Pr | SO₂Me | |
| 1-61 | CH₂OCH₂OEt | Me | SO₂Me | |
| 1-62 | CH₂OCH₂OEt | Et | SO₂Me | |
| 1-63 | CH₂OCH₂OEt | n-Pr | SO₂Me | |
| 1-64 | CH₂OCH₂OEt | i-Pr | SO₂Me | |
| 1-65 | CH₂OCH₂CH₂SO₂Me | Me | SO₂Me | |
| 1-66 | CH₂OCH₂CH₂SO₂Me | Et | SO₂Me | |
| 1-67 | CH₂OCH₂CH₂SO₂Me | n-Pr | SO₂Me | |
| 1-68 | CH₂OCH₂CH₂SO₂Me | i-Pr | SO₂Me | |
| 1-69 | CH₂SO₂CH₂CH₂OMe | Me | SO₂Me | |
| 1-70 | CH₂SO₂CH₂CH₂OMe | Et | SO₂Me | |
| 1-71 | CH₂SO₂CH₂CH₂OMe | n-Pr | SO₂Me | |
| 1-72 | CH₂SO₂CH₂CH₂OMe | i-Pr | SO₂Me | |
| 1-73 | CH₂SO₂CH₂CH₂SO₂Me | Me | SO₂Me | |
| 1-74 | CH₂SO₂CH₂CH₂SO₂Me | Et | SO₂Me | |
| 1-75 | CH₂SO₂CH₂CH₂SO₂Me | n-Pr | SO₂Me | |
| 1-76 | CH₂SO₂CH₂CH₂SO₂Me | i-Pr | SO₂Me | |
| 1-77 | Cl | Me | SO₂Et | |
| 1-78 | Cl | Et | SO₂Et | |
| 1-79 | Cl | n-Pr | SO₂Et | |
| 1-80 | Cl | i-Pr | SO₂Et | |
| 1-81 | Br | Me | SO₂Et | |
| 1-82 | Br | Et | SO₂Et | |
| 1-83 | Br | n-Pr | SO₂Et | |
| 1-84 | Br | i-Pr | SO₂Et | |
| 1-85 | Me | Me | SO₂Et | |
| 1-86 | Me | Et | SO₂Et | |
| 1-87 | Me | n-Pr | SO₂Et | |
| 1-88 | Me | i-Pr | SO₂Et | |
| 1-89 | Et | Me | SO₂Et | |
| 1-90 | Et | Et | SO₂Et | |
| 1-91 | Et | n-Pr | SO₂Et | |
| 1-92 | Et | i-Pr | SO₂Et | |
| 1-93 | CF₃ | Me | SO₂Et | |
| 1-94 | CF₃ | Et | SO₂Et | |
| 1-95 | CF₃ | n-Pr | SO₂Et | |
| 1-96 | CF₃ | i-Pr | SO₂Et | |
| 1-97 | OMe | Me | SO₂Et | |
| 1-98 | OMe | Et | SO₂Et | |
| 1-99 | OMe | n-Pr | SO₂Et | |
| 1-100 | OMe | i-Pr | SO₂Et | |
| 1-101 | OEt | Me | SO₂Et | |
| 1-102 | OEt | Et | SO₂Et | |
| 1-103 | OEt | n-Pr | SO₂Et | |
| 1-104 | OEt | i-Pr | SO₂Et | |
| 1-105 | NO₂ | Me | SO₂Et | |
| 1-106 | NO₂ | Et | SO₂Et | |
| 1-107 | NO₂ | n-Pr | SO₂Et | |
| 1-108 | NO₂ | i-Pr | SO₂Et | |
| 1-109 | SO₂Me | Me | SO₂Et | |
| 1-110 | SO₂Me | Et | SO₂Et | |
| 1-111 | SO₂Me | n-Pr | SO₂Et | |
| 1-112 | SO₂Me | i-Pr | SO₂Et | |
| 1-113 | CH₂OMe | Me | SO₂Et | |
| 1-114 | CH₂OMe | Et | SO₂Et | |
| 1-115 | CH₂OMe | n-Pr | SO₂Et | |
| 1-116 | CH₂OMe | i-Pr | SO₂Et | |
| 1-117 | CH₂SO₂Me | Me | SO₂Et | |
| 1-118 | CH₂SO₂Me | Et | SO₂Et | |
| 1-119 | CH₂SO₂Me | n-Pr | SO₂Et | |
| 1-120 | CH₂SO₂Me | i-Pr | SO₂Et | |
| 1-121 | CH₂OCH₂CH₂OMe | Me | SO₂Et | |
| 1-122 | CH₂OCH₂CH₂OMe | Et | SO₂Et | |
| 1-123 | CH₂OCH₂CH₂OMe | n-Pr | SO₂Et | |
| 1-124 | CH₂OCH₂CH₂OMe | i-Pr | SO₂Et | |
| 1-125 | CH₂OCH₂CH₂OEt | Me | SO₂Et | |
| 1-126 | CH₂OCH₂CH₂OEt | Et | SO₂Et | |
| 1-127 | CH₂OCH₂CH₂OEt | n-Pr | SO₂Et | |
| 1-128 | CH₂OCH₂CH₂OEt | i-Pr | SO₂Et | |
| 1-129 | CH₂OCH₂CH₂CH₂OMe | Me | SO₂Et | |
| 1-130 | CH₂OCH₂CH₂CH₂OMe | Et | SO₂Et | |
| 1-131 | CH₂OCH₂CH₂CH₂OMe | n-Pr | SO₂Et | |
| 1-132 | CH₂OCH₂CH₂CH₂OMe | i-Pr | SO₂Et | |
| 1-133 | CH₂OCH₂OMe | Me | SO₂Et | |
| 1-134 | CH₂OCH₂OMe | Et | SO₂Et | |
| 1-135 | CH₂OCH₂OMe | n-Pr | SO₂Et | |
| 1-136 | CH₂OCH₂OMe | i-Pr | SO₂Et | |
| 1-137 | CH₂OCH₂OEt | Me | SO₂Et | |
| 1-138 | CH₂OCH₂OEt | Et | SO₂Et | |
| 1-139 | CH₂OCH₂OEt | n-Pr | SO₂Et | |
| 1-140 | CH₂OCH₂OEt | i-Pr | SO₂Et | |
| 1-141 | CH₂OCH₂CH₂SO₂Me | Me | SO₂Et | |
| 1-142 | CH₂OCH₂CH₂SO₂Me | Et | SO₂Et | |
| 1-143 | CH₂OCH₂CH₂SO₂Me | n-Pr | SO₂Et | |
| 1-144 | CH₂OCH₂CH₂SO₂Me | i-Pr | SO₂Et | |
| 1-145 | CH₂SO₂CH₂CH₂OMe | Me | SO₂Et | |
| 1-146 | CH₂SO₂CH₂CH₂OMe | Et | SO₂Et | |
| 1-147 | CH₂SO₂CH₂CH₂OMe | n-Pr | SO₂Et | |
| 1-148 | CH₂SO₂CH₂CH₂OMe | i-Pr | SO₂Et | |
| 1-149 | CH₂SO₂CH₂CH₂SO₂Me | Me | SO₂Et | |
| 1-150 | CH₂SO₂CH₂CH₂SO₂Me | Et | SO₂Et | |
| 1-151 | CH₂SO₂CH₂CH₂SO₂Me | n-Pr | SO₂Et | |
| 1-152 | CH₂SO₂CH₂CH₂SO₂Me | i-Pr | SO₂Et | |
| 1-153 | Cl | Me | F | |
| 1-154 | Cl | Et | F | |
| 1-155 | Cl | n-Pr | F | |
| 1-156 | Cl | i-Pr | F | |
| 1-157 | Br | Me | F | |
| 1-158 | Br | Et | F | |
| 1-159 | Br | n-Pr | F | |
| 1-160 | Br | i-Pr | F | |
| 1-161 | Me | Me | F | |
| 1-162 | Me | Et | F | |
| 1-163 | Me | n-Pr | F | |
| 1-164 | Me | i-Pr | F | |
| 1-165 | Et | Me | F | |
| 1-166 | Et | Et | F | |
| 1-167 | Et | n-Pr | F | |
| 1-168 | Et | i-Pr | F | |
| 1-169 | CF₃ | Me | F | |
| 1-170 | CF₃ | Et | F | |
| 1-171 | CF₃ | n-Pr | F | |
| 1-172 | CF₃ | i-Pr | F | |
| 1-173 | OMe | Me | F | |
| 1-174 | OMe | Et | F | |
| 1-175 | OMe | n-Pr | F | |
| 1-176 | OMe | i-Pr | F | |
| 1-177 | OEt | Me | F | |
| 1-178 | OEt | Et | F | |
| 1-179 | OEt | n-Pr | F | |
| 1-180 | OEt | i-Pr | F | |
| 1-181 | NO₂ | Me | F | |
| 1-182 | NO₂ | Et | F | |
| 1-183 | NO₂ | n-Pr | F | |
| 1-184 | NO₂ | i-Pr | F | |
| 1-185 | SO₂Me | Me | F | |
| 1-186 | SO₂Me | Et | F | |
| 1-187 | SO₂Me | n-Pr | F | |
| 1-188 | SO₂Me | i-Pr | F | |
| 1-189 | CH₂OMe | Me | F | |
| 1-190 | CH₂OMe | Et | F | |
| 1-191 | CH₂OMe | n-Pr | F | |
| 1-192 | CH₂OMe | i-Pr | F | |
| 1-193 | CH₂SO₂Me | Me | F | |
| 1-194 | CH₂SO₂Me | Et | F | |
| 1-195 | CH₂SO₂Me | n-Pr | F | |
| 1-196 | CH₂SO₂Me | i-Pr | F | |
| 1-197 | CH₂OCH₂CH₂OMe | Me | F | |
| 1-198 | CH₂OCH₂CH₂OMe | Et | F | |
| 1-199 | CH₂OCH₂CH₂OMe | n-Pr | F | |
| 1-200 | CH₂OCH₂CH₂OMe | i-Pr | F | |
| 1-201 | CH₂OCH₂CH₂OEt | Me | F | |
| 1-202 | CH₂OCH₂CH₂OEt | Et | F | |
| 1-203 | CH₂OCH₂CH₂OEt | n-Pr | F | |
| 1-204 | CH₂OCH₂CH₂OEt | i-Pr | F | |
| 1-205 | CH₂OCH₂CH₂CH₂OMe | Me | F | |
| 1-206 | CH₂OCH₂CH₂CH₂OMe | Et | F | |
| 1-207 | CH₂OCH₂CH₂CH₂OMe | n-Pr | F | |
| 1-208 | CH₂OCH₂CH₂CH₂OMe | i-Pr | F | |
| 1-209 | CH₂OCH₂OMe | Me | F | |
| 1-210 | CH₂OCH₂OMe | Et | F | |
| 1-211 | CH₂OCH₂OMe | n-Pr | F | |
| 1-212 | CH₂OCH₂OMe | i-Pr | F | |
| 1-213 | CH₂OCH₂OEt | Me | F | |
| 1-214 | CH₂OCH₂OEt | Et | F | |
| 1-215 | CH₂OCH₂OEt | n-Pr | F | |
| 1-216 | CH₂OCH₂OEt | i-Pr | F | |
| 1-217 | CH₂OCH₂CH₂SO₂Me | Me | F | |
| 1-218 | CH₂OCH₂CH₂SO₂Me | Et | F | |
| 1-219 | CH₂OCH₂CH₂SO₂Me | n-Pr | F | |
| 1-220 | CH₂OCH₂CH₂SO₂Me | i-Pr | F | |
| 1-221 | CH₂SO₂CH₂CH₂OMe | Me | F | |
| 1-222 | CH₂SO₂CH₂CH₂OMe | Et | F | |
| 1-223 | CH₂SO₂CH₂CH₂OMe | n-Pr | F | |
| 1-224 | CH₂SO₂CH₂CH₂OMe | i-Pr | F | |
| 1-225 | CH₂SO₂CH₂CH₂SO₂Me | Me | F | |
| 1-226 | CH₂SO₂CH₂CH₂SO₂Me | Et | F | |
| 1-227 | CH₂SO₂CH₂CH₂SO₂Me | n-Pr | F | |
| 1-228 | CH₂SO₂CH₂CH₂SO₂Me | i-Pr | F | |
| 1-229 | Cl | Me | Cl | |
| 1-230 | Cl | Et | Cl | |
| 1-231 | Cl | n-Pr | Cl | |
| 1-232 | Cl | i-Pr | Cl | |
| 1-233 | Br | Me | Cl | |
| 1-234 | Br | Et | Cl | |
| 1-235 | Br | n-Pr | Cl | |
| 1-236 | Br | i-Pr | Cl | |
| 1-237 | Me | Me | Cl | 7.41 (d, 1H), 7.36 (d, 1H), 7.32 (s, 1H), 3.71 (s, 3H), 3.02 (s, 3H), 2.74 (s, 3H) |
| 1-238 | Me | Et | Cl | 7.41 (d, 1H), 7.36 (d, 1H), 7.31 (s, 1H), 3.71 (s, 3H), 3.38 (m, 1H), 3.16 (m, 1H), 2.72 (s, 3H), 1.41 (t, 3H) |
| 1-239 | Me | n-Pr | Cl | |
| 1-240 | Me | i-Pr | Cl | |
| 1-241 | Et | Me | Cl | |
| 1-242 | Et | Et | Cl | |
| 1-243 | Et | n-Pr | Cl | |
| 1-244 | Et | i-Pr | Cl | |
| 1-245 | CF₃ | Me | Cl | |
| 1-246 | CF₃ | Et | Cl | |
| 1-247 | CF₃ | n-Pr | Cl | |
| 1-248 | CF₃ | i-Pr | Cl | |
| 1-249 | OMe | Me | Cl | |
| 1-250 | OMe | Et | Cl | |
| 1-251 | OMe | n-Pr | Cl | |
| 1-252 | OMe | i-Pr | Cl | |
| 1-253 | OEt | Me | Cl | |
| 1-254 | OEt | Et | Cl | |
| 1-255 | OEt | n-Pr | Cl | |
| 1-256 | OEt | i-Pr | Cl | |
| 1-257 | NO₂ | Me | Cl | |
| 1-258 | NO₂ | Et | Cl | |
| 1-259 | NO₂ | n-Pr | Cl | |
| 1-260 | NO₂ | i-Pr | Cl | |
| 1-261 | SO₂Me | Me | Cl | |
| 1-262 | SO₂Me | Et | Cl | |
| 1-263 | SO₂Me | n-Pr | Cl | |
| 1-264 | SO₂Me | i-Pr | Cl | |
| 1-265 | CH₂OMe | Me | Cl | |
| 1-266 | CH₂OMe | Et | Cl | |
| 1-267 | CH₂OMe | n-Pr | Cl | |
| 1-268 | CH₂OMe | i-Pr | Cl | |
| 1-269 | CH₂SO₂Me | Me | Cl | |
| 1-270 | CH₂SO₂Me | Et | Cl | |
| 1-271 | CH₂SO₂Me | n-Pr | Cl | |
| 1-272 | CH₂SO₂Me | i-Pr | Cl | |
| 1-273 | CH₂OCH₂CH₂OMe | Me | Cl | |
| 1-274 | CH₂OCH₂CH₂OMe | Et | Cl | |
| 1-275 | CH₂OCH₂CH₂OMe | n-Pr | Cl | |
| 1-276 | CH₂OCH₂CH₂OMe | i-Pr | Cl | |
| 1-277 | CH₂OCH₂CH₂OEt | Me | Cl | |
| 1-278 | CH₂OCH₂CH₂OEt | Et | Cl | |
| 1-279 | CH₂OCH₂CH₂OEt | n-Pr | Cl | |
| 1-280 | CH₂OCH₂CH₂OEt | i-Pr | Cl | |
| 1-281 | CH₂OCH₂CH₂CH₂OMe | Me | Cl | |
| 1-282 | CH₂OCH₂CH₂CH₂OMe | Et | Cl | |
| 1-283 | CH₂OCH₂CH₂CH₂OMe | n-Pr | Cl | |
| 1-284 | CH₂OCH₂CH₂CH₂OMe | i-Pr | Cl | |
| 1-285 | CH₂OCH₂OMe | Me | Cl | |
| 1-286 | CH₂OCH₂OMe | Et | Cl | |
| 1-287 | CH₂OCH₂OMe | n-Pr | Cl | |
| 1-288 | CH₂OCH₂OMe | i-Pr | Cl | |
| 1-289 | CH₂OCH₂OEt | Me | Cl | |
| 1-290 | CH₂OCH₂OEt | Et | Cl | |
| 1-291 | CH₂OCH₂OEt | n-Pr | Cl | |
| .1-292 | CH₂OCH₂OEt | i-Pr | Cl | |
| 1-293 | CH₂OCH₂CH₂SO₂Me | Me | Cl | |
| 1-294 | CH₂OCH₂CH₂SO₂Me | Et | Cl | |
| 1-295 | CH₂OCH₂CH₂SO₂Me | n-Pr | Cl | |
| 1-296 | CH₂OCH₂CH₂SO₂Me | i-Pr | Cl | |
| 1-297 | CH₂SO₂CH₂CH₂OMe | Me | Cl | |
| 1-298 | CH₂SO₂CH₂CH₂OMe | Et | Cl | |
| 1-299 | CH₂SO₂CH₂CH₂OMe | n-Pr | Cl | |
| 1-300 | CH₂SO₂CH₂CH₂OMe | i-Pr | Cl | |
| 1-301 | CH₂SO₂CH₂CH₂SO₂Me | Me | Cl | |
| 1-302 | CH₂SO₂CH₂CH₂SO₂Me | Et | Cl | |
| 1-303 | CH₂SO₂CH₂CH₂SO₂Me | n-Pr | Cl | |
| 1-304 | CH₂SO₂CH₂CH₂SO₂Me | i-Pr | Cl | |
| 1-305 | Cl | Me | Br | |
| 1-306 | Cl | Et | Br | |
| 1-307 | Cl | n-Pr | Br | |
| 1-308 | Cl | i-Pr | Br | |
| 1-309 | Br | Me | Br | |
| 1-310 | Br | Et | Br | |
| 1-311 | Br | n-Pr | Br | |
| 1-312 | Br | i-Pr | Br | |
| 1-313 | Me | Me | Br | 7.55 (d, 1H), 7.32 (s, 1H), 7.30 (d, 1H), 3.72 (s, 3H), 3.01 (s, 3H), 2.77 (s, 3H) |
| 1-314 | Me | Et | Br | |
| 1-315 | Me | n-Pr | Br | |
| 1-316 | Me | i-Pr | Br | |
| 1-317 | Et | Me | Br | |
| 1-318 | Et | Et | Br | |
| 1-319 | Et | n-Pr | Br | |
| 1-320 | Et | i-Pr | Br | |
| 1-321 | CF₃ | Me | Br | |
| 1-322 | CF₃ | Et | Br | |
| 1-323 | CF₃ | n-Pr | Br | |
| 1-324 | CF₃ | i-Pr | Br | |
| 1-325 | OMe | Me | Br | |
| 1-326 | OMe | Et | Br | |
| 1-327 | OMe | n-Pr | Br | |
| 1-328 | OMe | i-Pr | Br | |
| 1-329 | OEt | Me | Br | |
| 1-330 | OEt | Et | Br | |
| 1-331 | OEt | n-Pr | Br | |
| 1-332 | OEt | i-Pr | Br | |
| 1-333 | NO₂ | Me | Br | |
| 1-334 | NO₂ | Et | Br | |
| 1-335 | NO₂ | n-Pr | Br | |
| 1-336 | NO₂ | i-Pr | Br | |
| 1-337 | SO₂Me | Me | Br | |
| 1-338 | SO₂Me | Et | Br | |
| 1-339 | SO₂Me | n-Pr | Br | |
| 1-340 | SO₂Me | i-Pr | Br | |
| 1-341 | CH₂OMe | Me | Br | |
| 1-342 | CH₂OMe | Et | Br | |
| 1-343 | CH₂OMe | n-Pr | Br | |
| 1-344 | CH₂OMe | i-Pr | Br | |
| 1-345 | CH₂SO₂Me | Me | Br | |
| 1-346 | CH₂SO₂Me | Et | Br | |
| 1-347 | CH₂SO₂Me | n-Pr | Br | |
| 1-348 | CH₂SO₂Me | i-Pr | Br | |
| 1-349 | CH₂OCH₂CH₂OMe | Me | Br | |
| 1-350 | CH₂OCH₂CH₂OMe | Et | Br | |
| 1-351 | CH₂OCH₂CH₂OMe | n-Pr | Br | |
| 1-352 | CH₂OCH₂CH₂OMe | i-Pr | Br | |
| 1-353 | CH₂OCH₂CH₂OEt | Me | Br | |
| 1-354 | CH₂OCH₂CH₂OEt | Et | Br | |
| 1-355 | CH₂OCH₂CH₂OEt | n-Pr | Br | |
| 1-356 | CH₂OCH₂CH₂OEt | i-Pr | Br | |
| 1-357 | CH₂OCH₂CH₂CH₂OMe | Me | Br | |
| 1-358 | CH₂OCH₂CH₂CH₂OMe | Et | Br | |
| 1-359 | CH₂OCH₂CH₂CH₂OMe | n-Pr | Br | |
| 1-360 | CH₂OCH₂CH₂CH₂OMe | i-Pr | Br | |
| 1-361 | CH₂OCH₂OMe | Me | Br | |
| 1-362 | CH₂OCH₂OMe | Et | Br | |
| 1-363 | CH₂OCH₂OMe | n-Pr | Br | |
| 1-364 | CH₂OCH₂OMe | i-Pr | Br | |
| 1-365 | CH₂OCH₂OEt | Me | Br | |
| 1-366 | CH₂OCH₂OEt | Et | Br | |
| 1-367 | CH₂OCH₂OEt | n-Pr | Br | |
| 1-368 | CH₂OCH₂OEt | i-Pr | Br | |
| 1-369 | CH₂OCH₂CH₂SO₂Me | Me | Br | |
| 1-370 | CH₂OCH₂CH₂SO₂Me | Et | Br | |
| 1-371 | CH₂OCH₂CH₂SO₂Me | n-Pr | Br | |
| 1-372 | CH₂OCH₂CH₂SO₂Me | i-Pr | Br | |
| 1-373 | CH₂SO₂CH₂CH₂OMe | Me | Br | |
| 1-374 | CH₂SO₂CH₂CH₂OMe | Et | Br | |
| 1-375 | CH₂SO₂CH₂CH₂OMe | n-Pr | Br | |
| 1-376 | CH₂SO₂CH₂CH₂OMe | i-Pr | Br | |
| 1-377 | CH₂SO₂CH₂CH₂SO₂Me | Me | Br | |
| 1-378 | CH₂SO₂CH₂CH₂SO₂Me | Et | Br | |
| 1-379 | CH₂SO₂CH₂CH₂SO₂Me | n-Pr | Br | |
| 1-380 | CH₂SO₂CH₂CH₂SO₂Me | i-Pr | Br | |
| 1-381 | Cl | Me | I | |
| 1-382 | Cl | Et | I | |
| 1-383 | Cl | n-Pr | I | |
| 1-384 | Cl | i-Pr | I | |
| 1-385 | Br | Me | I | |
| 1-386 | Br | Et | I | |
| 1-387 | Br | n-Pr | I | |
| 1-388 | Br | i-Pr | I | |
| 1-389 | Me | Me | I | 7.86 (d, 1H), 7.31 (s, 1H), 7.11 (d, 1H), 3.71 (s, 3H), 2.98 (s, 3H), 2.76 (s, 3H) |
| 1-390 | Me | Et | I | |
| 1-391 | Me | n-Pr | I | |
| 1-392 | Me | i-Pr | I | |
| 1-393 | Et | Me | I | |
| 1-394 | Et | Et | I | |
| 1-395 | Et | n-Pr | I | |
| 1-396 | Et | i-Pr | I | |
| 1-397 | CF₃ | Me | I | |
| 1-398 | CF₃ | Et | I | |
| 1-399 | CF₃ | n-Pr | I | |
| 1-400 | CF₃ | i-Pr | I | |
| 1-401 | OMe | Me | I | |
| 1-402 | OMe | Et | I | |
| 1-403 | OMe | n-Pr | I | |
| 1-404 | OMe | i-Pr | I | |
| 1-405 | OEt | Me | I | |
| 1-406 | OEt | Et | I | |
| 1-407 | OEt | n-Pr | I | |
| 1-408 | OEt | i-Pr | I | |
| 1-409 | NO₂ | Me | I | |
| 1-410 | NO₂ | Et | I | |
| 1-411 | NO₂ | n-Pr | I | |
| 1-412 | NO₂ | i-Pr | I | |
| 1-413 | SO₂Me | Me | I | |
| 1-414 | SO₂Me | Et | I | |
| 1-415 | SO₂Me | n-Pr | I | |
| 1-416 | SO₂Me | i-Pr | I | |
| 1-417 | CH₂OMe | Me | I | |
| 1-418 | CH₂OMe | Et | I | |
| 1-419 | CH₂OMe | n-Pr | I | |
| 1-420 | CH₂OMe | i-Pr | I | |
| 1-421 | CH₂SO₂Me | Me | I | |
| 1-422 | CH₂SO₂Me | Et | I | |
| 1-423 | CH₂SO₂Me | n-Pr | I | |
| 1-424 | CH₂SO₂Me | i-Pr | I | |
| 1-425 | CH₂OCH₂CH₂OMe | Me | I | |
| 1-426 | CH₂OCH₂CH₂OMe | Et | I | |
| 1-427 | CH₂OCH₂CH₂OMe | n-Pr | I | |
| 1-428 | CH₂OCH₂CH₂OMe | i-Pr | I | |
| 1-429 | CH₂OCH₂CH₂OEt | Me | I | |
| 1-430 | CH₂OCH₂CH₂OEt | Et | I | |
| 1-431 | CH₂OCH₂CH₂OEt | n-Pr | I | |
| 1-432 | CH₂OCH₂CH₂OEt | i-Pr | I | |
| 1-433 | CH₂OCH₂CH₂CH₂OMe | Me | I | |
| 1-434 | CH₂OCH₂CH₂CH₂OMe | Et | I | |
| 1-435 | CH₂OCH₂CH₂CH₂OMe | n-Pr | I | |
| 1-436 | CH₂OCH₂CH₂CH₂OMe | i-Pr | I | |
| 1-437 | CH₂OCH₂OMe | Me | I | |
| 1-438 | CH₂OCH₂OMe | Et | I | |
| 1-439 | CH₂OCH₂OMe | n-Pr | I | |
| 1-440 | CH₂OCH₂OMe | i-Pr | I | |
| 1-441 | CH₂OCH₂OEt | Me | I | |
| 1-442 | CH₂OCH₂OEt | Et | I | |
| 1-443 | CH₂OCH₂OEt | n-Pr | I | |
| 1-444 | CH₂OCH₂OEt | i-Pr | I | |
| 1-445 | CH₂OCH₂CH₂SO₂Me | Me | I | |
| 1-446 | CH₂OCH₂CH₂SO₂Me | Et | I | |
| 1-447 | CH₂OCH₂CH₂SO₂Me | n-Pr | I | |
| 1-448 | CH₂OCH₂CH₂SO₂Me | i-Pr | I | |
| 1-449 | CH₂SO₂CH₂CH₂OMe | Me | I | |
| 1-450 | CH₂SO₂CH₂CH₂OMe | Et | I | |
| 1-451 | CH₂SO₂CH₂CH₂OMe | n-Pr | I | |
| 1-452 | CH₂SO₂CH₂CH₂OMe | i-Pr | I | |
| 1-453 | CH₂SO₂CH₂CH₂SO₂Me | Me | I | |
| 1-454 | CH₂SO₂CH₂CH₂SO₂Me | Et | I | |
| 1-455 | CH₂SO₂CH₂CH₂SO₂Me | n-Pr | I | |
| 1-456 | CH₂SO₂CH₂CH₂SO₂Me | i-Pr | I | |
| 1-457 | Cl | Me | NO₂ | |
| 1-458 | Cl | Et | NO₂ | |
| 1-459 | Cl | n-Pr | NO₂ | |
| 1-460 | Cl | i-Pr | NO₂ | |
| 1-461 | Br | Me | NO₂ | |
| 1-462 | Br | Et | NO₂ | |
| 1-463 | Br | n-Pr | NO₂ | |
| 1-464 | Br | i-Pr | NO₂ | |
| 1-465 | Me | Me | NO₂ | |
| 1-466 | Me | Et | NO₂ | |
| 1-467 | Me | n-Pr | NO₂ | |
| 1-468 | Me | i-Pr | NO₂ | |
| 1-469 | Et | Me | NO₂ | |
| 1-470 | Et | Et | NO₂ | |
| 1-471 | Et | n-Pr | NO₂ | |
| 1-472 | Et | i-Pr | NO₂ | |
| 1-473 | CF₃ | Me | NO₂ | |
| 1-474 | CF₃ | Et | NO₂ | |
| 1-475 | CF₃ | n-Pr | NO₂ | |
| 1-476 | CF₃ | i-Pr | NO₂ | |
| 1-477 | OMe | Me | NO₂ | |
| 1-478 | OMe | Et | NO₂ | |
| 1-479 | OMe | n-Pr | NO₂ | |
| 1-480 | OMe | i-Pr | NO₂ | |
| 1-481 | OEt | Me | NO₂ | |
| 1-482 | OEt | Et | NO₂ | |
| 1-483 | OEt | n-Pr | NO₂ | |
| 1-484 | OEt | i-Pr | NO₂ | |
| 1-485 | NO₂ | Me | NO₂ | |
| 1-486 | NO₂ | Et | NO₂ | |
| 1-487 | NO₂ | n-Pr | NO₂ | |
| 1-488 | NO₂ | i-Pr | NO₂ | |
| 1-489 | SO₂Me | Me | NO₂ | |
| 1-490 | SO₂Me | Et | NO₂ | |
| 1-491 | SO₂Me | n-Pr | NO₂ | |
| 1-492 | SO₂Me | i-Pr | NO₂ | |
| 1-493 | CH₂OMe | Me | NO₂ | |
| 1-494 | CH₂OMe | Et | NO₂ | |
| 1-495 | CH₂OMe | n-Pr | NO₂ | |
| 1-496 | CH₂OMe | i-Pr | NO₂ | |
| 1-497 | CH₂SO₂me | Me | NO₂ | |
| 1-498 | CH₂SO₂Me | Et | NO₂ | |
| 1-499 | CH₂SO₂Me | n-Pr | NO₂ | |
| 1-500 | CH₂SO₂Me | i-Pr | NO₂ | |
| 1-501 | CH₂OCH₂CH₂OMe | Me | NO₂ | |
| 1-502 | CH₂OCH₂CH₂OMe | Et | NO₂ | |
| 1-503 | CH₂OCH₂CH₂OMe | n-Pr | NO₂ | |
| 1-504 | CH₂OCH₂CH₂OMe | i-Pr | NO₂ | |
| 1-505 | CH₂OCH₂CH₂OEt | Me | NO₂ | |
| 1-506 | CH₂OCH₂CH₂OEt | Et | NO₂ | |
| 1-507 | CH₂OCH₂CH₂OEt | n-Pr | NO₂ | |
| 1-508 | CH₂OCH₂CH₂OEt | i-Pr | NO₂ | |
| 1-509 | CH₂OCH₂CH₂CH₂OMe | Me | NO₂ | |
| 1-510 | CH₂OCH₂CH₂CH₂OMe | Et | NO₂ | |
| 1-511 | CH₂OCH₂CH₂CH₂OMe | n-Pr | NO₂ | |
| 1-512 | CH₂OCH₂CH₂CH₂OMe | i-Pr | NO₂ | |
| 1-513 | CH₂OCH₂OMe | Me | NO₂ | |
| 1-514 | CH₂OCH₂OMe | Et | NO₂ | |
| 1-515 | CH₂OCH₂OMe | n-Pr | NO₂ | |
| 1-516 | CH₂OCH₂OMe | i-Pr | NO₂ | |
| 1-517 | CH₂OCH₂OEt | Me | NO₂ | |
| 1-518 | CH₂OCH₂OEt | Et | NO₂ | |
| 1-519 | CH₂OCH₂OEt | n-Pr | NO₂ | |
| 1-520 | CH₂OCH₂OEt | i-Pr | NO₂ | |
| 1-521 | CH₂OCH₂CH₂SO₂Me | Me | NO₂ | |
| 1-522 | CH₂OCH₂CH₂SO₂Me | Et | NO₂ | |
| 1-523 | CH₂OCH₂CH₂SO₂Me | n-Pr | NO₂ | |
| 1-524 | CH₂OCH₂CH₂SO₂Me | i-Pr | NO₂ | |
| 1-525 | CH₂SO₂CH₂CH₂OMe | Me | NO₂ | |
| 1-526 | CH₂SO₂CH₂CH₂OMe | Et | NO₂ | |
| 1-527 | CH₂SO₂CH₂CH₂OMe | n-Pr | NO₂ | |
| 1-528 | CH₂SO₂CH₂CH₂OMe | i-Pr | NO₂ | |
| 1-529 | CH₂SO₂CH₂CH₂SO₂Me | Me | NO₂ | |
| 1-530 | CH₂SO₂CH₂CH₂SO₂Me | Et | NO*₂* | |
| 1-531 | CH₂SO₂CH₂CH₂SO₂Me | n-Pr | NO₂ | |
| 1-532 | CH₂SO₂CH₂CH₂SO₂Me | i-Pr | NO₂ | |

**Tabelle 2: Erfindungsgemäße Verbindungen der allgemeinen Formel (I), worin R¹ für Ethyl, R² und R⁴ jeweils für Wasserstoff stehen.**

| | | | | |
|---|---|---|---|---|
| 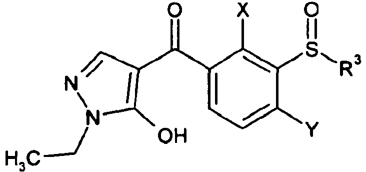 | | | | |

| Nr. | X | R³ | Y | Physikalische Daten: ¹H-NMR: δ [CDCl₃] |
|---|---|---|---|---|
| 2-1 | Cl | Me | SO₂Me | |
| 2-2 | Cl | Et | SO₂Me | |
| 2-3 | Cl | n-Pr | SO₂Me | |
| 2-4 | Cl | i-Pr | SO₂Me | |
| 2-5 | Br | Me | SO₂Me | |
| 2-6 | Br | Et | SO₂Me | |
| 2-7 | Br | n-Pr | SO₂Me | |
| 2-8 | Br | i-Pr | SO₂Me | |
| 2-9 | Me | Me | SO₂Me | 8.08 (d, 1H), 7.62 (d, 1H), 7.28 (s, 1H), 4.09 (q, 2H), 3.38 (s, 3H), 3.18 (s, 3H), 2.88 (s, 3H), 1.46 (t, 3H) |
| 2-10 | Me | Et | SO₂Me | 8.10 (d, 1H), 7.62 (d, 1H), 7.28 (s, 1H), 4.08 (q, 2H), 3.52 (m, 1H), 3.38 (s, 3H), 3.28 (m, 1H), 2.82 (s, 3H), 1.52 (t, 3H), 1.47 (t, 3H) |
| 2-11 | Me | n-Pr | SO₂Me | 8.08 (d, 1H), 7.61 (d, 1H), 7.27 (s, 1H), 4.08 (q, 2H), 3.52 (m, 1H), 3.37 (s, 3H), 3.16 (m, 1H), 2.83 (s, 3H), 2.02 (m, 2H), 1.47 (t, 3H), 1.17 (t, 3H) |
| 2-12 | Me | i-Pr | SO₂Me | 8.17 (m, 1H), 7.62 (d, 1H), 7.26 (s, 1H), 4.08 (q, 2H), 3.74 (m, 1H), 3.37 (s, 3H), 2.87 (s, 3H), 1.55 (d, 3H), 1.47 (t, 3H), 1.27 (m, 3H) |
| 2-13 | Et | Me | SO₂Me | |
| 2-14 | Et | Et | SO₂Me | |
| 2-15 | Et | n-Pr | SO₂Me | |
| 2-16 | Et | i-Pr | SO₂Me | |
| 2-17 | CF₃ | Me | SO₂Me | |
| 2-18 | CF₃ | Et | SO₂Me | |
| 2-19 | CF₃ | n-Pr | SO₂Me | |
| 2-20 | CF₃ | i-Pr | SO₂Me | |
| 2-21 | OMe | Me | SO₂Me | |
| 2-22 | OMe | Et | SO₂Me | |
| 2-23 | OMe | n-Pr | SO₂Me | |
| 2-24 | OMe | i-Pr | SO₂Me | |
| 2-25 | OEt | Me | SO₂Me | |
| 2-26 | OEt | Et | SO₂Me | |
| 2-27 | OEt | n-Pr | SO₂Me | |
| 2-28 | OEt | i-Pr | SO₂Me | |
| 2-29 | NO₂ | Me | SO₂Me | |
| 2-30 | NO₂ | Et | SO₂Me | |
| 2-31 | NO₂ | n-Pr | SO₂Me | |
| 2-32 | NO₂ | i-Pr | SO₂Me | |
| 2-33 | SO₂Me | Me | SO₂Me | |
| 2-34 | SO₂Me | Et | SO₂Me | |
| 2-35 | SO₂Me | n-Pr | SO₂Me | |
| 2-36 | SO₂Me | i-Pr | SO₂Me | |
| 2-37 | CH₂OMe | Me | SO₂Me | |
| 2-38 | CH₂OMe | Et | SO₂Me | |
| 2-39 | CH₂OMe | n-Pr | SO₂Me | |
| 2-40 | CH₂OMe | i-Pr | SO₂Me | |
| 2-41 | CH₂SO₂Me | Me | SO₂Me | |
| 2-42 | CH₂SO₂Me | Et | SO₂Me | |
| 2-43 | CH₂SO₂Me | n-Pr | SO₂Me | |
| 2-44 | CH₂SO₂Me | i-Pr | SO₂Me | |
| 2-45 | CH₂OCH₂CH₂OMe | Me | SO₂Me | |
| 2-46 | CH₂OCH₂CH₂OMe | Et | SO₂Me | |
| 2-47 | CH₂OCH₂CH₂OMe | n-Pr | SO₂Me | |
| 2-48 | CH₂OCH₂CH₂OMe | i-Pr | SO₂Me | |
| 2-49 | CH₂OCH₂CH₂OEt | Me | SO₂Me | 8.22 (d, 1H), 7.67 (d, 1H), 7.38 (s, 1H), 5.60 - 5.10 (br. Signal, 1H), 4.97 (d, 1H), 4.09 (q, 2H), 3.62 - 3.56 (m, 1H), 3.56 - 3.49 (m, 1H), 3.47 - 3.32 (m, 4H), 3.37 (s, 3H), 3.33 (s, 3H), 1.46 (t, 3H), 1.12 (t, 3H) |
| 2-50 | CH₂OCH₂CH₂OEt | Et | SO₂Me | |
| 2-51 | CH₂OCH₂CH₂OEt | n-Pr | SO₂Me | |
| 2-52 | CH₂OCH₂CH₂OEt | i-Pr | SO₂Me | |
| 2-53 | CH₂OCH₂CH₂CH₂OMe | Me | SO₂Me | |
| 2-54 | CH₂OCH₂CH₂CH₂OMe | Et | SO₂Me | |
| 2-55 | CH₂OCH₂CH₂CH₂OMe | n-Pr | SO₂Me | |
| 2-56 | CH₂OCH₂CH₂CH₂OMe | i-Pr | SO₂Me | |
| 2-57 | CH₂OCH₂OMe | Me | SO₂Me | |
| 2-58 | CH₂OCH₂OMe | Et | SO₂Me | |
| 2-59 | CH₂OCH₂OMe | n-Pr | SO₂Me | |
| 2-60 | CH₂OCH₂OMe | i-Pr | SO₂Me | |
| 2-61 | CH₂OCH₂OEt | Me | SO₂Me | |
| 2-62 | CH₂OCH₂OEt | Et | SO₂Me | |
| 2-63 | CH₂OCH₂OEt | n-Pr | SO₂Me | |
| 2-64 | CH₂OCH₂OEt | i-Pr | SO₂Me | |
| 2-65 | CH₂OCH₂CH₂SO₂Me | Me | SO₂Me | |
| 2-66 | CH₂OCH₂CH₂SO₂Me | Et | SO₂Me | |
| 2-67 | CH₂OCH₂CH₂SO₂Me | n-Pr | SO₂Me | |
| 2-68 | CH₂OCH₂CH₂SO₂Me | i-Pr | SO₂Me | |
| 2-69 | CH₂SO₂CH₂CH₂OMe | Me | SO₂Me | |
| 2-70 | CH₂SO₂CH₂CH₂OMe | Et | SO₂Me | |
| 2-71 | CH₂SO₂CH₂CH₂OMe | n-Pr | SO₂Me | |
| 2-72 | CH₂SO₂CH₂CH₂OMe | i-Pr | SO₂Me | |
| 2-73 | CH₂SO₂CH₂CH₂SO₂Me | Me | SO₂Me | |
| 2-74 | CH₂SO₂CH₂CH₂SO₂Me | Et | SO₂Me | |
| 2-75 | CH₂SO₂CH₂CH₂SO₂Me | n-Pr | SO₂Me | |
| 2-76 | CH₂SO₂CH₂CH₂SO₂Me | i-Pr | SO₂Me | |
| 2-77 | Cl | Me | SO₂Et | |
| 2-78 | Cl | Et | SO₂Et | |
| 2-79 | Cl | n-Pr | SO₂Et | |
| 2-80 | Cl | i-Pr | SO₂Et | |
| 2-81 | Br | Me | SO₂Et | |
| 2-82 | Br | Et | SO₂Et | |
| 2-83 | Br | n-Pr | SO₂Et | |
| 2-84 | Br | i-Pr | SO₂Et | |
| 2-85 | Me | Me | SO₂Et | |
| 2-86 | Me | Et | SO₂Et | |
| 2-87 | Me | n-Pr | SO₂Et | |
| 2-88 | Me | i-Pr | SO₂Et | |
| 2-89 | Et | Me | SO₂Et | |
| 2-90 | Et | Et | SO₂Et | |
| 2-91 | Et | n-Pr | SO₂Et | |
| 2-92 | Et | i-Pr | SO₂Et | |
| 2-93 | CF₃ | Me | SO₂Et | |
| 2-94 | CF₃ | Et | SO₂Et | |
| 2-95 | CF₃ | n-Pr | SO₂Et | |
| 2-96 | CF₃ | i-Pr | SO₂Et | |
| 2-97 | OMe | Me | SO₂Et | |
| 2-98 | OMe | Et | SO₂Et | |
| 2-99 | OMe | n-Pr | SO₂Et | |
| 2-100 | OMe | i-Pr | SO₂Et | |
| 2-101 | OEt | Me | SO₂Et | |
| 2-102 | OEt | Et | SO₂Et | |
| 2-103 | OEt | n-Pr | SO₂Et | |
| 2-104 | OEt | i-Pr | SO₂Et | |
| 2-105 | NO₂ | Me | SO₂Et | |
| 2-106 | NO₂ | Et | SO₂Et | |
| 2-107 | NO₂ | n-Pr | SO₂Et | |
| 2-108 | NO₂ | i-Pr | SO₂Et | |
| 2-109 | SO₂Me | Me | SO₂Et | |
| 2-110 | SO₂Me | Et | SO₂Et | |
| 2-111 | SO₂Me | n-Pr | SO₂Et | |
| 2-112 | SO₂Me | i-Pr | SO₂Et | |
| 2-113 | CH₂OMe | Me | SO₂Et | |
| 2-114 | CH₂OMe | Et | SO₂Et | |
| 2-115 | CH₂OMe | n-Pr | SO₂Et | |
| 2-116 | CH₂OMe | i-Pr | SO₂Et | |
| 2-117 | CH₂SO₂Me | Me | SO₂Et | |
| 2-118 | CH₂SO₂Me | Et | SO₂Et | |
| 2-119 | CH₂SO₂Me | n-Pr | SO₂Et | |
| 2-120 | CH₂SO₂Me | i-Pr | SO₂Et | |
| 2-121 | CH₂OCH₂CH₂OMe | Me | SO₂Et | |
| 2-122 | CH₂OCH₂CH₂OMe | Et | SO₂Et | |
| 2-123 | CH₂OCH₂CH₂OMe | n-Pr | SO₂Et | |
| 2-124 | CH₂OCH₂CH₂OMe | i-Pr | SO₂Et | |
| 2-125 | CH₂OCH₂CH₂OEt | Me | SO₂Et | |
| 2-126 | CH₂OCH₂CH₂OEt | Et | SO₂Et | |
| 2-127 | CH₂OCH₂CH₂OEt | n-Pr | SO₂Et | |
| 2-128 | CH₂OCH₂CH₂OEt | i-Pr | SO₂Et | |
| 2-129 | CH₂OCH₂CH₂CH₂OMe | Me | SO₂Et | |
| 2-130 | CH₂OCH₂CH₂CH₂OMe | Et | SO₂Et | |
| 2-131 | CH₂OCH₂CH₂CH₂OMe | n-Pr | SO₂Et | |
| 2-132 | CH₂OCH₂CH₂CH₂OMe | i-Pr | SO₂Et | |
| 2-133 | CH₂OCH₂OMe | Me | SO₂Et | |
| 2-134 | CH₂OCH₂OMe | Et | SO₂Et | |
| 2-135 | CH₂OCH₂OMe | n-Pr | SO₂Et | |
| 2-136 | CH₂OCH₂OMe | i-Pr | SO₂Et | |
| 2-137 | CH₂OCH₂OEt | Me | SO₂Et | |
| 2-138 | CH₂OCH₂OEt | Et | SO₂Et | |
| 2-139 | CH₂OCH₂OEt | n-Pr | SO₂Et | |
| 2-140 | CH₂OCH₂OEt | i-Pr | SO₂Et | |
| 2-141 | CH₂OCH₂CH₂SO₂Me | Me | SO₂Et | |
| 2-142 | CH₂OCH₂CH₂SO₂Me | Et | SO₂Et | |
| 2-143 | CH₂OCH₂CH₂SO₂Me | n-Pr | SO₂Et | |
| 2-144 | CH₂OCH₂CH₂SO₂Me | i-Pr | SO₂Et | |
| 2-145 | CH₂SO₂CH₂CH₂OMe | Me | SO₂Et | |
| 2-146 | CH₂SO₂CH₂CH₂OMe | Et | SO₂Et | |
| 2-147 | CH₂SO₂CH₂CH₂OMe | n-Pr | SO₂Et | |
| 2-148 | CH₂SO₂CH₂CH₂OMe | i-Pr | SO₂Et | |
| 2-149 | CH₂SO₂CH₂CH₂SO₂Me | Me | SO₂Et | |
| 2-150 | CH₂SO₂CH₂CH₂SO₂Me | Et | SO₂Et | |
| 2-151 | CH₂SO₂CH₂CH₂SO₂Me | n-Pr | SO₂Et | |
| 2-152 | CH₂SO₂CH₂CH₂SO₂Me | i-Pr | SO₂Et | |
| 2-153 | Cl | Me | F | |
| 2-154 | Cl | Et | F | |
| 2-155 | Cl | n-Pr | F | |
| 2-156 | Cl | i-Pr | F | |
| 2-157 | Br | Me | F | |
| 2-158 | Br | Et | F | |
| 2-159 | Br | n-Pr | F | |
| 2-160 | Br | i-Pr | F | |
| 2-161 | Me | Me | F | |
| 2-162 | Me | Et | F | |
| 2-163 | Me | n-Pr | F | |
| 2-164 | Me | i-Pr | F | |
| 2-165 | Et | Me | F | |
| 2-166 | Et | Et | F | |
| 2-167 | Et | n-Pr | F | |
| 2-168 | Et | i-Pr | F | |
| 2-169 | CF₃ | Me | F | |
| 2-170 | CF₃ | Et | F | |
| 2-171 | CF₃ | n-Pr | F | |
| 2-172 | CF₃ | i-Pr | F | |
| 2-173 | OMe | Me | F | |
| 2-174 | OMe | Et | F | |
| 2-175 | OMe | n-Pr | F | |
| 2-176 | OMe | i-Pr | F | |
| 2-177 | OEt | Me | F | |
| 2-178 | OEt | Et | F | |
| 2-179 | OEt | n-Pr | F | |
| 2-180 | OEt | i-Pr | F | |
| 2-181 | NO₂ | Me | F | |
| 2-182 | NO₂ | Et | F | |
| 2-183 | NO₂ | n-Pr | F | |
| 2-184 | NO₂ | i-Pr | F | |
| 2-185 | SO₂Me | Me | F | |
| 2-186 | SO₂Me | Et | F | |
| 2-187 | SO₂Me | n-Pr | F | |
| 2-188 | SO₂Me | i-Pr | F | |
| 2-189 | CH₂OMe | Me | F | |
| 2-190 | CH₂OMe | Et | F | |
| 2-191 | CH₂OMe | n-Pr | F | |
| 2-192 | CH₂OMe | i-Pr | F | |
| 2-193 | CH₂SO₂Me | Me | F | |
| 2-194 | CH₂SO₂Me | Et | F | |
| 2-195 | CH₂SO₂Me | n-Pr | F | |
| 2-196 | CH₂SO₂Me | i-Pr | F | |
| 2-197 | CH₂OCH₂CH₂OMe | Me | F | |
| 2-198 | CH₂OCH₂CH₂OMe | Et | F | |
| 2-199 | CH₂OCH₂CH₂OMe | n-Pr | F | |
| 2-200 | CH₂OCH₂CH₂OMe | i-Pr | F | |
| 2-201 | CH₂OCH₂CH₂OEt | Me | F | |
| 2-202 | CH₂OCH₂CH₂OEt | Et | F | |
| 2-203 | CH₂OCH₂CH₂OEt | n-Pr | F | |
| 2-204 | CH₂OCH₂CH₂OEt | i-Pr | F | |
| 2-205 | CH₂OCH₂CH₂CH₂OMe | Me | F | |
| 2-206 | CH₂OCH₂CH₂CH₂OMe | Et | F | |
| 2-207 | CH₂OCH₂CH₂CH₂OMe | n-Pr | F | |
| 2-208 | CH₂OCH₂CH₂CH₂OMe | i-Pr | F | |
| 2-209 | CH₂OCH₂OMe | Me | F | |
| 2-210 | CH₂OCH₂OMe | Et | F | |
| 2-211 | CH₂OCH₂OMe | n-Pr | F | |
| 2-212 | CH₂OCH₂OMe | i-Pr | F | |
| 2-213 | CH₂OCH₂OEt | Me | F | |
| 2-214 | CH₂OCH₂OEt | Et | F | |
| 2-215 | CH₂OCH₂OEt | n-Pr | F | |
| 2-216 | CH₂OCH₂OEt | i-Pr | F | |
| 2-217 | CH₂OCH₂CH₂SO₂Me | Me | F | |
| 2-218 | CH₂OCH₂CH₂SO₂Me | Et | F | |
| 2-219 | CH₂OCH₂CH₂SO₂Me | n-Pr | F | |
| 2-220 | CH₂OCH₂CH₂SO₂Me | i-Pr | F | |
| 2-221 | CH₂SO₂CH₂CH₂OMe | Me | F | |
| 2-222 | CH₂SO₂CH₂CH₂OMe | Et | F | |
| 2-223 | CH₂SO₂CH₂CH₂OMe | n-Pr | F | |
| 2-224 | CH₂SO₂CH₂CH₂OMe | i-Pr | F | |
| 2-225 | CH₂SO₂CH₂CH₂SO₂Me | Me | F | |
| 2-226 | CH₂SO₂CH₂CH₂SO₂Me | Et | F | |
| 2-227 | CH₂SO₂CH₂CH₂SO₂Me | n-Pr | F | |
| 2-228 | CH₂SO₂CH₂CH₂SO₂Me | i-Pr | F | |
| 2-229 | Cl | Me | Cl | |
| 2-230 | Cl | Et | Cl | |
| 2-231 | Cl | n-Pr | Cl | |
| 2-232 | Cl | i-Pr | Cl | |
| 2-233 | Br | Me | Cl | |
| 2-234 | Br | Et | Cl | |
| 2-235 | Br | n-Pr | Cl | |
| 2-236 | Br | i-Pr | Cl | |
| 2-237 | Me | Me | Cl | 7.43 (d, 1H), 7.37 (d, 1H), 7.33 (s, 1H), 4.08 (q, 2H), 3.03 (s, 3H), 2.73 (s, 3H), 1.45 (t, 3H) |
| 2-238 | Me | Et | Cl | 7.41 (d, 1H), 7.36 (d, 1 H), 7.32 (s, 1H), 4.07 (q, 2H), 3.38 (m, 1H), 3.17 (m, 1H), 2.72 (s, 3H), 1.46 (t, 3H), 1.41 (t, 3H) |
| 2-239 | Me | n-Pr | Cl | |
| 2-240 | Me | i-Pr | Cl | |
| 2-241 | Et | Me | Cl | |
| 2-242 | Et | Et | Cl | |
| 2-243 | Et | n-Pr | Cl | |
| 2-244 | Et | i-Pr | Cl | |
| 2-245 | CF₃ | Me | Cl | |
| 2-246 | CF₃ | Et | Cl | |
| 2-247 | CF₃ | n-Pr | Cl | |
| 2-248 | CF₃ | i-Pr | Cl | |
| 2-249 | OMe | Me | Cl | |
| 2-250 | OMe | Et | Cl | |
| 2-251 | OMe | n-Pr | Cl | |
| 2-252 | OMe | i-Pr | Cl | |
| 2-253 | OEt | Me | Cl | |
| 2-254 | OEt | Et | Cl | |
| 2-255 | OEt | n-Pr | Cl | |
| 2-256 | OEt | i-Pr | Cl | |
| 2-257 | NO₂ | Me | Cl | |
| 2-258 | NO₂ | Et | Cl | |
| 2-259 | NO₂ | n-Pr | Cl | |
| 2-260 | NO₂ | i-Pr | Cl | |
| 2-261 | SO₂Me | Me | Cl | |
| 2-262 | SO₂Me | Et | Cl | |
| 2-263 | SO₂Me | n-Pr | Cl | |
| 2-264 | SO₂Me | i-Pr | Cl | |
| 2-265 | CH₂OMe | Me | Cl | |
| 2-266 | CH₂OMe | Et | Cl | |
| 2-267 | CH₂OMe | n-Pr | Cl | |
| 2-268 | CH₂OMe | i-Pr | Cl | |
| 2-269 | CH₂SO₂Me | Me | Cl | |
| 2-270 | CH₂SO₂Me | Et | Cl | |
| 2-271 | CH₂SO₂Me | n-Pr | Cl | |
| 2-272 | CH₂SO₂Me | i-Pr | Cl | |
| 2-273 | CH₂OCH₂CH₂OMe | Me | Cl | |
| 2-274 | CH₂OCH₂CH₂OMe | Et | Cl | |
| 2-275 | CH₂OCH₂CH₂OMe | n-Pr | Cl | |
| 2-276 | CH₂OCH₂CH₂OMe | i-Pr | Cl | |
| 2-277 | CH₂OCH₂CH₂OEt | Me | Cl | |
| 2-278 | CH₂OCH₂CH₂OEt | Et | Cl | |
| 2-279 | CH₂OCH₂CH₂OEt | n-Pr | Cl | |
| 2-280 | CH₂OCH₂CH₂OEt | i-Pr | Cl | |
| 2-281 | CH₂OCH₂CH₂CH₂OMe | Me | Cl | |
| 2-282 | CH₂OCH₂CH₂CH₂OMe | Et | Cl | |
| 2-283 | CH₂OCH₂CH₂CH₂OMe | n-Pr | Cl | |
| 2-284 | CH₂OCH₂CH₂CH₂OMe | i-Pr | Cl | |
| 2-285 | CH₂OCH₂OMe | Me | Cl | |
| 2-286 | CH₂OCH₂OMe | Et | Cl | |
| 2-287 | CH₂OCH₂OMe | n-Pr | Cl | |
| 2-288 | CH₂OCH₂OMe | i-Pr | Cl | |
| 2.289 | CH₂OCH₂OEt | Me | Cl | |
| 2-290 | CH₂OCH₂OEt | Et | Cl | |
| 2-291 | CH₂OCH₂OEt | n-Pr | Cl | |
| 2-292 | CH₂OCH₂OEt | i-Pr | Cl | |
| 2-293 | CH₂OCH₂CH₂SO₂Me | Me | Cl | |
| 2-294 | CH₂OCH₂CH₂SO₂Me | Et | Cl | |
| 2-295 | CH₂OCH₂CH₂SO₂Me | n-Pr | Cl | |
| 2-296 | CH₂OCH₂CH₂SO₂Me | i-Pr | Cl | |
| 2-297 | CH₂SO₂CH₂CH₂OMe | Me | Cl | |
| 2-298 | CH₂SO₂CH₂CH₂OMe | Et | Cl | |
| 2-299 | CH₂SO₂CH₂CH₂OMe | n-Pr | Cl | |
| 2-300 | CH₂SO₂CH₂CH₂OMe | i-Pr | Cl | |
| 2-301 | CH₂SO₂CH₂CH₂SO₂Me | Me | Cl | |
| 2-302 | CH₂SO₂CH₂CH₂SO₂Me | Et | Cl | |
| 2-303 | CH₂SO₂CH₂CH₂SO₂Me | n-Pr | Cl | |
| 2-304 | CH₂SO₂CH₂CH₂SO₂Me | i-Pr | Cl | |
| 2-305 | Cl | Me | Br | |
| 2-306 | Cl | Et | Br | |
| 2-307 | Cl | n-Pr | Br | |
| 2-308 | Cl | i-Pr | Br | |
| 2-309 | Br | Me | Br | |
| 2-310 | Br | Et | Br | |
| 2-311 | Br | n-Pr | Br | |
| 2-312 | Br | i-Pr | Br | |
| 2-313 | Me | Me | Br | 7.56 (d, 1H), 7.35 (s, 1H), 7.33 (d, 1H), 4.09 (q, 2H), 3.03 (s, 3H), 2.77 (s, 3H), 1.46 (t, 3H) |
| 2-314 | Me | Et | Br | |
| 2-315 | Me | n-Pr | Br | |
| 2-316 | Me | i-Pr | Br | |
| 2-317 | Et | Me | Br | |
| 2-318 | Et | Et | Br | |
| 2-319 | Et | n-Pr | Br | |
| 2-320 | Et | i-Pr | Br | |
| 2-321 | CF₃ | Me | Br | |
| 2-322 | CF₃ | Et | Br | |
| 2-323 | CF₃ | n-Pr | Br | |
| 2-324 | CF₃ | i-Pr | Br | |
| 2-325 | OMe | Me | Br | |
| 2-326 | OMe | Et | Br | |
| 2-327 | OMe | n-Pr | Br | |
| 2-328 | OMe | i-Pr | Br | |
| 2-329 | OEt | Me | Br | |
| 2-330 | OEt | Et | Br | |
| 2-331 | OEt | n-Pr | Br | |
| 2-332 | OEt | i-Pr | Br | |
| 2-333 | NO₂ | Me | Br | |
| 2-334 | NO₂ | Et | Br | |
| 2-335 | NO₂ | n-Pr | Br | |
| 2-336 | NO₂ | i-Pr | Br | |
| 2-337 | SO₂Me | Me | Br | |
| 2-338 | SO₂Me | Et | Br | |
| 2-339 | SO₂Me | n-Pr | Br | |
| 2-340 | SO₂Me | i-Pr | Br | |
| 2-341 | CH₂OMe | Me | Br | |
| 2-342 | CH₂OMe | Et | Br | |
| 2-343 | CH₂OMe | n-Pr | Br | |
| 2-344 | CH₂OMe | i-Pr | Br | |
| 2-345 | CH₂SO₂Me | Me | Br | |
| 2-346 | CH₂SO₂Me | Et | Br | |
| 2-347 | CH₂SO₂Me | n-Pr | Br | |
| 2-348 | CH₂SO₂Me | i-Pr | Br | |
| 2-349 | CH₂OCH₂CH₂OMe | Me | Br | |
| 2-350 | CH₂OCH₂CH₂OMe | Et | Br | |
| 2-351 | CH₂OCH₂CH₂OMe | n-Pr | Br | |
| 2-352 | CH₂OCH₂CH₂OMe | i-Pr | Br | |
| 2-353 | CH₂OCH₂CH₂OEt | Me | Br | |
| 2-354 | CH₂OCH₂CH₂OEt | Et | Br | |
| 2-355 | CH₂OCH₂CH₂OEt | n-Pr | Br | |
| 2-356 | CH₂OCH₂CH₂OEt | i-Pr | Br | |
| 2-357 | CH₂OCH₂CH₂CH₂OMe | Me | Br | |
| 2-358 | CH₂OCH₂CH₂CH₂OMe | Et | Br | |
| 2-359 | CH₂OCH₂CH₂CH₂OMe | n-Pr | Br | |
| 2-360 | CH₂OCH₂CH₂CH₂OMe | i-Pr | Br | |
| 2-361 | CH₂OCH₂OMe | Me | Br | |
| 2-362 | CH₂OCH₂OMe | Et | Br | |
| 2-363 | CH₂OCH₂OMe | n-Pr | Br | |
| 2-364 | CH₂OCH₂OMe | i-Pr | Br | |
| 2-365 | CH₂OCH₂OEt | Me | Br | |
| 2-366 | CH₂OCH₂OEt | Et | Br | |
| 2-367 | CH₂OCH₂OEt | n-Pr | Br | |
| 2-368 | CH₂OCH₂OEt | i-Pr | Br | |
| 2-369 | CH₂OCH₂CH₂SO₂Me | Me | Br | |
| 2-370 | CH₂OCH₂CH₂SO₂Me | Et | Br | |
| 2-371 | CH₂OCH₂CH₂SO₂Me | n-Pr | Br | |
| 2-372 | CH₂OCH₂CH₂SO₂Me | i-Pr | Br | |
| 2-373 | CH₂SO₂CH₂CH₂OMe | Me | Br | |
| 2-374 | CH₂SO₂CH₂CH₂OMe | Et | Br | |
| 2-375 | CH₂SO₂CH₂CH₂OMe | n-Pr | Br | |
| 2-376 | CH₂SO₂CH₂CH₂OMe | i-Pr | Br | |
| 2-377 | CH₂SO₂CH₂CH₂SO₂Me | Me | Br | |
| 2-378 | CH₂SO₂CH₂CH₂SO₂Me | Et | Br | |
| 2-379 | CH₂SO₂CH₂CH₂SO₂Me | n-Pr | Br | |
| 2-380 | CH₂SO₂CH₂CH₂SO₂Me | i-Pr | Br | |
| 2-381 | Cl | Me | I | |
| 2-382 | Cl | Et | I | |
| 2-383 | Cl | n-Pr | I | |
| 2-384 | Cl | i-Pr | I | |
| 2-385 | Br | Me | I | |
| 2-386 | Br | Et | I | |
| 2-387 | Br | n-Pr | I | |
| 2-388 | Br | i-Pr | I | |
| 2-389 | Me | Me | I | 7.87 (d, 1H), 7.31 (s, 1H), 7.12 (d, 1H), 4.08 (q, 2H), 2.97 (s, 3H), 2.77 (s, 3H), 1.45 (t, 3H) |
| 2-390 | Me | Et | I | |
| 2-391 | Me | n-Pr | I | |
| 2-392 | Me | i-Pr | I | |
| 2-393 | Et | Me | I | |
| 2-394 | Et | Et | I | |
| 2-395 | Et | n-Pr | I | |
| 2-396 | Et | i-Pr | I | |
| 2-397 | CF₃ | Me | I | |
| 2-398 | CF₃ | Et | I | |
| 2-399 | CF₃ | n-Pr | I | |
| 2-400 | CF₃ | i-Pr | I | |
| 2-401 | OMe | Me | I | |
| 2-402 | OMe | Et | I | |
| 2-403 | OMe | n-Pr | I | |
| 2-404 | OMe | i-Pr | I | |
| 2-405 | OEt | Me | I | |
| 2-406 | OEt | Et | I | |
| 2-407 | OEt | n-Pr | I | |
| 2-408 | OEt | i-Pr | I | |
| 2-409 | NO₂ | Me | I | |
| 2-410 | NO₂ | Et | I | |
| 2-411 | NO₂ | n-Pr | I | |
| 2-412 | NO₂ | i-Pr | I | |
| 2-413 | SO₂Me | Me | I | |
| 2-414 | SO₂Me | Et | I | |
| 2-415 | SO₂Me | n-Pr | I | |
| 2-416 | SO₂Me | i-Pr | I | |
| 2-417 | CH₂OMe | Me | I | |
| 2-418 | CH₂OMe | Et | I | |
| 2-419 | CH₂OMe | n-Pr | I | |
| 2-420 | CH₂OMe | i-Pr | I | |
| 2-421 | CH₂SO₂Me | Me | I | |
| 2-422 | CH₂SO₂Me | Et | I | |
| 2-423 | CH₂SO₂Me | n-Pr | I | |
| 2-424 | CH₂SO₂Me | i-Pr | I | |
| 2-425 | CH₂OCH₂CH₂OMe | Me | I | |
| 2-426 | CH₂OCH₂CH₂OMe | Et | I | |
| 2-427 | CH₂OCH₂CH₂OMe | n-Pr | I | |
| 2-428 | CH₂OCH₂CH₂OMe | i-Pr | I | |
| 2-429 | CH₂OCH₂CH₂OEt | Me | I | |
| 2-430 | CH₂OCH₂CH₂OEt | Et | I | |
| 2-431 | CH₂OCH₂CH₂OEt | n-Pr | I | |
| 2-432 | CH₂OCH₂CH₂OEt | i-Pr | I | |
| 2-433 | CH₂OCH₂CH₂CH₂OMe | Me | I | |
| 2-434 | CH₂OCH₂CH₂CH₂OMe | Et | I | |
| 2-435 | CH₂OCH₂CH₂CH₂OMe | n-Pr | I | |
| 2-436 | CH₂OCH₂CH₂CH₂OMe | i-Pr | I | |
| 2-437 | CH₂OCH₂OMe | Me | I | |
| 2-438 | CH₂OCH₂OMe | Et | I | |
| 2-439 | CH₂OCH₂OMe | n-Pr | I | |
| 2-440 | CH₂OCH₂OMe | i-Pr | I | |
| 2-441 | CH₂OCH₂OEt | Me | I | |
| 2-442 | CH₂OCH₂OEt | Et | I | |
| 2-443 | CH₂OCH₂OEt | n-Pr | I | |
| 2-444 | CH₂OCH₂OEt | i-Pr | I | |
| 2-445 | CH₂OCH₂CH₂SO₂Me | Me | I | |
| 2-446 | CH₂OCH₂CH₂SO₂Me | Et | I | |
| 2-447 | CH₂OCH₂CH₂SO₂Me | n-Pr | I | |
| 2-448 | CH₂OCH₂CH₂SO₂Me | i-Pr | I | |
| 2-449 | CH₂SO₂CH₂CH₂OMe | Me | I | |
| 2-450 | CH₂SO₂CH₂CH₂OMe | Et | I | |
| 2-451 | CH₂SO₂CH₂CH₂OMe | n-Pr | I | |
| 2-452 | CH₂SO₂CH₂CH₂OMe | i-Pr | I | |
| 2-453 | CH₂SO₂CH₂CH₂SO₂Me | Me | I | |
| 2-454 | CH₂SO₂CH₂CH₂SO₂Me | Et | I | |
| 2-455 | CH₂SO₂CH₂CH₂SO₂Me | n-Pr | I | |
| 2-456 | CH₂SO₂CH₂CH₂SO₂Me | i-Pr | I | |
| 2-457 | Cl | Me | NO₂ | |
| 2-458 | Cl | Et | NO₂ | |
| 2-459 | Cl | n-Pr | NO₂ | |
| 2-460 | Cl | i-Pr | NO₂ | |
| 2-461 | Br | Me | NO₂ | |
| 2-462 | Br | Et | NO₂ | |
| 2-463 | Br | n-Pr | NO₂ | |
| 2-464 | Br | i-Pr | NO₂ | |
| 2-465 | Me | Me | NO₂ | |
| 2-466 | Me | Et | NO₂ | |
| 2-467 | Me | n-Pr | NO₂ | |
| 2-468 | Me | i-Pr | NO₂ | |
| 2-469 | Et | Me | NO₂ | |
| 2-470 | Et | Et | NO₂ | |
| 2-471 | Et | n-Pr | NO₂ | |
| 2-472 | Et | i-Pr | NO₂ | |
| 2-473 | CF₃ | Me | NO₂ | |
| 2-474 | CF₃ | Et | NO₂ | |
| 2-475 | CF₃ | n-Pr | NO₂ | |
| 2-476 | CF₃ | i-Pr | NO₂ | |
| 2-477 | OMe | Me | NO₂ | |
| 2-478 | OMe | Et | NO₂ | |
| 2-479 | OMe | n-Pr | NO₂ | |
| 2-480 | OMe | i-Pr | NO₂ | |
| 2-481 | OEt | Me | NO₂ | |
| 2-482 | OEt | Et | NO₂ | |
| 2-483 | OEt | n-Pr | NO₂ | |
| 2-484 | OEt | i-Pr | NO₂ | |
| 2-485 | NO₂ | Me | NO₂ | |
| 2-486 | NO₂ | Et | NO₂ | |
| 2-487 | NO₂ | n-Pr | NO₂ | |
| 2-488 | NO₂ | i-Pr | NO₂ | |
| 2-489 | SO₂Me | Me | NO₂ | |
| 2-490 | SO₂Me | Et | NO₂ | |
| 2-491 | SO₂Me | n-Pr | NO₂ | |
| 2-492 | SO₂Me | i-Pr | NO₂ | |
| 2-493 | CH₂OMe | Me | NO₂ | |
| 2-494 | CH₂OMe | Et | NO₂ | |
| 2-495 | CH₂OMe | n-Pr | NO₂ | |
| 2-496 | CH₂OMe | i-Pr | NO₂ | |
| 2-497 | CH₂SO₂Me | Me | NO₂ | |
| 2-498 | CH₂SO₂Me | Et | NO₂ | |
| 2-499 | CH₂SO₂Me | n-Pr | NO₂ | |
| 2-500 | CH₂SO₂Me | i-Pr | NO₂ | |
| 2-501 | CH₂OCH₂CH₂OMe | Me | NO₂ | |
| 2-502 | CH₂OCH₂CH₂OMe | Et | NO₂ | |
| 2-503 | CH₂OCH₂CH₂OMe | n-Pr | NO₂ | |
| 2-504 | CH₂OCH₂CH₂OMe | i-Pr | NO₂ | |
| 2-505 | CH₂OCH₂CH₂OEt | Me | NO₂ | |
| 2-506 | CH₂OCH₂CH₂OEt | Et | NO₂ | |
| 2-507 | CH₂OCH₂CH₂OEt | n-Pr | NO₂ | |
| 2-508 | CH₂OCH₂CH₂OEt | i-Pr | NO₂ | |
| 2-509 | CH₂OCH₂CH₂CH₂OMe | Me | NO₂ | |
| 2-510 | CH₂OCH₂CH₂CH₂OMe | Et | NO₂ | |
| 2-511 | CH₂OCH₂CH₂CH₂OMe | n-Pr | NO₂ | |
| 2-512 | CH₂OCH₂CH₂CH₂OMe | i-Pr | NO₂ | |
| 2-513 | CH₂OCH₂OMe | Me | NO₂ | |
| 2-514 | CH₂OCH₂OMe | Et | NO₂ | |
| 2-515 | CH₂OCH₂OMe | n-Pr | NO₂ | |
| 2-516 | CH₂OCH₂OMe | i-Pr | NO₂ | |
| 2-517 | CH₂OCH₂OEt | Me | NO₂ | |
| 2-518 | CH₂OCH₂OEt | Et | NO₂ | |
| 2-519 | CH₂OCH₂OEt | n-Pr | NO₂ | |
| 2-520 | CH₂OCH₂OEt | i-Pr | NO₂ | |
| 2-521 | CH₂OCH₂CH₂SO₂Me | Me | NO₂ | |
| 2-522 | CH₂OCH₂CH₂SO₂Me | Et | NO₂ | |
| 2-523 | CH₂OCH₂CH₂SO₂Me | n-Pr | NO₂ | |
| 2-524 | CH₂OCH₂CH₂SO₂Me | i-Pr | NO₂ | |
| 2-525 | CH₂SO₂CH₂CH₂OMe | Me | NO₂ | |
| 2-526 | CH₂SO₂CH₂CH₂OMe | Et | NO₂ | |
| 2-527 | CH₂SO₂CH₂CH₂OMe | n-Pr | NO₂ | |
| 2-528 | CH₂SO₂CH₂CH₂OMe | i-Pr | NO₂ | |
| 2-529 | CH₂SO₂CH₂CH₂SO₂Me | Me | NO₂ | |
| 2-530 | CH₂SO₂CH₂CH₂SO₂Me | Et | NO₂ | |
| 2-531 | CH₂SO₂CH₂CH₂SO₂Me | n-Pr | NO₂ | |
| 2-532 | CH₂SO₂CH₂CH₂SO₂Me | i-Pr | NO₂ | |

**Tabelle 3: Erfindungsgemäße Verbindungen der allgemeinen Formel (I), worin R⁴ für Wasserstoff sowie R¹ und R² jeweils für Methyl, stehen.**

| | | | | |
|---|---|---|---|---|
| 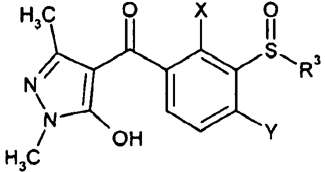 | | | | |

| Nr. | X | R³ | Y | Physikalische Daten: ¹H-NMR: δ [CDCl₃] |
|---|---|---|---|---|
| 3-1 | Cl | Me | SO₂Me | |
| 3-2 | Cl | Et | SO₂Me | |
| 3-3 | Cl | n-Pr | SO₂Me | |
| 3-4 | Cl | i-Pr | SO₂Me | |
| 3-5 | Br | Me | SO₂Me | |
| 3-6 | Br | Et | SO₂Me | |
| 3-7 | Br | n-Pr | SO₂Me | |
| 3-8 | Br | i-Pr | SO₂Me | |
| 3-9 | Me | Me | SO₂Me | 8.09 (d, 1 H), 7.49 (d, 1 H), 3.64 (s, 3H), 3.38 (s, 3H), 3.18 (s, 3H), 2.81 (s, 3H), 1.69 (s, 3H) |
| 3-10 | Me | Et | SO₂Me | |
| 3-11 | Me | n-Pr | SO₂Me | |
| 3-12 | Me | i-Pr | SO₂Me | |
| 3-13 | Et | Me | SO₂Me | |
| 3-14 | Et | Et | SO₂Me | |
| 3-15 | Et | n-Pr | SO₂Me | |
| 3-16 | Et | i-Pr | SO₂Me | |
| 3-17 | CF₃ | Me | SO₂Me | |
| 3-18 | CF₃ | Et | SO₂Me | |
| 3-19 | CF₃ | n-Pr | SO₂Me | |
| 3-20 | CF₃ | i-Pr | SO₂Me | |
| 3-21 | OMe | Me | SO₂Me | |
| 3-22 | OMe | Et | SO₂Me | |
| 3-23 | OMe | n-Pr | SO₂Me | |
| 3-24 | OMe | i-Pr | SO₂Me | |
| 3-25 | OEt | Me | SO₂Me | |
| 3-26 | OEt | Et | SO₂Me | |
| 3-27 | OEt | n-Pr | SO₂Me | |
| 3-28 | OEt | i-Pr | SO₂Me | |
| 3-29 | NO₂ | Me | SO₂Me | |
| 3-30 | NO₂ | Et | SO₂Me | |
| 3-31 | NO₂ | n-Pr | SO₂Me | |
| 3-32 | NO₂ | i-Pr | SO₂Me | |
| 3-33 | SO₂Me | Me | SO₂Me | |
| 3-34 | SO₂Me | Et | SO₂Me | |
| 3-35 | SO₂Me | n-Pr | SO₂Me | |
| 3-36 | SO₂Me | i-Pr | SO₂Me | |
| 3-37 | CH₂OMe | Me | SO₂Me | |
| 3-38 | CH₂OMe | Et | SO₂Me | |
| 3-39 | CH₂OMe | n-Pr | SO₂Me | |
| 3-40 | CH₂OMe | i-Pr | SO₂Me | |
| 3-41 | CH₂SO₂Me | Me | SO₂Me | |
| 3-42 | CH₂SO₂Me | Et | SO₂Me | |
| 3-43 | CH₂SO₂Me | n-Pr | SO₂Me | |
| 3-44 | CH₂SO₂Me | i-Pr | SO₂Me | |
| 3-45 | CH₂OCH₂CH₂OMe | Me | SO₂Me | |
| 3-46 | CH₂OCH₂CH₂OMe | Et | SO₂Me | |
| 3-47 | CH₂OCH₂CH₂OMe | n-Pr | SO₂Me | |
| 3-48 | CH₂OCH₂CH₂OMe | i-Pr | SO₂Me | |
| 3-49 | CH₂OCH₂CH₂OEt | Me | SO₂Me | |
| 3-50 | CH₂OCH₂CH₂OEt | Et | SO₂Me | |
| 3-51 | CH₂OCH₂CH₂OEt | n-Pr | SO₂Me | |
| 3-52 | CH₂OCH₂CH₂OEt | i-Pr | SO₂Me | |
| 3-53 | CH₂OCH₂CH₂CH₂OMe | Me | SO₂Me | |
| 3-54 | CH₂OCH₂CH₂CH₂OMe | Et | SO₂Me | |
| 3-55 | CH₂OCH₂CH₂CH₂OMe | n-Pr | SO₂Me | |
| 3-56 | CH₂OCH₂CH₂CH₂OMe | i-Pr | SO₂Me | |
| 3-57 | CH₂OCH₂OMe | Me | SO₂Me | |
| 3-58 | CH₂OCH₂OMe | Et | SO₂Me | |
| 3-59 | CH₂OCH₂OMe | n-Pr | SO₂Me | |
| 3-60 | CH₂OCH₂OMe | i-Pr | SO₂Me | |
| 3-61 | CH₂OCH₂OEt | Me | SO₂Me | |
| 3-62 | CH₂OCH₂OEt | Et | SO₂Me | |
| 3-63 | CH₂OCH₂OEt | n-Pr | SO₂Me | |
| 3-64 | CH₂OCH₂OEt | i-Pr | SO₂Me | |
| 3-65 | CH₂OCH₂CH₂SO₂Me | Me | SO₂Me | |
| 3-66 | CH₂OCH₂CH₂SO₂Me | Et | SO₂Me | |
| 3-67 | CH₂OCH₂CH₂SO₂Me | n-Pr | SO₂Me | |
| 3-68 | CH₂OCH₂CH₂SO₂Me | i-Pr | SO₂Me | |
| 3-69 | CH₂SO₂CH₂CH₂OMe | Me | SO₂Me | |
| 3-70 | CH₂SO₂CH₂CH₂OMe | Et | SO₂Me | |
| 3-71 | CH₂SO₂CH₂CH₂OMe | n-Pr | SO₂Me | |
| 3-72 | CH₂SO₂CH₂CH₂OMe | i-Pr | SO₂Me | |
| 3-73 | CH₂SO₂CH₂CH₂SO₂Me | Me | SO₂Me | |
| 3-74 | CH₂SO₂CH₂CH₂SO₂Me | Et | SO₂Me | |
| 3-75 | CH₂SO₂CH₂CH₂SO₂Me | n-Pr | SO₂Me | |
| 3-76 | CH₂SO₂CH₂CH₂SO₂Me | i-Pr | SO₂Me | |
| 3-77 | Cl | Me | SO₂Et | |
| 3-78 | Cl | Et | SO₂Et | |
| 3-79 | Cl | n-Pr | SO₂Et | |
| 3-80 | Cl | i-Pr | SO₂Et | |
| 3-81 | Br | Me | SO₂Et | |
| 3-82 | Br | Et | SO₂Et | |
| 3-83 | Br | n-Pr | SO₂Et | |
| 3-84 | Br | i-Pr | SO₂Et | |
| 3-85 | Me | Me | SO₂Et | |
| 3-86 | Me | Et | SO₂Et | |
| 3-87 | Me | n-Pr | SO₂Et | |
| 3-88 | Me | i-Pr | SO₂Et | |
| 3-89 | Et | Me | SO₂Et | |
| 3-90 | Et | Et | SO₂Et | |
| 3-91 | Et | n-Pr | SO₂Et | |
| 3-92 | Et | i-Pr | SO₂Et | |
| 3-93 | CF₃ | Me | SO₂Et | |
| 3-94 | CF₃ | Et | SO₂Et | |
| 3-95 | CF₃ | n-Pr | SO₂Et | |
| 3-96 | CF₃ | i-Pr | SO₂Et | |
| 3-97 | OMe | Me | SO₂Et | |
| 3-98 | OMe | Et | SO₂Et | |
| 3-99 | OMe | n-Pr | SO₂Et | |
| 3-100 | OMe | i-Pr | SO₂Et | |
| 3-101 | OEt | Me | SO₂Et | |
| 3-102 | OEt | Et | SO₂Et | |
| 3-103 | OEt | n-Pr | SO₂Et | |
| 3-104 | OEt | i-Pr | SO₂Et | |
| 3-105 | NO₂ | Me | SO₂Et | |
| 3-106 | NO₂ | Et | SO₂Et | |
| 3-107 | NO₂ | n-Pr | SO₂Et | |
| 3-108 | NO₂ | i-Pr | SO₂Et | |
| 3-109 | SO₂Me | Me | SO₂Et | |
| 3-110 | SO₂Me | Et | SO₂Et | |
| 3-111 | SO₂Me | n-Pr | SO₂Et | |
| 3-112 | SO₂Me | i-Pr | SO₂Et | |
| 3-113 | CH₂OMe | Me | SO₂Et | |
| 3-114 | CH₂OMe | Et | SO₂Et | |
| 3-115 | CH₂OMe | n-Pr | SO₂Et | |
| 3-116 | CH₂OMe | i-Pr | SO₂Et | |
| 3-117 | CH₂SO₂Me | Me | SO₂Et | |
| 3-118 | CH₂SO₂Me | Et | SO₂Et | |
| 3-119 | CH₂SO₂Me | n-Pr | SO₂Et | |
| 3-120 | CH₂SO₂Me | i-Pr | SO₂Et | |
| 3-121 | CH₂OCH₂CH₂OMe | Me | SO₂Et | |
| 3-122 | CH₂OCH₂CH₂OMe | Et | SO₂Et | |
| 3-123 | CH₂OCH₂CH₂OMe | n-Pr | SO₂Et | |
| 3-124 | CH₂OCH₂CH₂OMe | i-Pr | SO₂Et | |
| 3-125 | CH₂OCH₂CH₂OEt | Me | SO₂Et | |
| 3-126 | CH₂OCH₂CH₂OEt | Et | SO₂Et | |
| 3-127 | CH₂OCH₂CH₂OEt | n-Pr | SO₂Et | |
| 3-128 | CH₂OCH₂CH₂OEt | i-Pr | SO₂Et | |
| 3-129 | CH₂OCH₂CH₂CH₂OMe | Me | SO₂Et | |
| 3-130 | CH₂OCH₂CH₂CH₂OMe | Et | SO₂Et | |
| 3-131 | CH₂OCH₂CH₂CH₂OMe | n-Pr | SO₂Et | |
| 3-132 | CH₂OCH₂CH₂CH₂OMe | i-Pr | SO₂Et | |
| 3-133 | CH₂OCH₂OMe | Me | SO₂Et | |
| 3-134 | CH₂OCH₂OMe | Et | SO₂Et | |
| 3-135 | CH₂OCH₂OMe | n-Pr | SO₂Et | |
| 3-136 | CH₂OCH₂OMe | i-Pr | SO₂Et | |
| 3-137 | CH₂OCH₂OEt | Me | SO₂Et | |
| 3-138 | CH₂OCH₂OEt | Et | SO₂Et | |
| 3-139 | CH₂OCH₂OEt | n-Pr | SO₂Et | |
| 3-140 | CH₂OCH₂OEt | i-Pr | SO₂Et | |
| 3-141 | CH₂OCH₂CH₂SO₂Me | Me | SO₂Et | |
| 3-142 | CH₂OCH₂CH₂SO₂Me | Et | SO₂Et | |
| 3-143 | CH₂OCH₂CH₂SO₂Me | n-Pr | SO₂Et | |
| 3-144 | CH₂OCH₂CH₂SO₂Me | i-Pr | SO₂Et | |
| 3-145 | CH₂SO₂CH₂CH₂OMe | Me | SO₂Et | |
| 3-146 | CH₂SO₂CH₂CH₂OMe | Et | SO₂Et | |
| 3-147 | CH₂SO₂CH₂CH₂OMe | n-Pr | SO₂Et | |
| 3-148 | CH₂SO₂CH₂CH₂OMe | i-Pr | SO₂Et | |
| 3-149 | CH₂SO₂CH₂CH₂SO₂Me | Me | SO₂Et | |
| 3-150 | CH₂SO₂CH₂CH₂SO₂Me | Et | SO₂Et | |
| 3-151 | CH₂SO₂CH₂CH₂SO₂Me | n-Pr | SO₂Et | |
| 3-152 | CH₂SO₂CH₂CH₂SO₂Me | i-Pr | SO₂Et | |
| 3-153 | Cl | Me | F | |
| 3-154 | Cl | Et | F | |
| 3-155 | Cl | n-Pr | F | |
| 3-156 | Cl | i-Pr | F | |
| 3-157 | Br | Me | F | |
| 3-158 | Br | Et | F | |
| 3-159 | Br | n-Pr | F | |
| 3-160 | Br | i-Pr | F | |
| 3-161 | Me | Me | F | |
| 3-162 | Me | Et | F | |
| 3-163 | Me | n-Pr | F | |
| 3-164 | Me | i-Pr | F | |
| 3-165 | Et | Me | F | |
| 3-166 | Et | Et | F | |
| 3-167 | Et | n-Pr | F | |
| 3-168 | Et | i-Pr | F | |
| 3-169 | CF₃ | Me | F | |
| 3-170 | CF₃ | Et | F | |
| 3-171 | CF₃ | n-Pr | F | |
| 3-172 | CF₃ | i-Pr | F | |
| 3-173 | OMe | Me | F | |
| 3-174 | OMe | Et | F | |
| 3-175 | OMe | n-Pr | F | |
| 3-176 | OMe | i-Pr | F | |
| 3-177 | OEt | Me | F | |
| 3-178 | OEt | Et | F | |
| 3-179 | OEt | n-Pr | F | |
| 3-180 | OEt | i-Pr | F | |
| 3-181 | NO₂ | Me | F | |
| 3-182 | NO₂ | Et | F | |
| 3-183 | NO₂ | n-Pr | F | |
| 3-184 | NO₂ | i-Pr | F | |
| 3-185 | SO₂Me | Me | F | |
| 3-186 | SO₂Me | Et | F | |
| 3-187 | SO₂Me | n-Pr | F | |
| 3-188 | SO₂Me | i-Pr | F | |
| 3-189 | CH₂OMe | Me | F | |
| 3-190 | CH₂OMe | Et | F | |
| 3-191 | CH₂OMe | n-Pr | F | |
| 3-192 | CH₂OMe | i-Pr | F | |
| 3-193 | CH₂SO₂Me | Me | F | |
| 3-194 | CH₂SO₂Me | Et | F | |
| 3-195 | CH₂SO₂Me | n-Pr | F | |
| 3-196 | CH₂SO₂Me | i-Pr | F | |
| 3-197 | CH₂OCH₂CH₂OMe | Me | F | |
| 3-198 | CH₂OCH₂CH₂OMe | Et | F | |
| 3-199 | CH₂OCH₂CH₂OMe | n-Pr | F | |
| 3-200 | CH₂OCH₂CH₂OMe | i-Pr | F | |
| 3-201 | CH₂OCH₂CH₂OEt | Me | F | |
| 3-202 | CH₂OCH₂CH₂OEt | Et | F | |
| 3-203 | CH₂OCH₂CH₂OEt | n-Pr | F | |
| 3-204 | CH₂OCH₂CH₂OEt | i-Pr | F | |
| 3-205 | CH₂OCH₂CH₂CH₂OMe | Me | F | |
| 3-206 | CH₂OCH₂CH₂CH₂OMe | Et | F | |
| 3-207 | CH₂OCH₂CH₂CH₂OMe | n-Pr | F | |
| 3-208 | CH₂OCH₂CH₂CH₂OMe | i-Pr | F | |
| 3-209 | CH₂OCH₂OMe | Me | F | |
| 3-210 | CH₂OCH₂OMe | Et | F | |
| 3-211 | CH₂OCH₂OMe | n-Pr | F | |
| 3-212 | CH₂OCH₂OMe | i-Pr | F | |
| 3-213 | CH₂OCH₂OEt | Me | F | |
| 3-214 | CH₂OCH₂OEt | Et | F | |
| 3-215 | CH₂OCH₂OEt | n-Pr | F | |
| 3-216 | CH₂OCH₂OEt | i-Pr | F | |
| 3-217 | CH₂OCH₂CH₂SO₂Me | Me | F | |
| 3-218 | CH₂OCH₂CH₂SO₂Me | Et | F | |
| 3-219 | CH₂OCH₂CH₂SO₂Me | n-Pr | F | |
| 3-220 | CH₂OCH₂CH₂SO₂Me | i-Pr | F | |
| 3-221 | CH₂SO₂CH₂CH₂OMe | Me | F | |
| 3-222 | CH₂SO₂CH₂CH₂OMe | Et | F | |
| 3-223 | CH₂SO₂CH₂CH₂OMe | n-Pr | F | |
| 3-224 | CH₂SO₂CH₂CH₂OMe | i-Pr | F | |
| 3-225 | CH₂SO₂CH₂CH₂SO₂Me | Me | F | |
| 3-226 | CH₂SO₂CH₂CH₂SO₂Me | Et | F | |
| 3-227 | CH₂SO₂CH₂CH₂SO₂Me | n-Pr | F | |
| 3-228 | CH₂SO₂CH₂CH₂SO₂Me | i-Pr | F | |
| 3-229 | Cl | Me | Cl | |
| 3-230 | Cl | Et | Cl | |
| 3-231 | Cl | n-Pr | Cl | |
| 3-232 | Cl | i-Pr | Cl | |
| 3-233 | Br | Me | Cl | |
| 3-234 | Br | Et | Cl | |
| 3-235 | Br | n-Pr | Cl | |
| 3-236 | Br | i-Pr | Cl | |
| 3-237 | Me | Me | Cl | 7.37 (d, 1H), 7.23 (d, 1H), 3.63 (s, 3H), 3.01 (s, 3H), 2.68 (s, 3H), 1.72(s, 3H) |
| 3-238 | Me | Et | Cl | 7.37 (d, 1H), 7.23 (d, 1H), 3.63 (s, 3H), 3.36 (m, 1H), 3.16 (m, 1H), 2.63 (s, 3H), 1.72 (s, 3H), 1.40 (t, 3H) |
| 3-239 | Me | n-Pr | Cl | |
| 3-240 | Me | i-Pr | Cl | |
| 3-241 | Et | Me | Cl | |
| 3-242 | Et | Et | Cl | |
| 3-243 | Et | n-Pr | Cl | |
| 3-244 | Et | i-Pr | Cl | |
| 3-245 | CF₃ | Me | Cl | |
| 3-246 | CF₃ | Et | Cl | |
| 3-247 | CF₃ | n-Pr | Cl | |
| 3-248 | CF₃ | i-Pr | Cl | |
| 3-249 | OMe | Me | Cl | |
| 3-250 | OMe | Et | Cl | |
| 3-251 | OMe | n-Pr | Cl | |
| 3-252 | OMe | i-Pr | Cl | |
| 3-253 | OEt | Me | Cl | |
| 3-254 | OEt | Et | Cl | |
| 3-255 | OEt | n-Pr | Cl | |
| 3-256 | OEt | i-Pr | Cl | |
| 3-257 | NO₂ | Me | Cl | |
| 3-258 | NO₂ | Et | Cl | |
| 3-259 | NO₂ | n-Pr | Cl | |
| 3-260 | NO₂ | i-Pr | Cl | |
| 3-261 | SO₂Me | Me | Cl | |
| 3-262 | SO₂Me | Et | Cl | |
| 3-263 | SO₂Me | n-Pr | Cl | |
| 3-264 | SO₂Me | i-Pr | Cl | |
| 3-265 | CH₂OMe | Me | Cl | |
| 3-266 | CH₂OMe | Et | Cl | |
| 3-267 | CH₂OMe | n-Pr | Cl | |
| 3-268 | CH₂OMe | i-Pr | Cl | |
| 3-269 | CH₂SO₂Me | Me | Cl | |
| 3-270 | CH₂SO₂Me | Et | Cl | |
| 3-271 | CH₂SO₂Me | n-Pr | Cl | |
| 3-272 | CH₂SO₂Me | i-Pr | Cl | |
| 3-273 | CH₂OCH₂CH₂OMe | Me | Cl | |
| 3-274 | CH₂OCH₂CH₂OMe | Et | Cl | |
| 3-275 | CH₂OCH₂CH₂OMe | n-Pr | Cl | |
| 3-276 | CH₂OCH₂CH₂OMe | i-Pr | Cl | |
| 3-277 | CH₂OCH₂CH₂OEt | Me | Cl | |
| 3-278 | CH₂OCH₂CH₂OEt | Et | Cl | |
| 3-279 | CH₂OCH₂CH₂OEt | n-Pr | Cl | |
| 3-280 | CH₂OCH₂CH₂OEt | i-Pr | Cl | |
| 3-281 | CH₂OCH₂CH₂CH₂OMe | Me | Cl | |
| 3-282 | CH₂OCH₂CH₂CH₂OMe | Et | Cl | |
| 3-283 | CH₂OCH₂CH₂CH₂OMe | n-Pr | Cl | |
| 3-284 | CH₂OCH₂CH₂CH₂OMe | i-Pr | Cl | |
| 3-285 | CH₂OCH₂OMe | Me | Cl | |
| 3-286 | CH₂OCH₂OMe | Et | Cl | |
| 3-287 | CH₂OCH₂OMe | n-Pr | Cl | |
| 3-288 | CH₂OCH₂OMe | i-Pr | Cl | |
| 3-289 | CH₂OCH₂OEt | Me | Cl | |
| 3-290 | CH₂OCH₂OEt | Et | Cl | |
| 3-291 | CH₂OCH₂OEt | n-Pr | Cl | |
| 3-292 | CH₂OCH₂OEt | i-Pr | Cl | |
| 3-293 | CH₂OCH₂CH₂SO₂Me | Me | Cl | |
| 3-294 | CH_{z}OCH₂CH₂SO₂Me | Et | Cl | |
| 3-295 | CH₂OCH₂CH₂SO₂Me | n-Pr | Cl | |
| 3-296 | CH₂OCH₂CH₂SO₂Me | i-Pr | Cl | |
| 3-297 | CH₂SO₂CH₂CH₂OMe | Me | Cl | |
| 3-298 | CH₂SO₂CH₂CH₂OMe | Et | Cl | |
| 3-299 | CH₂SO₂CH₂CH₂OMe | n-Pr | Cl | |
| 3-300 | CH₂SO₂CH₂CH₂OMe | i-Pr | Cl | |
| 3-301 | CH₂SO₂CH₂CH₂SO₂Me | Me | Cl | |
| 3-302 | CH₂SO₂CH₂CH₂SO₂Me | Et | Cl | |
| 3-303 | CH₂SO₂CH₂CH₂SO₂Me | n-Pr | Cl | |
| 3-304 | CH₂SO₂CH₂CH₂SO₂Me | i-Pr | Cl | |
| 3-305 | Cl | Me | Br | |
| 3-306 | Cl | Et | Br | |
| 3-307 | Cl | n-Pr | Br | |
| 3-308 | Cl | i-Pr | Br | |
| 3-309 | Br | Me | Br | |
| 3-310 | Br | Et | Br | |
| 3-311 | Br | n-Pr | Br | |
| 3-312 | Br | i-Pr | Br | |
| 3-313 | Me | Me | Br | 7.57 (d, 1H), 7.16 (d, 1H), 3.67 (s, 3H), 3.02 (s, 3H), 2.69 (s, 3H), 1.75 (s, 3H) |
| 3-314 | Me | Et | Br | |
| 3-315 | Me | n-Pr | Br | |
| 3-316 | Me | i-Pr | Br | |
| 3-317 | Et | Me | Br | |
| 3-318 | Et | Et | Br | |
| 3-319 | Et | n-Pr | Br | |
| 3-320 | Et | i-Pr | Br | |
| 3-321 | CF₃ | Me | Br | |
| 3-322 | CF₃ | Et | Br | |
| 3-323 | CF₃ | n-Pr | Br | |
| 3-324 | CF₃ | i-Pr | Br | |
| 3-325 | OMe | Me | Br | |
| 3-326 | OMe | Et | Br | |
| 3-327 | OMe | n-Pr | Br | |
| 3-328 | OMe | i-Pr | Br | |
| 3-329 | OEt | Me | Br | |
| 3-330 | OEt | Et | Br | |
| 3-331 | OEt | n-Pr | Br | |
| 3-332 | OEt | i-Pr | Br | |
| 3-333 | NO₂ | Me | Br | |
| 3-334 | NO₂ | Et | Br | |
| 3-335 | NO₂ | n-Pr | Br | |
| 3-336 | NO₂ | i-Pr | Br | |
| 3-337 | SO₂Me | Me | Br | |
| 3-338 | SO₂Me | Et | Br | |
| 3-339 | SO₂Me | n-Pr | Br | |
| 3-340 | SO₂Me | i-Pr | Br | |
| 3-341 | CH₂OMe | Me | Br | |
| 3-342 | CH₂OMe | Et | Br | |
| 3-343 | CH₂OMe | n-Pr | Br | |
| 3-344 | CH₂OMe | i-Pr | Br | |
| 3-345 | CH₂SO₂Me | Me | Br | |
| 3-346 | CH₂SO₂Me | Et | Br | |
| 3-347 | CH₂SO₂Me | n-Pr | Br | |
| 3-348 | CH₂SO₂Me | i-Pr | Br | |
| 3-349 | CH₂OCH₂CH₂OMe | Me | Br | |
| 3-350 | CH₂OCH₂CH₂OMe | Et | Br | |
| 3-351 | CH₂OCH₂CH₂OMe | n-Pr | Br | |
| 3-352 | CH₂OCH₂CH₂OMe | i-Pr | Br | |
| 3-353 | CH₂OCH₂CH₂OEt | Me | Br | |
| 3-354 | CH₂OCH₂CH₂OEt | Et | Br | |
| 3-355 | CH₂OCH₂CH₂OEt | n-Pr | Br | |
| 3-356 | CH₂OCH₂CH₂OEt | i-Pr | Br | |
| 3-357 | CH₂OCH₂CH₂CH₂OMe | Me | Br | |
| 3-358 | CH₂OCH₂CH₂CH₂OMe | Et | Br | |
| 3-359 | CH₂OCH₂CH₂CH₂OMe | n-Pr | Br | |
| 3-360 | CH₂OCH₂CH₂CH₂OMe | i-Pr | Br | |
| 3-361 | CH₂OCH₂OMe | Me | Br | |
| 3-362 | CH₂OCH₂OMe | Et | Br | |
| 3-363 | CH₂oCH₂OMe | n-Pr | Br | |
| 3-364 | CH₂OCH₂OMe | i-Pr | Br | |
| 3-365 | CH₂OCH₂OEt | Me | Br | |
| 3-366 | CH₂OCH₂OEt | Et | Br | |
| 3-367 | CH₂OCH₂OEt | n-Pr | Br | |
| 3-368 | CH₂OCH₂OEt | i-Pr | Br | |
| 3-369 | CH₂OCH₂CH₂SO₂Me | Me | Br | |
| 3-370 | CH₂OCH₂CH₂SO₂Me | Et | Br | |
| 3-371 | CH₂OCH₂CH₂SO₂Me | n-Pr | Br | |
| 3-372 | CH₂OCH₂CH₂SO₂Me | i-Pr | Br | |
| 3-373 | CH₂SO₂CH₂CH₂oMe | Me | Br | |
| 3-374 | CH₂SO₂CH₂CH₂OMe | Et | Br | |
| 3-375 | CH₂SO₂CH₂CH₂OMe | n-Pr | Br | |
| 3-376 | CH₂SO₂CH₂CH₂OMe | i-Pr | Br | |
| 3-377 | CH₂SO₂CH₂CH₂SO₂Me | Me | Br | |
| 3-378 | CH₂SO₂CH₂CH₂SO₂Me | Et | Br | |
| 3-379 | CH₂SO₂CH₂CH₂SO₂Me | n-Pr | Br | |
| 3-380 | CH₂SO₂CH₂CH₂SO₂Me | i-Pr | Br | |
| 3-381 | Cl | Me | I | |
| 3-382 | Cl | Et | I | |
| 3-383 | Cl | n-Pr | I | |
| 3-384 | Cl | i-Pr | I | |
| 3-385 | Br | Me | I | |
| 3-386 | Br | Et | I | |
| 3-387 | Br | n-Pr | I | |
| 3-388 | Br | i-Pr | I | |
| 3-389 | Me | Me | I | 7.68 (d, 1H), 6.96(d, 1H), 3.65 (s, 3H), 2.96 (s, 3H), 2.68 (s, 3H), 1.74 (s, 3H) |
| 3-390 | Me | Et | I | |
| 3-391 | Me | n-Pr | I | |
| 3-392 | Me | i-Pr | I | |
| 3-393 | Et | Me | I | |
| 3-394 | Et | Et | I | |
| 3-395 | Et | n-Pr | I | |
| 3-396 | Et | i-Pr | I | |
| 3-397 | CF₃ | Me | I | |
| 3-398 | CF₃ | Et | I | |
| 3-399 | CF₃ | n-Pr | I | |
| 3-400 | CF₃ | i-Pr | I | |
| 3-401 | OMe | Me | I | |
| 3-402 | OMe | Et | I | |
| 3-403 | OMe | n-Pr | I | |
| 3-404 | OMe | i-Pr | I | |
| 3-405 | OEt | Me | I | |
| 3-406 | OEt | Et | I | |
| 3-407 | OEt | n-Pr | I | |
| 3-408 | OEt | i-Pr | I | |
| 3-409 | NO₂ | Me | I | |
| 3-410 | NO₂ | Et | I | |
| 3-411 | NO₂ | n-Pr | I | |
| 3-412 | NO₂ | i-Pr | I | |
| 3-413 | SO₂Me | Me | I | |
| 3-414 | SO₂Me | Et | I | |
| 3-415 | SO₂Me | n-Pr | I | |
| 3-416 | SO₂Me | i-Pr | I | |
| 3-417 | CH₂OMe | Me | I | |
| 3-418 | CH₂OMe | Et | I | |
| 3-419 | CH₂OMe | n-Pr | I | |
| 3-420 | CH₂OMe | i-Pr | I | |
| 3-421 | CH₂SO₂Me | Me | I | |
| 3-422 | CH₂SO₂Me | Et | I | |
| 3-423 | CH₂SO₂Me | n-Pr | I | |
| 3-424 | CH₂SO₂Me | i-Pr | I | |
| 3-425 | CH₂OCH₂CH₂OMe | Me | I | |
| 3-426 | CH₂OCH₂CH₂OMe | Et | I | |
| 3-427 | CH₂OCH₂CH₂OMe | n-Pr | I | |
| 3-428 | CH_{z}OCH₂CH₂OMe | i-Pr | I | |
| 3-429 | CH₂OCH₂CH₂OEt | Me | I | |
| 3-430 | CH₂OCH₂CH₂OEt | Et | I | |
| 3-431 | CH₂OCH₂CH₂OEt | n-Pr | I | |
| 3-432 | CH₂OCH₂CH₂OEt | i-Pr | I | |
| 3-433 | CH₂OCH₂CH₂CH₂OMe | Me | I | |
| 3-434 | CH₂OCH₂CH₂CH₂OMe | Et | I | |
| 3-435 | CH₂OCH₂CH₂CH₂OMe | n-Pr | I | |
| 3-436 | CH₂OCH₂CH₂CH₂OMe | i-Pr | I | |
| 3-437 | CH₂OCH₂OMe | Me | I | |
| 3-438 | CH₂OCH₂OMe | Et | I | |
| 3-439 | CH₂OCH₂OMe | n-Pr | I | |
| 3-440 | CH₂OCH₂OMe | i-Pr | I | |
| 3-441 | CH₂OCH₂OEt | Me | I | |
| 3-442 | CH₂OCH₂OEt | Et | I | |
| 3-443 | CH₂OCH₂OEt | n-Pr | I | |
| 3-444 | CH₂OCH₂OEt | i-Pr | I | |
| 3-445 | CH₂OCH₂CH₂SO₂Me | Me | I | |
| 3-446 | CH₂OCH₂CH₂SO₂Me | Et | I | |
| 3-447 | CH₂OCH₂CH₂SO₂Me | n-Pr | I | |
| 3-448 | CH₂OCH₂CH₂SO₂Me | i-Pr | I | |
| 3-449 | CH₂SO₂CH₂CH₂OMe | Me | I | |
| 3-450 | CH₂SO₂CH₂CH₂OMe | Et | I | |
| 3-451 | CH₂SO₂CH₂CH₂OMe | n-Pr | I | |
| 3-452 | CH₂SO₂CH₂CH₂OMe | i-Pr | I | |
| 3-453 | CH₂SO₂CH₂CH₂SO₂Me | Me | I | |
| 3-454 | CH₂SO₂CH₂CH₂SO₂Me | Et | I | |
| 3-455 | CH₂SO₂CH₂CH₂SO₂Me | n-Pr | I | |
| 3-456 | CH₂SO₂CH₂CH₂SO₂Me | i-Pr | I | |
| 3-457 | Cl | Me | NO₂ | |
| 3-458 | Cl | Et | NO₂ | |
| 3-459 | Cl | n-Pr | NO₂ | |
| 3-460 | Cl | i-Pr | NO₂ | |
| 3-461 | Br | Me | NO₂ | |
| 3-462 | Br | Et | NO₂ | |
| 3-463 | Br | n-Pr | NO₂ | |
| 3-464 | Br | i-Pr | NO₂ | |
| 3-465 | Me | Me | NO₂ | |
| 3-466 | Me | Et | NO₂ | |
| 3-467 | Me | n-Pr | NO₂ | |
| 3-468 | Me | i-Pr | NO₂ | |
| 3-469 | Et | Me | NO₂ | |
| 3-470 | Et | Et | NO₂ | |
| 3-471 | Et | n-Pr | NO₂ | |
| 3-472 | Et | i-Pr | NO₂ | |
| 3-473 | CF₃ | Me | NO₂ | |
| 3-474 | CF₃ | Et | NO₂ | |
| 3-475 | CF₃ | n-Pr | NO₂ | |
| 3-476 | CF₃ | i-Pr | NO₂ | |
| 3-477 | OMe | Me | NO₂ | |
| 3-478 | OMe | Et | NO₂ | |
| 3-479 | OMe | n-Pr | NO₂ | |
| 3-480 | OMe | i-Pr | NO₂ | |
| 3-481 | OEt | Me | NO₂ | |
| 3-482 | OEt | Et | NO₂ | |
| 3-483 | OEt | n-Pr | NO₂ | |
| 3-484 | OEt | i-Pr | NO₂ | |
| 3-485 | NO₂ | Me | NO₂ | |
| 3-486 | NO₂ | Et | NO₂ | |
| 3-487 | NO₂ | n-Pr | NO₂ | |
| 3-488 | NO₂ | i-Pr | NO₂ | |
| 3-489 | SO₂Me | Me | NO₂ | |
| 3-490 | SO₂Me | Et | NO₂ | |
| 3-491 | SO₂Me | n-Pr | NO₂ | |
| 3-492 | SO₂Me | i-Pr | NO₂ | |
| 3-493 | CH₂OMe | Me | NO₂ | |
| 3-494 | CH₂OMe | Et | NO₂ | |
| 3-495 | CH₂OMe | n-Pr | NO₂ | |
| 3-496 | CH₂OMe | i-Pr | NO₂ | |
| 3-497 | CH₂SO₂Me | Me | NO₂ | |
| 3-498 | CH₂SO₂Me | Et | NO₂ | |
| 3-499 | CH₂SO₂Me | n-Pr | NO₂ | |
| 3-500 | CH₂SO₂Me | i-Pr | NO₂ | |
| 3-501 | CH₂OCH₂CH₂OMe | Me | NO₂ | |
| 3-502 | CH₂OCH₂CH₂OMe | Et | NO₂ | |
| 3-503 | CH₂OCH₂CH₂OMe | n-Pr | NO₂ | |
| 3-504 | CH₂oCH₂CH₂OMe | i-Pr | NO₂ | |
| 3-505 | CH₂OCH₂CH₂OEt | Me | NO₂ | |
| 3-506 | CH₂oCH₂CH₂OEt | Et | NO₂ | |
| 3-507 | CH₂OCH₂CH₂OEt | n-Pr | NO₂ | |
| 3-508 | CH₂OCH₂CH₂OEt | i-Pr | NO₂ | |
| 3-509 | CH₂OCH₂CH₂CH₂OMe | Me | NO₂ | |
| 3-510 | CH₂OCH₂CH₂CH₂OMe | Et | NO₂ | |
| 3-511 | CH₂OCH₂CH₂CH₂OMe | n-Pr | NO₂ | |
| 3-512 | CH₂OCH₂CH₂CH₂OMe | i-Pr | NO₂ | |
| 3-513 | CH₂oCH₂OMe | Me | NO₂ | |
| 3-514 | CH₂OCH₂OMe | Et | NO₂ | |
| 3-515 | CH₂OCH₂OMe | n-Pr | NO₂ | |
| 3-516 | CH₂OCH₂OMe | i-Pr | NO₂ | |
| 3-517 | CH₂OCH₂OEt | Me | NO₂ | |
| 3-518 | CH₂OCH₂OEt | Et | NO₂ | |
| 3-519 | CH₂OCH₂OEt | n-Pr | NO₂ | |
| 3-520 | CH₂OCH₂OEt | i-Pr | NO₂ | |
| 3-521 | CH₂OCH₂CH₂SO₂Me | Me | NO₂ | |
| 3-522 | CH₂OCH₂CH₂SO₂Me | Et | NO₂ | |
| 3-523 | CH₂OCH₂CH₂SO₂Me | n-Pr | NO₂ | |
| 3-524 | CH₂OCH₂CH₂SO₂Me | i-Pr | NO₂ | |
| 3-525 | CH₂SO₂CH₂CH₂OMe | Me | NO₂ | |
| 3-526 | CH₂SO₂CH₂CH₂OMe | Et | NO₂ | |
| 3-527 | CH₂SO₂CH₂CH₂OMe | n-Pr | NO₂ | |
| 3-528 | CH₂SO₂CH₂CH₂OMe | i-Pr | NO₂ | |
| 3-529 | CH₂SO₂CH₂CH₂SO₂Me | Me | NO₂ | |
| 3-530 | CH₂SO₂CH₂CH₂SO₂Me | Et | NO₂ | |
| 3-531 | CH₂SO₂CH₂CH₂SO₂Me | n-Pr | NO₂ | |
| 3-532 | CH₂SO₂CH₂CH₂SO₂Me | i-Pr | NO₂ | |

**Tabelle 4: Erfindungsgemäße Verbindungen der allgemeinen Formel (II)**

| | | | | |
|---|---|---|---|---|
| 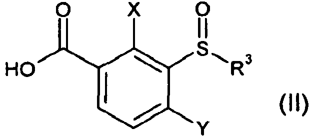 | | | | |

| Nr. | X | R³ | Y | Physikalische Daten: ¹H-NMR: δ [CDCl₃] |
|---|---|---|---|---|
| 4-1 | Cl | Me | SO₂Me | |
| 4-2 | Cl | Et | SO₂Me | |
| 4-3 | Cl | n-Pr | SO₂Me | |
| 4-4 | Cl | i-Pr | SO₂Me | |
| 4-5 | Br | Me | SO₂Me | |
| 4-6 | Br | Et | SO₂Me | |
| 4-7 | Br | n-Pr | SO₂Me | |
| 4-8 | Br | i-Pr | SO₂Me | |
| 4-9 | Me | Me | SO₂Me | (DMSO-d₆): 7.93 (s, 2H), 3.46 (s, 3H), 3.08 (s, 3H), 2.87 (s, 3H) |
| 4-10 | Me | Et | SO₂Me | (DMSO-d₆): 7.93 (s, 2H), 3.47 (m, 1H), 3.46 (s, 3H), 3.08 (m, 1H), 2.82 (s, 3H), 1.33 (t, 3H) |
| 4-11 | Me | n-Pr | SO₂Me | |
| 4-12 | Me | i-Pr | SO₂Me | |
| 4-13 | Et | Me | SO₂Me | |
| 4-14 | Et | Et | SO₂Me | |
| 4-15 | Et | n-Pr | SO₂Me | |
| 4-16 | Et | i-Pr | SO₂Me | |
| 4-17 | CF₃ | Me | SO₂Me | |
| 4-18 | CF₃ | Et | SO₂Me | |
| 4-19 | CF₃ | n-Pr | SO₂Me | |
| 4-20 | CF₃ | i-Pr | SO₂Me | |
| 4-21 | OMe | Me | SO₂Me | |
| 4-22 | OMe | Et | SO₂Me | |
| 4-23 | OMe | n-Pr | SO₂Me | |
| 4-24 | OMe | i-Pr | SO₂Me | |
| 4-25 | OEt | Me | SO₂Me | |
| 4-26 | OEt | Et | SO₂Me | |
| 4-27 | OEt | n-Pr | SO₂Me | |
| 4-28 | OEt | i-Pr | SO₂Me | |
| 4-29 | NO₂ | Me | SO₂Me | |
| 4-30 | NO₂ | Et | SO₂Me | |
| 4-31 | NO₂ | n-Pr | SO₂Me | |
| 4-32 | NO₂ | i-Pr | SO₂Me | |
| 4-33 | SO₂Me | Me | SO₂Me | |
| 4-34 | SO₂Me | Et | SO₂Me | |
| 4-35 | SO₂Me | n-Pr | SO₂Me | |
| 4-36 | SO₂Me | i-Pr | SO₂Me | |
| 4-37 | CH₂OMe | Me | SO₂Me | |
| 4-38 | CH₂OMe | Et | SO₂Me | |
| 4-39 | CH₂OMe | n-Pr | SO₂Me | |
| 4-40 | CH₂OMe | i-Pr | SO₂Me | |
| 4-41 | CH₂SO₂Me | Me | SO₂Me | |
| 4-42 | CH₂SO₂Me | Et | SO₂Me | |
| 4-43 | CH₂SO₂Me | n-Pr | SO₂Me | |
| 4-44 | CH₂SO₂Me | i-Pr | SO₂Me | |
| 4-45 | CH₂OCH₂CH₂OMe | Me | SO₂Me | |
| 4-46 | CH₂OCH₂CH₂OMe | Et | SO₂Me | |
| 4-47 | CH₂OCH₂CH₂OMe | n-Pr | SO₂Me | |
| 4-48 | CH₂OCH₂CH₂OMe | i-Pr | SO₂Me | |
| 4-49 | CH₂OCH₂CH₂OEt | Me | SO₂Me | |
| 4-50 | CH₂OCH₂CH₂OEt | Et | SO₂Me | |
| 4-51 | CH₂OCH₂CH₂OEt | n-Pr | SO₂Me | |
| 4-52 | CH₂OCH₂CH₂OEt | i-Pr | SO₂Me | |
| 4-53 | CH₂OCH₂CH₂CH₂OMe | Me | SO₂Me | |
| 4-54 | CH₂OCH₂CH₂CH₂OMe | Et | SO₂Me | |
| 4-55 | CH₂OCH₂CH₂CH₂OMe | n-Pr | SO₂Me | |
| 4-56 | CH₂OCH₂CH₂CH₂OMe | i-Pr | SO₂Me | |
| 4-57 | CH₂OCH₂OMe | Me | SO₂Me | |
| 4-58 | CH₂OCH₂Me | Et | SO₂Me | |
| 4-59 | CH₂OCH₂OMe | n-Pr | SO₂Me | |
| 4-60 | CH₂OCH₂OMe | i-Pr | SO₂Me | |
| 4-61 | CH₂OCH₂OEt | Me | SO₂Me | |
| 4-62 | CH₂OCH₂OEt | Et | SO₂Me | |
| 4-63 | CH₂OCH₂OEt | n-Pr | SO₂Me | |
| 4-64 | CH₂OCH₂OEt | i-Pr | SO₂Me | |
| 4-65 | CH₂OCH₂CH₂SO₂Me | Me | SO₂Me | |
| 4-66 | CH₂OCH₂CH₂SO₂Me | Et | SO₂Me | |
| 4-67 | CH₂OCH₂CH₂SO₂Me | n-Pr | SO₂Me | |
| 4-68 | CH₂OCH₂CH₂SO₂Me | i-Pr | SO₂Me | |
| 4-69 | CH₂SO₂CH₂CH₂OMe | Me | SO₂Me | |
| 4-70 | CH₂SO₂CH₂CH₂OMe | Et | SO₂Me | |
| 4-71 | CH₂SO₂CH₂CH₂OMe | n-Pr | SO₂Me | |
| 4-72 | CH₂SO₂CH₂CH₂OMe | i-Pr | SO₂Me | |
| 4-73 | CH₂SO₂CH₂CH₂SO₂Me | Me | SO₂Me | |
| 4-74 | CH₂SO₂CH₂CH₂SO₂Me | Et | SO₂Me | |
| 4-75 | CH₂SO₂CH₂CH₂SO₂Me | n-Pr | SO₂Me | |
| 4-76 | CH₂SO₂CH₂CH₂SO₂Me | i-Pr | SO₂Me | |
| 4-77 | Cl | Me | SO₂Et | |
| 4-78 | Cl | Et | SO₂Et | |
| 4-79 | Cl | n-Pr | SO₂Et | |
| 4-80 | Cl | i-Pr | SO₂Et | |
| 4-81 | Br | Me | SO₂Et | |
| 4-82 | Br | Et | SO₂Et | |
| 4-83 | Br | n-Pr | SO₂Et | |
| 4-84 | Br | i-Pr | SO₂Et | |
| 4-85 | Me | Me | SO₂Et | |
| 4-86 | Me | Et | SO₂Et | |
| 4-87 | Me | n-Pr | SO₂Et | |
| 4-88 | Me | i-Pr | SO₂Et | |
| 4-89 | Et | Me | SO₂Et | |
| 4-90 | Et | Et | SO₂Et | |
| 4-91 | Et | n-Pr | SO₂Et | |
| 4-92 | Et | i-Pr | SO₂Et | |
| 4-93 | CF₃ | Me | SO₂Et | |
| 4-94 | CF₃ | Et | SO₂Et | |
| 4-95 | CF₃ | n-Pr | SO₂Et | |
| 4-96 | CF₃ | i-Pr | SO₂Et | |
| 4-97 | OMe | Me | SO₂Et | |
| 4-98 | OMe | Et | SO₂Et | |
| 4-99 | OMe | n-Pr | SO₂Et | |
| 4-100 | OMe | i-Pr | SO₂Et | |
| 4-101 | OEt | Me | SO₂Et | |
| 4-102 | OEt | Et | SO₂Et | |
| 4-103 | OEt | n-Pr | SO₂Et | |
| 4-104 | OEt | i-Pr | SO₂Et | |
| 4-105 | NO₂ | Me | SO₂Et | |
| 4-106 | NO₂ | Et | SO₂Et | |
| 4-107 | NO₂ | n-Pr | SO₂Et | |
| 4-108 | NO₂ | i-Pr | SO₂Et | |
| 4-109 | SO₂Me | Me | SO₂Et | |
| 4-110 | SO₂Me | Et | SO₂Et | |
| 4-111 | SO₂Me | n-Pr | SO₂Et | |
| 4-112 | SO₂Me | i-Pr | SO₂Et | |
| 4-113 | CH₂OMe | Me | SO₂Et | |
| 4-114 | CH₂OMe | Et | SO₂Et | |
| 4-115 | CH₂OMe | n-Pr | SO₂Et | |
| 4-116 | CH₂OMe | i-Pr | SO₂Et | |
| 4-117 | CH₂SO₂Me | Me | SO₂Et | |
| 4-118 | CH₂SO₂Me | Et | SO₂Et | |
| 4-119 | CH₂SO₂Me | n-Pr | SO₂Et | |
| 4-120 | CH₂SO₂Me | i-Pr | SO₂Et | |
| 4-121 | CH₂OCH₂CH₂OMe | Me | SO₂Et | |
| 4-122 | CH₂OCH₂CH₂OMe | Et | SO₂Et | |
| 4-123 | CH₂OCH₂CH₂OMe | n-Pr | SO₂Et | |
| 4-124 | CH₂2CH₂CH₂OMe | i-Pr | SO₂Et | |
| 4-125 | CH₂OCH₂CH₂OEt | Me | SO₂Et | |
| 4-126 | CH₂OCH₂CH₂OEt | Et | SO₂Et | |
| 4-127 | CH₂OCH₂CH₂OEt | n-Pr | SO₂Et | |
| 4-128 | CH₂OCH₂CH₂OEt | i-Pr | SO₂Et | |
| 4-129 | CH₂OCH₂CH₂CH₂OMe | Me | SO₂Et | |
| 4-130 | CH₂OCH₂CH₂CH₂OMe | Et | SO₂Et | |
| 4-131 | CH₂OCH₂CH₂CH₂OMe | n-Pr | SO₂Et | |
| 4-132 | CH₂OCH₂CH₂CH₂OMe | i-Pr | SO₂Et | |
| 4-133 | CH₂OCH₂OMe | Me | SO₂Et | |
| 4-134 | CH₂OCH₂OMe | Et | SO₂Et | |
| 4-135 | CH₂OCH₂OMe | n-Pr | SO₂Et | |
| 4-136 | CH₂OCH₂OMe | i-Pr | SO₂Et | |
| 4-137 | CH₂OCH₂OEt | Me | SO₂Et | |
| 4-138 | CH₂OCH₂OEt | Et | SO₂Et | |
| 4-139 | CH₂OCH₂OEt | n-Pr | SO₂Et | |
| 4-140 | CH₂OCH₂OEt | i-Pr | SO₂Et | |
| 4-141 | CH₂OCH₂CH₂SO₂Me | Me | SO₂Et | |
| 4-142 | CH₂OCH₂CH₂SO₂Me | Et | SO₂Et | |
| 4-143 | CH₂OCH₂CH₂SO₂Me | n-Pr | SO₂Et | |
| 4-144 | CH₂OCH₂CH₂SO₂Me | i-Pr | SO₂Et | |
| 4-145 | CH₂SO₂CH₂CH₂OMe | Me | SO₂Et | |
| 4-146 | CH₂SO₂CH₂CH₂OMe | Et | SO₂Et | |
| 4-147 | CH₂SO₂CH₂CH₂OMe | n-Pr | SO₂Et | |
| 4-148 | CH₂SO₂CH₂CH₂OMe | i-Pr | SO₂Et | |
| 4-149 | CH₂SO₂CH₂CH₂SO₂Me | Me | SO₂Et | |
| 4-150 | CH₂SO₂CH₂CH₂SO₂Me | Et | SO₂Et | |
| 4-151 | CH₂SO₂CH₂CH₂SO₂Me | n-Pr | SO₂Et | |
| 4-152 | CH₂SO₂CH₂CH₂SO₂Me | i-Pr | SO₂Et | |
| 4-153 | Cl | Me | F | |
| 4-154 | Cl | Et | F | |
| 4-155 | Cl | n-Pr | F | |
| 4-156 | Cl | i-Pr | F | |
| 4-157 | Br | Me | F | |
| 4-158 | Br | Et | F | |
| 4-159 | Br | n-Pr | F | |
| 4-160 | Br | i-Pr | F | |
| 4-161 | Me | Me | F | |
| 4-162 | Me | Et | F | |
| 4-163 | Me | n-Pr | F | |
| 4-164 | Me | i-Pr | F | |
| 4-165 | Et | Me | F | |
| 4-166 | Et | Et | F | |
| 4-167 | Et | n-Pr | F | |
| 4-168 | Et | i-Pr | F | |
| 4-169 | CF₃ | Me | F | |
| 4-170 | CF₃ | Et | F | |
| 4-171 | CF₃ | n-Pr | F | |
| 4-172 | CF₃ | i-Pr | F | |
| 4-173 | OMe | Me | F | |
| 4-174 | OMe | Et | F | |
| 4-175 | OMe | n-Pr | F | |
| 4-176 | OMe | i-Pr | F | |
| 4-177 | OEt | Me | F | |
| 4-178 | OEt | Et | F | |
| 4-179 | OEt | n-Pr | F | |
| 4-180 | OEt | i-Pr | F | |
| 4-181 | NO₂ | Me | F | |
| 4-182 | NO₂ | Et | F | |
| 4-183 | NO₂ | n-Pr | F | |
| 4-184 | NO₂ | i-Pr | F | |
| 4-185 | SO₂Me | Me | F | |
| 4-186 | SO₂Me | Et | F | |
| 4-187 | SO₂Me | n-Pr | F | |
| 4-188 | SO₂Me | i-Pr | F | |
| 4-189 | CH₂OMe | Me | F | |
| 4-190 | CH₂OMe | Et | F | |
| 4-191 | CH₂OMe | n-Pr | F | |
| 4-192 | CH₂OMe | i-Pr | F | |
| 4-193 | CH₂SO₂Me | Me | F | |
| 4-194 | CH₂SO₂Me | Et | F | |
| 4-195 | CH₂SO₂Me | n-Pr | F | |
| 4-196 | CH₂SO₂Me | i-Pr | F | |
| 4-197 | CH₂OCH₂CH₂OMe | Me | F | |
| 4-198 | CH₂OCH₂CH₂OMe | Et | F | |
| 4-199 | CH₂OCH₂CH₂OMe | n-Pr | F | |
| 4-200 | CH₂OCH₂CH₂OMe | i-Pr | F | |
| 4-201 | CH₂OCH₂CH₂OEt | Me | F | |
| 4-202 | CH₂OCH₂CH₂OEt | Et | F | |
| 4-203 | CH₂oCH₂CH₂OEt | n-Pr | F | |
| 4-204 | CH₂OCH₂CH₂OEt | i-Pr | F | |
| 4-205 | CH₂OCH₂CH₂CH₂OMe | Me | F | |
| 4-206 | CH₂OCH₂CH₂CH₂OMe | Et | F | |
| 4-207 | CH₂OCH₂CH₂CH₂OMe | n-Pr | F | |
| 4-208 | CH₂OCH₂CH₂CH₂OMe | i-Pr | F | |
| 4-209 | CH₂OCH₂OMe | Me | F | |
| 4-210 | CH₂OCH₂OMe | Et | F | |
| 4-211 | CH₂OCH₂OMe | n-Pr | F | |
| 4-212 | CH₂OCH₂OMe | i-Pr | F | |
| 4-213 | CH₂OCH₂OEt | Me | F | |
| 4-214 | CH₂OCH₂OEt | Et | F | |
| 4-215 | CH₂OCH₂OEt | n-Pr | F | |
| 4-216 | CH₂OCH₂OEt | i-Pr | F | |
| 4-217 | CH₂OCH₂CH₂SO₂Me | Me | F | |
| 4-218 | CH₂OCH₂CH₂SO₂Me | Et | F | |
| 4-219 | CH₂OCH₂CH₂SO₂Me | n-Pr | F | |
| 4-220 | CH₂OCH₂CH₂SO₂Me | i-Pr | F | |
| 4-221 | CH₂SO₂CH₂CH₂OMe | Me | F | |
| 4-222 | CH₂SO₂CH₂CH₂OMe | Et | F | |
| 4-223 | CH₂SO₂CH₂CH₂OMe | n-Pr | F | |
| 4-224 | CH₂SO₂CH₂CH₂OMe | i-Pr | F | |
| 4-225 | CH₂SO₂CH₂CH₂SO₂Me | Me | F | |
| 4-226 | CH₂SO₂CH₂CH₂SO₂Me | Et | F | |
| 4-227 | CH₂SO₂CH₂CH₂SO₂Me | n-Pr | F | |
| 4-228 | CH₂SO₂CH₂CH₂SO₂Me | i-Pr | F | |
| 4-229 | Cl | Me | Cl | |
| 4-230 | Cl | Et | Cl | |
| 4-231 | Cl | n-Pr | Cl | |
| 4-232 | Cl | i-Pr | Cl | |
| 4-233 | Br | Me | Cl | |
| 4-234 | Br | Et | Cl | |
| 4-235 | Br | n-Pr | Cl | |
| 4-236 | Br | i-Pr | Cl | |
| 4-237 | Me | Me | Cl | (DMSO-d₆): 7.77 (d, 1H), 7.47 (d, 1H), 3.01 (s, 3H), 2.78 (s, 3H) |
| 4-238 | Me | Et | Cl | 7.92 (d, 1H), 7.35 (d, 1H), 3.39 (m, 1H), 3.16(m, 1H), 2.92 (s, 3H), 1.38 (t, 3H) |
| 4-239 | Me | n-Pr | Cl | |
| 4-240 | Me | i-Pr | Cl | |
| 4-241 | Et | Me | Cl | |
| 4-242 | Et | Et | Cl | |
| 4-243 | Et | n-Pr | Cl | |
| 4-244 | Et | i-Pr | Cl | |
| 4-245 | CF₃ | Me | Cl | |
| 4-246 | CF₃ | Et | Cl | |
| 4-247 | CF₃ | n-Pr | Cl | |
| 4-248 | CF₃ | i-Pr | Cl | |
| 4-249 | OMe | Me | Cl | |
| 4-250 | OMe | Et | Cl | |
| 4-251 | OMe | n-Pr | Cl | |
| 4-252 | OMe | i-Pr | Cl | |
| 4-253 | OEt | Me | Cl | |
| 4-254 | OEt | Et | Cl | |
| 4-255 | OEt | n-Pr | Cl | |
| 4-256 | OEt | i-Pr | Cl | |
| 4-257 | NO₂ | Me | Cl | |
| 4-258 | NO₂ | Et | Cl | |
| 4-259 | NO₂ | n-Pr | Cl | |
| 4-260 | NO₂ | i-Pr | Cl | |
| 4-261 | SO₂Me | Me | Cl | |
| 4-262 | SO₂Me | Et | Cl | |
| 4-263 | SO₂Me | n-Pr | Cl | |
| 4-264 | SO₂Me | i-Pr | Cl | |
| 4-265 | CH₂OMe | Me | Cl | |
| 4-266 | CH₂OMe | Et | Cl | |
| 4-267 | CH₂OMe | n-Pr | Cl | |
| 4-268 | CH₂OMe | i-Pr | Cl | |
| 4-269 | CH₂SO₂Me | Me | Cl | |
| 4-270 | CH₂SO₂Me | Et | Cl | |
| 4-271 | CH₂SO₂Me | n-Pr | Cl | |
| 4-272 | CH₂SO₂Me | i-Pr | Cl | |
| 4-273 | CH₂OCH₂CH₂OMe | Me | Cl | |
| 4-274 | CH₂OCH₂CH₂OMe | Et | Cl | |
| 4-275 | CH₂OCH₂CH₂OMe | n-Pr | Cl | |
| 4-276 | CH₂OCH₂CH₂OMe | i-Pr | Cl | |
| 4-277 | CH₂OCH₂CH₂OEt | Me | Cl | |
| 4-278 | CH₂OCH₂CH₂OEt | Et | Cl | |
| 4-279 | CH₂OCH₂CH₂OEt | n-Pr | Cl | |
| 4-280 | CH₂OCH₂CH₂OEt | i-Pr | Cl | |
| 4-281 | CH₂OCH₂CH₂CH₂OMe | Me | Cl | |
| 4-282 | CH₂OCH₂CH₂CH₂OMe | Et | Cl | |
| 4-283 | CH₂OCH₂CH₂CH₂OMe | n-Pr | Cl | |
| 4-284 | CH₂OCH₂CH₂CH₂OMe | i-Pr | Cl | |
| 4-285 | CH₂OCH₂OMe | Me | Cl | |
| 4-286 | CH₂OCH₂OMe | Et | Cl | |
| 4-287 | CH₂OCH₂OMe | n-Pr | Cl | |
| 4-288 | CH₂OCH₂OMe | i-Pr | Cl | |
| 4-289 | CH₂OCH₂OEt | Me | Cl | |
| 4-290 | CH₂OCH₂OEt | Et | Cl | |
| 4-291 | CH₂OCH₂OEt | n-Pr | Cl | |
| 4-292 | CH₂OCH₂OEt | i-Pr | Cl | |
| 4-293 | CH₂OCH₂CH₂SO₂Me | Me | Cl | |
| 4-294 | CH₂OCH₂CH₂SO₂Me | Et | Cl | |
| 4-295 | CH₂oCH₂CH₂SO₂Me | n-Pr | Cl | |
| 4-296 | CH₂OCH₂CH₂SO₂Me | i-Pr | Cl | |
| 4-297 | CH₂SO₂CH₂CH₂OMe | Me | Cl | |
| 4-298 | CH₂SO₂CH₂CH₂OMe | Et | Cl | |
| 4-299 | CH₂SO₂CH₂CH₂OMe | n-Pr | Cl | |
| 4-300 | CH₂SO₂CH₂CH₂OMe | i-Pr | Cl | |
| 4-301 | CH₂SO₂CH₂CH₂SO₂Me | Me | Cl | |
| 4-302 | CH₂SO₂CH₂CH₂SO₂Me | Et | Cl | |
| 4-303 | CH₂SO₂CH₂CH₂SO₂Me | n-Pr | Cl | |
| 4-304 | CH₂SO₂CH₂CH₂SO₂Me | i-Pr | Cl | |
| 4-305 | Cl | Me | Br | |
| 4-306 | Cl | Et | Br | |
| 4-307 | Cl | n-Pr | Br | |
| 4-308 | Cl | i-Pr | Br | |
| 4-309 | Br | Me | Br | |
| 4-310 | Br | Et | Br | |
| 4-311 | Br | n-Pr | Br | |
| 4-312 | Br | i-Pr | Br | |
| 4-313 | Me | Me | Br | (DMSO-d₆): 7.66 (d, 1H), 7.64 (d, 1H), 2.99 (s, 3H), 2.80 (s, 3H) |
| 4-314 | Me | Et | Br | |
| 4-315 | Me | n-Pr | Br | |
| 4-316 | Me | i-Pr | Br | |
| 4-317 | Et | Me | Br | |
| 4-318 | Et | Et | Br | |
| 4-319 | Et | n-Pr | Br | |
| 4-320 | Et | i-Pr | Br | |
| 4-321 | CF₃ | Me | Br | |
| 4-322 | CF₃ | Et | Br | |
| 4-323 | CF₃ | n-Pr | Br | |
| 4-324 | CF₃ | i-Pr | Br | |
| 4-325 | OMe | Me | Br | |
| 4-326 | OMe | Et | Br | |
| 4-327 | OMe | n-Pr | Br | |
| 4-328 | OMe | i-Pr | Br | |
| 4-329 | OEt | Me | Br | |
| 4-330 | OEt | Et | Br | |
| 4-331 | OEt | n-Pr | Br | |
| 4-332 | OEt | i-Pr | Br | |
| 4-333 | NO₂ | Me | Br | |
| 4-334 | NO₂ | Et | Br | |
| 4-335 | NO₂ | n-Pr | Br | |
| 4-336 | NO₂ | i-Pr | Br | |
| 4-337 | SO₂Me | Me | Br | |
| 4-338 | SO₂Me | Et | Br | |
| 4-339 | SO₂Me | n-Pr | Br | |
| 4-340 | SO₂Me | i-Pr | Br | |
| 4-341 | CH₂OMe | Me | Br | |
| 4-342 | CH₂OMe | Et | Br | |
| 4-343 | CH₂OMe | n-Pr | Br | |
| 4-344 | CH₂OMe | i-Pr | Br | |
| 4-345 | CH₂SO₂Me | Me | Br | |
| 4-346 | CH₂SO₂Me | Et | Br | |
| 4-347 | CH₂SO₂Me | n-Pr | Br | |
| 4-348 | CH₂SO₂Me | i-Pr | Br | |
| 4-349 | CH₂OCH₂CH₂OMe | Me | Br | |
| 4-350 | CH₂OCH₂CH₂OMe | Et | Br | |
| 4-351 | CH₂OCH₂CH₂OMe | n-Pr | Br | |
| 4-352 | CH₂OCH₂CH₂OMe | i-Pr | Br | |
| 4-353 | CH₂OCH₂CH₂OEt | Me | Br | |
| 4-354 | CH₂OCH₂CH₂OEt | Et | Br | |
| 4-355 | CH₂OCH₂CH₂OEt | n-Pr | Br | |
| 4-356 | CH₂OCH₂CH₂OEt | i-Pr | Br | |
| 4-357 | CH₂OCH₂CH₂CH₂OMe | Me | Br | |
| 4-358 | CH₂OCH₂CH₂CH₂OMe | Et | Br | |
| 4-359 | CH₂OCH₂CH₂CH₂OMe | n-Pr | Br | |
| 4-360 | CH₂OCH₂CH₂CH₂OMe | i-Pr | Br | |
| 4-361 | CH₂OCH₂OMe | Me | Br | |
| 4-362 | CH₂OCH₂OMe | Et | Br | |
| 4-363 | CH₂OCH₂OMe | n-Pr | Br | |
| 4-364 | CH₂OCH₂OMe | i-Pr | Br | |
| 4-365 | CH₂OCH₂OEt | Me | Br | |
| 4-366 | CH₂OCH₂OEt | Et | Br | |
| 4-367 | CH₂OCH₂OEt | n-Pr | Br | |
| 4-368 | CH₂OCH₂OEt | i-Pr | Br | |
| 4-369 | CH₂OCH₂CH₂SO₂Me | Me | Br | |
| 4-370 | CH₂OCH₂CH₂SO₂Me | Et | Br | |
| 4-371 | CH₂OCH₂CH₂SO₂Me | n-Pr | Br | |
| 4-372 | CH₂OCH₂CH₂SO₂Me | i-Pr | Br | |
| 4-373 | CH₂SO₂CH₂CH₂OMe | Me | Br | |
| 4-374 | CH₂SO₂CH₂CH₂OMe | Et | Br | |
| 4-375 | CH₂SO₂CH₂CH₂OMe | n-Pr | Br | |
| 4-376 | CH₂SO₂CH₂CH₂OMe | i-Pr | Br | |
| 4-377 | CH₂SO₂CH₂CH₂SO₂Me | Me | Br | |
| 4-378 | CH₂SO₂CH₂CH₂SO₂Me | Et | Br | |
| 4-379 | CH₂SO₂CH₂CH₂SO₂Me | n-Pr | Br | |
| 4-380 | CH₂SO₂CH₂CH₂SO₂Me | i-Pr | Br | |
| 4-381 | Cl | Me | I | |
| 4-382 | Cl | Et | I | |
| 4-383 | Cl | n-Pr | I | |
| 4-384 | Cl | i-Pr | I | |
| 4-385 | Br | Me | I | |
| 4-386 | Br | Et | I | |
| 4-387 | Br | n-Pr | I | |
| 4-388 | Br | i-Pr | I | |
| 4-389 | Me | Me | I | 7.87 (d, 1H), 7.61 (d, 1H), 2.99 (s, 3H), 2.98 (s, 3H) |
| 4-390 | Me | Et | I | |
| 4-391 | Me | n-Pr | I | |
| 4-392 | Me | i-Pr | I | |
| 4-393 | Et | Me | I | |
| 4-394 | Et | Et | I | |
| 4-395 | Et | n-Pr | I | |
| 4-396 | Et | i-Pr | I | |
| 4-397 | CF₃ | Me | I | |
| 4-398 | CF₃ | Et | I | |
| 4-399 | CF₃ | n-Pr | I | |
| 4-400 | CF₃ | i-Pr | I | |
| 4-401 | OMe | Me | I | |
| 4-402 | OMe | Et | I | |
| 4-403 | OMe | n-Pr | I | |
| 4-404 | OMe | i-Pr | I | |
| 4-405 | OEt | Me | I | |
| 4-406 | OEt | Et | I | |
| 4-407 | OEt | n-Pr | I | |
| 4-408 | OEt | i-Pr | I | |
| 4-409 | NO₂ | Me | I | |
| 4-410 | NO₂ | Et | I | |
| 4-411 | NO₂ | n-Pr | I | |
| 4-412 | NO₂ | i-Pr | I | |
| 4-413 | SO₂Me | Me | I | |
| 4-414 | SO₂Me | Et | I | |
| 4-415 | SO₂Me | n-Pr | I | |
| 4-416 | SO₂Me | i-Pr | I | |
| 4-417 | CH₂OMe | Me | I | |
| 4-418 | CH₂OMe | Et | I | |
| 4-419 | CH₂OMe | n-Pr | I | |
| 4-420 | CH₂OMe | i-Pr | I | |
| 4-421 | CH₂SO₂Me | Me | I | |
| 4-422 | CH₂SO₂Me | Et | I | |
| 4-423 | CH₂SO₂Me | n-Pr | I | |
| 4-424 | CH₂SO₂Me | i-Pr | I | |
| 4-425 | CH₂OCH₂CH₂OMe | Me | I | |
| 4-426 | CH₂OCH₂CH₂OMe | Et | I | |
| 4-427 | CH₂OCH₂CH₂OMe | n-Pr | I | |
| 4-428 | CH₂OCH₂CH₂OMe | i-Pr | I | |
| 4-429 | CH₂OCH₂CH₂OEt | Me | I | |
| 4-430 | CH₂OCH₂CH₂OEt | Et | I | |
| 4-431 | CH₂OCH₂CH₂OEt | n-Pr | I | |
| 4-432 | CH₂OCH₂CH₂OEt | i-Pr | I | |
| 4-433 | CH₂OCH₂CH₂CH₂OMe | Me | I | |
| 4-434 | CH₂OCH₂CH₂CH₂OMe | Et | I | |
| 4-435 | CH₂OCH₂CH₂CH₂OMe | n-Pr | I | |
| 4-436 | CH₂OCH₂CH₂CH₂OMe | i-Pr | I | |
| 4-437 | CH₂OCH₂OMe | Me | I | |
| 4-438 | CH₂OCH₂OMe | Et | I | |
| 4-439 | CH₂OCH₂OMe | n-Pr | I | |
| 4-440 | CH₂OCH₂OMe | i-Pr | I | |
| 4-441 | CH₂OCH₂OEt | Me | I | |
| 4-442 | CH₂OCH₂OEt | Et | I | |
| 4-443 | CH₂OCH₂OEt | n-Pr | I | |
| 4-444 | CH₂OCH₂OEt | i-Pr | I | |
| 4-445 | CH₂OCH₂CH₂SO₂Me | Me | I | |
| 4-446 | CH₂OCH₂CH₂SO₂Me | Et | I | |
| 4-447 | CH₂OCH₂CH₂SO₂Me | n-Pr | I | |
| 4-448 | CH₂OCH₂CH₂SO₂Me | i-Pr | I | |
| 4-449 | CH₂SO₂CH₂CH₂OMe | Me | I | |
| 4-450 | CH₂SO₂CH₂CH₂OMe | Et | I | |
| 4-451 | CH₂SO₂CH₂CH₂OMe | n-Pr | I | |
| 4-452 | CH₂SO₂CH₂CH₂OMe | i-Pr | I | |
| 4-453 | CH₂SO₂CH₂CH₂SO₂Me | Me | I | |
| 4-454 | CH₂SO₂CH₂CH₂SO₂Me | Et | I | |
| 4-455 | CH₂SO₂CH₂CH₂SO₂Me | n-Pr | I | |
| 4-456 | CH₂SO₂CH₂CH₂SO₂Me | i-Pr | I | |
| 4-457 | Cl | Me | NO₂ | |
| 4-458 | Cl | Et | NO₂ | |
| 4-459 | Cl | n-Pr | NO₂ | |
| 4-460 | Cl | i-Pr | NO₂ | |
| 4-461 | Br | Me | NO₂ | |
| 4-462 | Br | Et | NO₂ | |
| 4-463 | Br | n-Pr | NO₂ | |
| 4-464 | Br | i-Pr | NO₂ | |
| 4-465 | Me | Me | NO₂ | |
| 4-466 | Me | Et | NO₂ | |
| 4-467 | Me | n-Pr | NO₂ | |
| 4-468 | Me | i-Pr | NO₂ | |
| 4-469 | Et | Me | NO₂ | |
| 4-470 | Et | Et | NO₂ | |
| 4-471 | Et | n-Pr | NO₂ | |
| 4-472 | Et | i-Pr | NO₂ | |
| 4-473 | CF₃ | Me | NO₂ | |
| 4-474 | CF₃ | Et | NO₂ | |
| 4-475 | CF₃ | n-Pr | NO₂ | |
| 4-476 | CF₃ | i-Pr | NO₂ | |
| 4-477 | OMe | Me | NO₂ | |
| 4-478 | OMe | Et | NO₂ | |
| 4-479 | OMe | n-Pr | NO₂ | |
| 4-480 | OMe | i-Pr | NO₂ | |
| 4-481 | OEt | Me | NO₂ | |
| 4-482 | OEt | Et | NO₂ | |
| 4-483 | OEt | n-Pr | NO₂ | |
| 4-484 | OEt | i-Pr | NO₂ | |
| 4-485 | NO₂ | Me | NO₂ | |
| 4-486 | NO₂ | Et | NO₂ | |
| 4-487 | NO₂ | n-Pr | NO₂ | |
| 4-488 | NO₂ | i-Pr | NO₂ | |
| 4-489 | SO₂Me | Me | NO₂ | |
| 4-490 | SO₂Me | Et | NO₂ | |
| 4-491 | SO₂Me | n-Pr | NO₂ | |
| 4-492 | SO₂Me | i-Pr | NO₂ | |
| 4-493 | CH₂OMe | Me | NO₂ | |
| 4-494 | CH₂OMe | Et | NO₂ | |
| 4-495 | CH₂OMe | n-Pr | NO₂ | |
| 4-496 | CH₂OMe | i-Pr | NO₂ | |
| 4-497 | CH₂SO₂Me | Me | NO₂ | |
| 4-498 | CH₂SO₂Me | Et | NO₂ | |
| 4-499 | CH₂SO₂Me | n-Pr | NO₂ | |
| 4-500 | CH₂SO₂Me | i-Pr | NO₂ | |
| 4-501 | CH₂OCH₂CH₂OMe | Me | NO₂ | |
| 4-502 | CH₂OCH₂CH₂OMe | Et | NO₂ | |
| 4-503 | CH₂OCH₂CH₂OMe | n-Pr | NO₂ | |
| 4-504 | CH₂OCH₂CH₂OMe | i-Pr | NO₂ | |
| 4-505 | CH₂OCH₂CH₂OEt | Me | NO₂ | |
| 4-506 | CH₂OCH₂CH₂OEt | Et | NO₂ | |
| 4-507 | CH₂OCH₂CH₂OEt | n-Pr | NO₂ | |
| 4-508 | CH₂OCH₂CH₂OEt | i-Pr | NO₂ | |
| 4-509 | CH₂OCH₂CH₂CH₂OMe | Me | NO₂ | |
| 4-510 | CH₂OCH₂CH₂CH₂OMe | Et | NO₂ | |
| 4-511 | CH₂OCH₂CH₂CH₂OMe | n-Pr | NO₂ | |
| 4-512 | CH₂OCH₂CH₂CH₂OMe | i-Pr | NO₂ | |
| 4-513 | CH₂OCH₂OMe | Me | NO₂ | |
| 4-514 | CH₂OCH₂OMe | Et | NO₂ | |
| 4-515 | CH₂OCH₂OMe | n-Pr | NO₂ | |
| 4-516 | CH₂OCH₂OMe | i-Pr | NO₂ | |
| 4-517 | CH₂OCH₂OEt | Me | NO₂ | |
| 4-518 | CH₂OCH₂OEt | Et | NO₂ | |
| 4-519 | CH₂OCH₂OEt | n-Pr | NO₂ | |
| 4-520 | CH₂OCH₂OEt | i-Pr | NO₂ | |
| 4-521 | CH₂OCH₂CH₂SO₂Me | Me | NO₂ | |
| 4-522 | CH₂OCH₂CH₂SO₂Me | Et | NO₂ | |
| 4-523 | CH₂OCH₂CH₂SO₂Me | n-Pr | NO₂ | |
| 4-524 | CH₂OCH₂CH₂SO₂Me | i-Pr | NO₂ | |
| 4-525 | CH₂SO₂CH₂CH₂OMe | Me | NO₂ | |
| 4-526 | CH₂SO₂CH₂CH₂OMe | Et | NO₂ | |
| 4-527 | CH₂SO₂CH₂CH₂OMe | n-Pr | NO₂ | |
| 4-528 | CH₂SO₂CH₂CH₂OMe | i-Pr | NO₂ | |
| 4-529 | CH₂SO₂CH₂CH₂SO₂Me | Me | NO₂ | |
| 4-530 | CH₂SO₂CH₂CH₂SO₂Me | Et | NO₂ | |
| 4-531 | CH₂SO₂CH₂CH₂SO₂Me | n-Pr | NO₂ | |
| 4-532 | CH₂SO₂CH₂CH₂SO₂Me | i-Pr | NO₂ | |

**Tabelle 5: Erfindungsgemäße Verbindungen der allgemeinen Formel (I), worin R¹ für Methyl und R² für Wasserstoff stehen.**

| | | | | | |
|---|---|---|---|---|---|
| 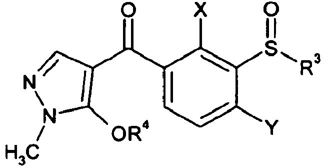 | | | | | |

| Nr. | X | R³ | Y | R⁴ | Physikalische Daten: ¹H-NMR: δ [CDCl₃] |
|---|---|---|---|---|---|
| 5-1 | Cl | Me | SO₂Me | - SO₂-n-Pr | |
| 5-2 | Cl | Et | SO₂Me | - SO₂-n-Pr | |
| 5-3 | Me | Me | SO₂Me | - SO₂-n-Pr | |
| 5-4 | Me | Et | SO₂Me | - SO₂-n-Pr | |
| 5-5 | CF₃ | Me | SO₂Me | - SO₂-n-Pr | |
| 5-6 | CF₃ | Et | SO₂Me | - SO₂-n-Pr | |
| 5-7 | OMe | Me | SO₂Me | - SO₂-n-Pr | |
| 5-8 | OMe | Et | SO₂Me | - SO₂-n-Pr | |
| 5-9 | NO₂ | Me | SO₂Me | - SO₂-n-Pr | |
| 5-10 | NO₂ | Et | SO₂Me | - SO₂-n-Pr | |
| 5-11 | SO₂Me | Me | SO₂Me | - SO₂-n-Pr | |
| 5-12 | SO₂Me | Et | SO₂Me | - SO₂-n-Pr | |
| 5-13 | CH₂OMe | Me | SO₂Me | - SO₂-n-Pr | |
| 5-14 | CH₂OMe | Et | SO₂Me | - SO₂-n-Pr | |
| 5-15 | CH₂SO₂Me | Me | SO₂Me | - SO₂-n-Pr | |
| 5-16 | CH₂SO₂Me | Et | SO₂Me | - SO₂-n-Pr | |
| 5-17 | CH₂OCH₂CH₂OMe | Me | SO₂Me | - SO₂-n-Pr | |
| 5-18 | CH₂OCH₂CH₂OMe | Et | SO₂Me | - SO₂-n-Pr | |
| 5-19 | Cl | Me | Cl | - SO₂-n-Pr | |
| 5-20 | Cl | Et | Cl | - SO₂-n-Pr | |
| 5-21 | Me | Me | Cl | - SO₂-n-Pr | |
| 5-22 | Me | Et | Cl | - SO₂-n-Pr | |
| 5-23 | CF₃ | Me | Cl | - SO₂-n-Pr | |
| 5-24 | CF₃ | Et | Cl | - SO₂-n-Pr | |
| 5-25 | OMe | Me | Cl | - SO₂-n-Pr | |
| 5-26 | OMe | Et | Cl | - SO₂-n-Pr | |
| 5-27 | NO₂ | Me | Cl | - SO₂-n-Pr | |
| 5-28 | NO₂ | Et | Cl | - SO₂-n-Pr | |
| 5-29 | SO₂Me | Me | Cl | - SO₂-n-Pr | |
| 5-30 | SO₂Me | Et | Cl | - SO₂-n-Pr | |
| 5-31 | CH₂OMe | Me | Cl | - SO₂-n-Pr | |
| 5-32 | CH₂OMe | Et | Cl | - SO₂-n-Pr | |
| 5-33 | CH₂SO₂Me | Me | Cl | - SO₂-n-Pr | |
| 5-34 | CH₂SO₂Me | Et | Cl | - SO₂-n-Pr | |
| 5-35 | CH₂OCH₂CH₂OMe | Me | Cl | - SO₂-n-Pr | |
| 5-36 | CH₂oCH₂CH₂OMe | Et | Cl | - SO₂-n-Pr | |
| 5-37 | Cl | Me | Br | - SO₂-n-Pr | |
| 5-38 | Cl | Et | Br | - SO₂-n-Pr | |
| 5-39 | Me | Me | Br | - SO₂-n-Pr | |
| 5-40 | Me | Et | Br | - SO₂-n-Pr | |
| 5-41 | CF₃ | Me | Br | - SO₂-n-Pr | |
| 5-42 | CF₃ | Et | Br | - SO₂-n-Pr | |
| 5-43 | OMe | Me | Br | - SO₂-n-Pr | |
| 5-44 | OMe | Et | Br | - SO₂-n-Pr | |
| 5-45 | NO₂ | Me | Br | - SO₂-n-Pr | |
| 5-46 | NO₂ | Et | Br | - SO₂-n-Pr | |
| 5-47 | SO₂Me | Me | Br | - SO₂-n-Pr | |
| 5-48 | SO₂Me | Et | Br | - SO₂-n-Pr | |
| 5-49 | CH₂OMe | Me | Br | - SO₂-n-Pr | |
| 5-50 | CH₂OMe | Et | Br | - SO₂-n-Pr | |
| 5-51 | CH₂SO₂Me | Me | Br | - SO₂-n-Pr | |
| 5-52 | CH₂SO₂Me | Et | Br | - SO₂-n-Pr | |
| 5-53 | CH₂OCH₂CH₂OMe | Me | Br | - SO₂-n-Pr | |
| 5-54 | CH₂OCH₂CH₂OMe | Et | Br | - SO₂-n-Pr | |
| 5-55 | Cl | Me | I | - SO₂-n-Pr | |
| 5-56 | Cl | Et | I | - SO₂-n-Pr | |
| 5-57 | Me | Me | I | - SO₂-n-Pr | |
| 5-58 | Me | Et | I | - SO₂-n-Pr | |
| 5-59 | CF₃ | Me | I | - SO₂-n-Pr | |
| 5-60 | CF₃ | Et | I | - SO₂-n-Pr | |
| 5-61 | OMe | Me | I | - SO₂-n-Pr | |
| 5-62 | OMe | Et | I | - SO₂-n-Pr | |
| 5-63 | NO₂ | Me | I | - SO₂-n-Pr | |
| 5-64 | NO₂ | Et | I | - SO₂-n-Pr | |
| 5-65 | SO₂Me | Me | I | - SO₂-n-Pr | |
| 5-66 | SO₂Me | Et | I | - SO₂-n-Pr | |
| 5-67 | CH₂OMe | Me | I | - SO₂-n-Pr | |
| 5-68 | CH₂OMe | Et | I | - SO₂-n-Pr | |
| 5-69 | CH₂OS₂Me | Me | I | - SO₂-n-Pr | |
| 5-70 | CH₂SO₂Me | Et | I | - SO₂-n-Pr | |
| 5-71 | CH₂OCH₂CH₂OMe | Me | I | - SO₂-n-Pr | |
| 5-72 | CH₂OCH₂CH₂OMe | Et | I | - SO₂-n-Pr | |
| 5-73 | Cl | Me | NO₂ | - SO₂-n-Pr | |
| 5-74 | Cl | Et | NO₂ | - SO₂-n-Pr | |
| 5-75 | Me | Me | NO₂ | - SO₂-n-Pr | |
| 5-76 | Me | Et | NO₂ | - SO₂-n-Pr | |
| 5-77 | CF₃ | Me | NO₂ | - SO₂-n-Pr | |
| 5-78 | CF₃ | Et | NO₂ | - SO₂-n-Pr | |
| 5-79 | OMe | Me | NO₂ | - SO₂-n-Pr | |
| 5-80 | OMe | Et | NO₂ | - SO₂-n-Pr | |
| 5-81 | NO₂ | Me | NO₂ | - SO₂-n-Pr | |
| 5-82 | NO₂ | Et | NO₂ | - SO₂-n-Pr | |
| 5-83 | SO₂Me | Me | NO₂ | - SO₂-n-Pr | |
| 5-84 | SO₂Me | Et | NO₂ | - SO₂-n-Pr | |
| 5-85 | CH₂OMe | Me | NO₂ | - SO₂-n-Pr | |
| 5-86 | CH₂OMe | Et | NO₂ | - SO₂-n-Pr | |
| 5-87 | CH₂SO₂Me | Me | NO₂ | - SO₂-n-Pr | |
| 5-88 | CH₂SO₂Me | Et | NO₂ | - SO₂-n-Pr | |
| 5-89 | CH₂OCH₂CH₂OMe | Me | NO₂ | - SO₂-n-Pr | |
| 5-90 | CH₂OCH₂CH₂OMe | Et | NO₂ | - SO₂-n-Pr | |
| 5-91 | Cl | Me | SO₂Me | - CO-Ph | |
| 5-92 | Cl | Et | SO₂Me | - CO-Ph | |
| 5-93 | Me | Me | SO₂Me | - CO-Ph | |
| 5-94 | Me | Et | SO₂Me | - CO-Ph | |
| 5-95 | CF₃ | Me | SO₂Me | - CO-Ph | |
| 5-96 | CF₃ | Et | SO₂Me | - CO-Ph | |
| 5-97 | OMe | Me | SO₂Me | - CO-Ph | |
| 5-98 | OMe | Et | SO₂Me | - CO-Ph | |
| 5-99 | NO₂ | Me | SO₂Me | - CO-Ph | |
| 5-100 | NO₂ | Et | SO₂Me | - CO-Ph | |
| 5-101 | SO₂Me | Me | SO₂Me | - CO-Ph | |
| 5-102 | SO₂Me | Et | SO₂Me | - CO-Ph | |
| 5-103 | CH₂OMe | Me | SO₂Me | - CO-Ph | |
| 5-104 | CH₂OMe | Et | SO₂Me | - CO-Ph | |
| 5-105 | CH₂SO₂Me | Me | SO₂Me | - CO-Ph | |
| 5-106 | CH₂SO₂Me | Et | SO₂Me | - CO-Ph | |
| 5-107 | CH₂OCH₂CH₂OMe | Me | SO₂Me | - CO-Ph | |
| 5-108 | CH₂OCH₂CH₂OMe | Et | SO₂Me | - CO-Ph | |
| 5-109 | Cl | Me | Cl | - CO-Ph | |
| 5-110 | Cl | Et | Cl | - CO-Ph | |
| 5-111 | Me | Me | Cl | - CO-Ph | |
| 5-112 | Me | Et | Cl | - CO-Ph | |
| 5-113 | CF₃ | Me | Cl | - CO-Ph | |
| 5-114 | CF₃ | Et | Cl | - CO-Ph | |
| 5-115 | OMe | Me | Cl | - CO-Ph | |
| 5-116 | OMe | Et | Cl | - CO-Ph | |
| 5-117 | NO₂ | Me | Cl | - CO-Ph | |
| 5-118 | NO₂ | Et | Cl | - CO-Ph | |
| 5-119 | SO₂Me | Me | Cl | - CO-Ph | |
| 5-120 | SO₂Me | Et | Cl | - CO-Ph | |
| 5-121 | CH₂OMe | Me | Cl | - CO-Ph | |
| 5-122 | CH₂OMe | Et | Cl | - CO-Ph | |
| 5-123 | CH₂SO₂Me | Me | Cl | - CO-Ph | |
| 5-124 | CH₂SO₂Me | Et | Cl | - CO-Ph | |
| 5-125 | CH₂OCH₂CH₂OMe | Me | Cl | - CO-Ph | |
| 5-126 | CH₂OCH₂CH₂OMe | Et | Cl | - CO-Ph | |
| 5-127 | Cl | Me | Br | - CO-Ph | |
| 5-128 | Cl | Et | Br | - CO-Ph | |
| 5-129 | Me | Me | Br | - CO-Ph | |
| 5-130 | Me | Et | Br | - CO-Ph | |
| 5-131 | CF₃ | Me | Br | - CO-Ph | |
| 5-132 | CF₃ | Et | Br | - CO-Ph | |
| 5-133 | OMe | Me | Br | - CO-Ph | |
| 5-134 | OMe | Et | Br | - CO-Ph | |
| 5-135 | NO₂ | Me | Br | - CO-Ph | |
| 5-136 | NO₂ | Et | Br | - CO-Ph | |
| 5-137 | SO₂Me | Me | Br | - CO-Ph | |
| 5-138 | SO₂Me | Et | Br | - CO-Ph | |
| 5-139 | CH₂OMe | Me | Br | - CO-Ph | |
| 5-140 | CH₂OMe | Et | Br | - CO-Ph | |
| 5-141 | CH₂SO₂Me | Me | Br | - CO-Ph | |
| 5-142 | CH₂SO₂Me | Et | Br | - CO-Ph | |
| 5-143 | CH₂OCH₂CH₂OMe | Me | Br | - CO-Ph | |
| 5-144 | CH₂OCH₂CH₂OMe | Et | Br | - CO-Ph | |
| 5-145 | Cl | Me | I | - CO-Ph | |
| 5-146 | Cl | Et | I | - CO-Ph | |
| 5-147 | Me | Me | I | - CO-Ph | |
| 5-148 | Me | Et | I | - CO-Ph | |
| 5-149 | CF₃ | Me | I | - CO-Ph | |
| 5-150 | CF₃ | Et | I | - CO-Ph | |
| 5-151 | OMe | Me | I | - CO-Ph | |
| 5-152 | OMe | Et | I | - CO-Ph | |
| 5-153 | NO₂ | Me | I | - CO-Ph | |
| 5-154 | NO₂ | Et | I | - CO-Ph | |
| 5-155 | SO₂Me | Me | I | - CO-Ph | |
| 5-156 | SO₂Me | Et | I | - CO-Ph | |
| 5-157 | CH₂OMe | Me | I | - CO-Ph | |
| 5-158 | CH₂OMe | Et | I | - CO-Ph | |
| 5-159 | CH₂SO₂Me | Me | I | - CO-Ph | |
| 5-160 | CH₂SO₂Me | Et | I | - CO-Ph | |
| 5-161 | CH₂OCH₂CH₂OMe | Me | I | - CO-Ph | |
| 5-162 | CH₂OCH₂CH₂OMe | Et | I | - CO-Ph | |
| 5-163 | Cl | Me | NO₂ | - CO-Ph | |
| 5-164 | Cl | Et | NO₂ | - CO-Ph | |
| 5-165 | Me | Me | NO₂ | - CO-Ph | |
| 5-166 | Me | Et | NO₂ | - CO-Ph | |
| 5-167 | CF₃ | Me | NO₂ | - CO-Ph | |
| 5-168 | CF₃ | Et | NO₂ | - CO-Ph | |
| 5-169 | OMe | Me | NO₂ | - CO-Ph | |
| 5-170 | OMe | Et | NO₂ | - CO-Ph | |
| 5-171 | NO₂ | Me | NO₂ | - CO-Ph | |
| 5-172 | NO₂ | Et | NO₂ | - CO-Ph | |
| 5-173 | SO₂Me | Me | NO₂ | - CO-Ph | |
| 5-174 | SO₂Me | Et | NO₂ | - CO-Ph | |
| 5-175 | CH₂OMe | Me | NO₂ | - CO-Ph | |
| 5-176 | CH₂OMe | Et | NO₂ | - CO-Ph | |
| 5-177 | CH₂SO₂Me | Me | NO₂ | - CO-Ph | |
| 5-178 | CH₂SO₂Me | Et | NO₂ | - CO-Ph | |
| 5-179 | CH₂OCH₂CH₂OMe | Me | NO₂ | - CO-Ph | |
| 5-180 | CH₂OCH₂CH₂OMe | Et | NO₂ | - CO-Ph | |
| 5-181 | Cl | Me | SO₂Me | - CH₂-CO- (4-Me-Ph) | |
| 5-182 | Cl | Et | SO₂Me | - CH₂-CO- (4-Me-Ph) | |
| 5-183 | Me | Me | SO₂Me | - CH₂-CO- (4-Me-Ph) | |
| 5-184 | Me | Et | SO₂Me | - CH₂-CO- (4-Me-Ph) | |
| 5-185 | CF₃ | Me | SO₂Me | - CH₂-CO- (4-Me-Ph) | |
| 5-186 | CF₃ | Et | SO₂Me | - CH₂-CO- (4-Me-Ph) | |
| 5-187 | OMe | Me | SO₂Me | - CH₂-CO- (4-Me-Ph) | |
| 5-188 | OMe | Et | SO₂Me | - CH₂-CO- (4-Me-Ph) | |
| 5-189 | NO₂ | Me | SO₂Me | - CH₂-CO- (4-Me-Ph) | |
| 5-190 | NO₂ | Et | SO₂Me | - CH₂-CO- (4-Me-Ph) | |
| 5-191 | SO₂Me | Me | SO₂Me | - CH₂-CO- (4-Me-Ph) | |
| 5-192 | SO₂Me | Et | SO₂Me | - CH₂-CO- (4-Me-Ph) | |
| 5-193 | CH₂OMe | Me | SO₂Me | - CH₂-CO- (4-Me-Ph) | |
| 5-194 | CH₂OMe | Et | SO₂Me | - CH₂-CO- (4-Me-Ph) | |
| 5-195 | CH₂SO₂Me | Me | SO₂Me | - CH₂-CO- (4-Me-Ph) | |
| 5-196 | CH₂SO₂Me | Et | SO₂Me | - CH₂-CO- (4-Me-Ph) | |
| 5-197 | CH₂OCH₂CH₂OMe | Me | SO₂Me | - CH₂-CO- (4-Me-Ph) | |
| 5-198 | CH₂OCH₂CH₂OMe | Et | SO₂Me | - CH₂-CO- (4-Me-Ph) | |
| 5-199 | Cl | Me | Cl | - CH₂-CO- (4-Me-Ph) | |
| 5-200 | Cl | Et | Cl | - CH₂-CO- (4-Me-Ph) | |
| 5-201 | Me | Me | Cl | - CH₂-CO- (4-Me-Ph) | |
| 5-202 | Me | Et | Cl | - CH₂-CO- (4-Me-Ph) | |
| 5-203 | CF₃ | Me | Cl | - CH₂-CO- (4-Me-Ph) | |
| 5-204 | CF₃ | Et | Cl | - CH₂-CO- (4-Me-Ph) | |
| 5-205 | OMe | Me | Cl | - CH₂-CO- (4-Me-Ph) | |
| 5-206 | OMe | Et | Cl | - CH₂-CO- (4-Me-Ph) | |
| 5-207 | NO₂ | Me | Cl | - CH₂-CO- (4-Me-Ph) | |
| 5-208 | NO₂ | Et | Cl | - CH₂-CO- (4-Me-Ph) | |
| 5-209 | SO₂Me | Me | Cl | - CH₂-CO- (4-Me-Ph) | |
| 5-210 | SO₂Me | Et | Cl | - CH₂-CO- (4-Me-Ph) | |
| 5-211 | CH₂OMe | Me | Cl | - CH₂-CO- (4-Me-Ph) | |
| 5-212 | CH₂OMe | Et | Cl | - CH₂-CO- (4-Me-Ph) | |
| 5-213 | CH₂SO₂Me | Me | Cl | - CH₂-CO- (4-Me-Ph) | |
| 5-214 | CH₂SO₂Me | Et | Cl | - CH₂-CO- (4-Me-Ph) | |
| 5-215 | CH₂OCH₂CH₂OMe | Me | Cl | - CH₂-CO- (4-Me-Ph) | |
| 5-216 | CH₂OCH₂CH₂OMe | Et | Cl | - CH₂-CO- (4-Me-Ph) | |
| 5-217 | Cl | Me | Br | - CH₂-CO- (4-Me-Ph) | |
| 5-218 | Cl | Et | Br | - CH₂-CO- (4-Me-Ph) | |
| 5-219 | Me | Me | Br | - CH₂-CO- (4-Me-Ph) | |
| 5-220 | Me | Et | Br | - CH₂-CO- (4-Me-Ph) | |
| 5-221 | CF₃ | Me | Br | - CH₂-CO- (4-Me-Ph) | |
| 5-222 | CF₃ | Et | Br | - CH₂-CO- (4-Me-Ph) | |
| 5-223 | OMe | Me | Br | - CH₂-CO- (4-Me-Ph) | |
| 5-224 | OMe | Et | Br | - CH₂-CO- (4-Me-Ph) | |
| 5-225 | NO₂ | Me | Br | - CH₂-CO- (4-Me-Ph) | |
| 5-226 | NO₂ | Et | Br | - CH₂-CO- (4-Me-Ph) | |
| 5-227 | SO₂Me | Me | Br | - CH₂-CO- (4-Me-Ph) | |
| 5-228 | SO₂Me | Et | Br | - CH₂-CO- (4-Me-Ph) | |
| 5-229 | CH₂OMe | Me | Br | - CH₂-CO- (4-Me-Ph) | |
| 5-230 | CH₂OMe | Et | Br | - CH₂-CO- (4-Me-Ph) | |
| 5-231 | CH₂SO₂Me | Me | Br | - CH₂-CO- (4-Me-Ph) | |
| 5-232 | CH₂SO₂Me | Et | Br | - CH₂-CO- (4-Me-Ph) | |
| 5-233 | CH₂OCH₂CH₂OMe | Me | Br | - CH₂-CO- (4-Me-Ph) | |
| 5-234 | CH₂OCH₂CH₂OMe | Et | Br | - CH₂-CO- (4-Me-Ph) | |
| 5-235 | Cl | Me | I | - CH₂-CO- (4-Me-Ph) | |
| 5-236 | Cl | Et | I | - CH₂-CO- (4-Me-Ph) | |
| 5-237 | Me | Me | I | - CH₂-CO- (4-Me-Ph) | |
| 5-238 | Me | Et | I | - CH₂-CO- (4-Me-Ph) | |
| 5-239 | CF₃ | Me | I | - CH₂-CO- (4-Me-Ph) | |
| 5-240 | CF₃ | Et | I | - CH₂-CO- (4-Me-Ph) | |
| 5-241 | OMe | Me | I | - CH₂-CO- (4-Me-Ph) | |
| 5-242 | OMe | Et | I | - CH₂-CO- (4-Me-Ph) | |
| 5-243 | NO₂ | Me | I | - CH₂-CO- (4-Me-Ph) | |
| 5-244 | NO₂ | Et | I | - CH₂-CO- (4-Me-Ph) | |
| 5-245 | SO₂Me | Me | I | - CH₂-CO- (4-Me-Ph) | |
| 5-246 | SO₂Me | Et | I | - CH₂-CO- (4-Me-Ph) | |
| 5-247 | CH₂OMe | Me | I | - CH₂-CO- (4-Me-Ph) | |
| 5-248 | CH₂OMe | Et | I | - CH₂-CO- (4-Me-Ph) | |
| 5-249 | CH₂SO₂Me | Me | I | - CH₂-CO- (4-Me-Ph) | |
| 5-250 | CH₂SO₂Me | Et | I | - CH₂-CO- (4-Me-Ph) | |
| 5-251 | CH₂OCH₂CH₂OMe | Me | I | - CH₂-CO- (4-Me-Ph) | |
| 5-252 | CH₂OCH₂CH₂OMe | Et | I | - CH₂-CO- (4-Me-Ph) | |
| 5-253 | Cl | Me | NO₂ | - CH₂-CO- (4-Me-Ph) | |
| 5-254 | Cl | Et | NO₂ | - CH₂-CO- (4-Me-Ph) | |
| 5-255 | Me | Me | NO₂ | - CH₂-CO- (4-Me-Ph) | |
| 5-256 | Me | Et | NO₂ | - CH₂-CO- (4-Me-Ph) | |
| 5-257 | CF₃ | Me | NO₂ | - CH₂-CO- (4-Me-Ph) | |
| 5-258 | CF₃ | Et | NO₂ | - CH₂-CO- (4-Me-Ph) | |
| 5-259 | OMe | Me | NO₂ | - CH₂-CO- (4-Me-Ph) | |
| 5-260 | OMe | Et | NO₂ | - CH₂-CO- (4-Me-Ph) | |
| 5-261 | NO₂ | Me | NO₂ | - CH₂-CO- (4-Me-Ph) | |
| 5-262 | NO₂ | Et | NO₂ | - CH₂-CO- (4-Me-Ph) | |
| 5-263 | SO₂Me | Me | NO₂ | - CH₂-CO- (4-Me-Ph) | |
| 5-264 | SO₂Me | Et | NO₂ | - CH₂-CO- (4-Me-Ph) | |
| 5-265 | CH₂OMe | Me | NO₂ | - CH₂-CO- (4-Me-Ph) | |
| 5-266 | CH₂OMe | Et | NO₂ | - CH₂-CO- (4-Me-Ph) | |
| 5-267 | CH₂SO₂Me | Me | NO₂ | - CH₂-CO- (4-Me-Ph) | |
| 5-268 | CH₂SO₂Me | Et | NO₂ | - CH₂-CO-(4-Me-Ph) | |
| 5-269 | CH₂OCH₂CH₂OMe | Me | NO₂ | - CH₂-CO- (4-Me-Ph) | |
| 5-270 | CH₂OCH₂CH₂OMe | Et | NO₂ | - CH₂-CO- (4-Me-Ph) | |

**Tabelle 6: Erfindungsgemäße Verbindungen der allgemeinen Formel (1), worin R¹ für Ethyl und R² für Wasserstoff stehen.**

| | | | | | |
|---|---|---|---|---|---|
| 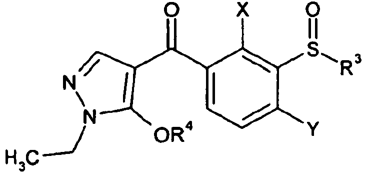 | | | | | |

| Nr. | X | R³ | Y | R⁴ | Physikalische Daten: ¹H-NMR: δ [CDCl₃] |
|---|---|---|---|---|---|
| 6-1 | Cl | Me | SO₂Me | - SO₂-n-Pr | |
| 6-2 | Cl | Et | SO₂Me | - SO₂-n-Pr | |
| 6-3 | Me | Me | SO₂Me | - SO₂-n-Pr | |
| 6-4 | Me | Et | SO₂Me | - SO₂-n-Pr | |
| 6-5 | CF₃ | Me | SO₂Me | - SO₂-n-Pr | |
| 6-6 | CF₃ | Et | SO₂Me | - SO₂-n-Pr | |
| 6-7 | OMe | Me | SO₂Me | - SO₂-n-Pr | |
| 6-8 | OMe | Et | SO₂Me | - SO₂-n-Pr | |
| 6-9 | NO₂ | Me | SO₂Me | - SO₂-n-Pr | |
| 6-10 | NO₂ | Et | SO₂Me | - SO₂-n-Pr | |
| 6-11 | SO₂Me | Me | SO₂Me | - SO₂-n-Pr | |
| 6-12 | SO₂Me | Et | SO₂Me | - SO₂-n-Pr | |
| 6-13 | CH₂OMe | Me | SO₂Me | - SO₂-n-Pr | |
| 6-14 | CH₂OMe | Et | SO₂Me | - SO₂-n-Pr | |
| 6-15 | CH₂SO₂Me | Me | SO₂Me | - SO₂-n-Pr | |
| 6-16 | CH₂SO₂Me | Et | SO₂Me | - SO₂-n-Pr | |
| 6-17 | CH₂OCH₂CH₂OMe | Me | SO₂Me | - SO₂-n-Pr | |
| 6-18 | CH₂OCH₂CH₂OMe | Et | SO₂Me | - SO₂-n-Pr | |
| 6-19 | Cl | Me | Cl | - SO₂-n-Pr | |
| 6-20 | Cl | Et | Cl | - SO₂-n-Pr | |
| 6-21 | Me | Me | Cl | - SO₂-n-Pr | |
| 6-22 | Me | Et | Cl | - SO₂-n-Pr | |
| 6-23 | CF₃ | Me | Cl | - SO₂-n-Pr | |
| 6-24 | CF₃ | Et | Cl | - SO₂-n-Pr | |
| 6-25 | OMe | Me | Cl | - SO₂-n-Pr | |
| 6-26 | OMe | Et | Cl | - SO₂-n-Pr | |
| 6-27 | NO₂ | Me | Cl | - SO₂-n-Pr | |
| 6-28 | NO₂ | Et | Cl | - SO₂-n-Pr | |
| 6-29 | SO₂Me | Me | Cl | - SO₂-n-Pr | |
| 6-30 | SO₂Me | Et | Cl | - SO₂-n-Pr | |
| 6-31 | CH₂OMe | Me | Cl | - SO₂-n-Pr | |
| 6-32 | CH₂OMe | Et | Cl | - SO₂-n-Pr | |
| 6-33 | CH₂SO₂Me | Me | Cl | - SO₂-n-Pr | |
| 6-34 | CH₂SO₂Me | Et | Cl | - SO₂-n-Pr | |
| 6-35 | CH₂OCH₂CH₂OMe | Me | Cl | - SO₂-n-Pr | |
| 6-36 | CH₂OCH₂CH₂OMe | Et | Cl | - SO₂-n-Pr | |
| 6-37 | Cl | Me | Br | - SO₂-n-Pr | |
| 6-38 | Cl | Et | Br | - SO₂-n-Pr | |
| 6-39 | Me | Me | Br | - SO₂-n-Pr | |
| 6-40 | Me | Et | Br | - SO₂-n-Pr | |
| 6-41 | CF₃ | Me | Br | - SO₂-n-Pr | |
| 6-42 | CF₃ | Et | Br | - SO₂-n-Pr | |
| 6-43 | OMe | Me | Br | - SO₂-n-Pr | |
| 6-44 | OMe | Et | Br | - SO₂-n-Pr | |
| 6-45 | NO₂ | Me | Br | - SO₂-n-Pr | |
| 6-46 | NO₂ | Et | Br | - SO₂-n-Pr | |
| 6-47 | SO₂Me | Me | Br | - SO₂-n-Pr | |
| 6-48 | SO₂Me | Et | Br | - SO₂-n-Pr | |
| 6-49 | CH₂OMe | Me | Br | - SO₂-n-Pr | |
| 6-50 | CH₂OMe | Et | Br | - SO₂-n-Pr | |
| 6-51 | CH₂SO₂Me | Me | Br | - SO₂-n-Pr | |
| 6-52 | CH₂SO₂Me | Et | Br | - SO₂-n-Pr | |
| 6-53 | CH₂OCH₂CH₂OMe | Me | Br | - SO₂-n-Pr | |
| 6-54 | CH₂OCH₂CH₂OMe | Et | Br | - SO₂-n-Pr | |
| 6-55 | Cl | Me | I | - SO₂-n-Pr | |
| 6-56 | Cl | Et | I | - SO₂-n-Pr | |
| 6-57 | Me | Me | I | - SO₂-n-Pr | |
| 6-58 | Me | Et | I | - SO₂-n-Pr | |
| 6-59 | CF₃ | Me | I | - SO₂-n-Pr | |
| 6-60 | CF₃ | Et | I | - SO₂-n-Pr | |
| 6-61 | OMe | Me | I | - SO₂-n-Pr | |
| 6-62 | OMe | Et | I | - SO₂-n-Pr | |
| 6-63 | NO₂ | Me | I | - SO₂-n-Pr | |
| 6-64 | NO₂ | Et | I | - SO₂-n-Pr | |
| 6-65 | SO₂Me | Me | I | - SO₂-n-Pr | |
| 6-66 | SO₂Me | Et | I | - SO₂-n-Pr | |
| 6-67 | CH₂OMe | Me | I | - SO₂-n-Pr | |
| 6-68 | CH₂OMe | Et | I | - SO₂-n-Pr | |
| 6-69 | CH₂SO₂Me | Me | I | - SO₂-n-Pr | |
| 6-70 | CH₂SO₂Me | Et | I | - SO₂-n-Pr | |
| 6-71 | CH₂OCH₂CH₂OMe | Me | I | - SO₂-n-Pr | |
| 6-72 | CH₂OCH₂CH₂OMe | Et | I | - SO₂-n-Pr | |
| 6-73 | Cl | Me | NO₂ | - SO₂-n-Pr | |
| 6-74 | Cl | Et | NO₂ | - SO₂-n-Pr | |
| 6-75 | Me | Me | NO₂ | - SO₂-n-Pr | |
| 6-76 | Me | Et | NO₂ | - SO₂-n-Pr | |
| 6-77 | CF₃ | Me | NO₂ | - SO₂-n-Pr | |
| 6-78 | CF₃ | Et | NO₂ | - SO₂-n-Pr | |
| 6-79 | OMe | Me | NO₂ | - SO₂-n-Pr | |
| 6-80 | OMe | Et | NO₂ | - SO₂-n-Pr | |
| 6-81 | NO₂ | Me | NO₂ | - SO₂-n-Pr | |
| 6-82 | NO₂ | Et | NO₂ | - SO₂-n-Pr | |
| 6-83 | SO₂Me | Me | NO₂ | - SO₂-n-Pr | |
| 6-84 | SO₂Me | Et | NO₂ | - SO₂-n-Pr | |
| 6-85 | CH₂OMe | Me | NO₂ | - SO₂-n-Pr | |
| 6-86 | CH₂OMe | Et | NO₂ | - SO₂-n-Pr | |
| 6-87 | CH₂SO₂Me | Me | NO₂ | - SO₂-n-Pr | |
| 6-88 | CH₂SO₂Me | Et | NO₂ | - SO₂-n-Pr | |
| 6-89 | CH₂OCH₂CH₂OMe | Me | NO₂ | - SO₂-n-Pr | |
| 6-90 | CH₂OCH₂CH₂OMe | Et | NO₂ | - SO₂-n-Pr | |
| 6-91 | Cl | Me | SO₂Me | - CO-Ph | |
| 6-92 | Cl | Et | SO₂Me | - CO-Ph | |
| 6-93 | Me | Me | SO₂Me | - CO-Ph | |
| 6-94 | Me | Et | SO₂Me | - CO-Ph | |
| 6-95 | CF₃ | Me | SO₂Me | - CO-Ph | |
| 6-96 | CF₃ | Et | SO₂Me | - CO-Ph | |
| 6-97 | OMe | Me | SO₂Me | - CO-Ph | |
| 6-98 | OMe | Et | SO₂Me | - CO-Ph | |
| 6-99 | NO₂ | Me | SO₂Me | - CO-Ph | |
| 6-100 | NO₂ | Et | SO₂Me | - CO-Ph | |
| 6-101 | SO₂Me | Me | SO₂Me | - CO-Ph | |
| 6-102 | SO₂Me | Et | SO₂Me | - CO-Ph | |
| 6-103 | CH₂OMe | Me | SO₂Me | - CO-Ph | |
| 6-104 | CH₂OMe | Et | SO₂Me | - CO-Ph | |
| 6-105 | CH₂SO₂Me | Me | SO₂Me | - CO-Ph | |
| 6-106 | CH₂SO₂Me | Et | SO₂Me | - CO-Ph | |
| 6-107 | CH₂OCH₂CH₂OMe | Me | SO₂Me | - CO-Ph | |
| 6-108 | CH₂OCH₂CH₂OMe | Et | SO₂Me | - CO-Ph | |
| 6-109 | Cl | Me | Cl | - CO-Ph | |
| 6-110 | Cl | Et | Cl | - CO-Ph | |
| 6-111 | Me | Me | Cl | - CO-Ph | |
| 6-112 | Me | Et | Cl | - CO-Ph | |
| 6-113 | CF₃ | Me | Cl | - CO-Ph | |
| 6-114 | CF₃ | Et | Cl | - CO-Ph | |
| 6-115 | OMe | Me | Cl | - CO-Ph | |
| 6-116 | OMe | Et | Cl | - CO-Ph | |
| 6-117 | NO₂ | Me | Cl | - CO-Ph | |
| 6-118 | NO₂ | Et | Cl | - CO-Ph | |
| 6-119 | SO₂Me | Me | Cl | - CO-Ph | |
| *6-120 | SO₂Me | Et | Cl | - CO-Ph | |
| 6-121 | CH₂OMe | Me | Cl | - CO-Ph | |
| 6-122 | CH₂OMe | Et | Cl | - CO-Ph | |
| 6-123 | CH₂SO₂Me | Me | Cl | - CO-Ph | |
| 6-124 | CH₂SO₂Me | Et | Cl | - CO-Ph | |
| 6-125 | CH₂OCH₂CH₂OMe | Me | Cl | - CO-Ph | |
| 6-126 | CH₂OCH₂CH₂OMe | Et | Cl | - CO-Ph | |
| 6-127 | Cl | Me | Br | - CO-Ph | |
| 6-128 | Cl | Et | Br | - CO-Ph | |
| 6-129 | Me | Me | Br | - CO-Ph | |
| 6-130 | Me | Et | Br | - CO-Ph | |
| 6-131 | CF₃ | Me | Br | - CO-Ph | |
| 6-132 | CF₃ | Et | Br | - CO-Ph | |
| 6-133 | OMe | Me | Br | - CO-Ph | |
| 6-134 | OMe | Et | Br | - CO-Ph | |
| 6-135 | NO₂ | Me | Br | - CO-Ph | |
| 6-136 | NO₂ | Et | Br | - CO-Ph | |
| 6-137 | SO₂Me | Me | Br | - CO-Ph | |
| 6-138 | SO₂Me | Et | Br | - CO-Ph | |
| 6-139 | CH₂OMe | Me | Br | - CO-Ph | |
| 6-140 | CH₂OMe | Et | Br | - CO-Ph | |
| 6-141 | CH₂SO₂Me | Me | Br | - CO-Ph | |
| 6-142 | CH₂SO₂Me | Et | Br | - CO-Ph | |
| 6-143 | CH₂OCH₂CH₂OMe | Me | Br | - CO-Ph | |
| 6-144 | CH₂OCH₂CH₂OMe | Et | Br | - CO-Ph | |
| 6-145 | Cl | Me | I | - CO-Ph | |
| 6-146 | Cl | Et | I | - CO-Ph | |
| 6-147 | Me | Me | I | - CO-Ph | |
| 6-148 | Me | Et | I | - CO-Ph | |
| 6-149 | CF₃ | Me | I | - CO-Ph | |
| 6-150 | CF₃ | Et | I | - CO-Ph | |
| 6-151 | OMe | Me | I | - CO-Ph | |
| 6-152 | OMe | Et | I | - CO-Ph | |
| 6-153 | NO₂ | Me | I | - CO-Ph | |
| 6-154 | NO₂ | Et | I | - CO-Ph | |
| 6-155 | SO₂Me | Me | I | - CO-Ph | |
| 6-156 | SO₂Me | Et | I | - CO-Ph | |
| 6-157 | CH₂OMe | Me | I | - CO-Ph | |
| 6-158 | CH₂OMe | Et | I | - CO-Ph | |
| 6-159 | CH₂SO₂Me | Me | I | - CO-Ph | |
| 6-160 | CH₂SO₂Me | Et | I | - CO-Ph | |
| 6-161 | CH₂OCH₂CH₂OMe | Me | I | - CO-Ph | |
| 6-162 | CH₂OCH₂CH₂OMe | Et | I | - CO-Ph | |
| 6-163 | Cl | Me | NO₂ | - CO-Ph | |
| 6-164 | Cl | Et | NO₂ | - CO-Ph | |
| 6-165 | Me | Me | NO₂ | - CO-Ph | |
| 6-166 | Me | Et | NO₂ | - CO-Ph | |
| 6-167 | CF₃ | Me | NO₂ | - CO-Ph | |
| 6-168 | CF₃ | Et | NO₂ | - CO-Ph | |
| 6-169 | OMe | Me | NO₂ | - CO-Ph | |
| 6-170 | OMe | Et | NO₂ | - CO-Ph | |
| 6-171 | NO₂ | Me | NO₂ | - CO-Ph | |
| 6-172 | NO₂ | Et | NO₂ | - CO-Ph | |
| 6-173 | SO₂Me | Me | NO₂ | - CO-Ph | |
| 6-174 | SO₂Me | Et | NO₂ | - CO-Ph | |
| 6-175 | CH₂OMe | Me | NO₂ | - CO-Ph | |
| 6-176 | CH₂OMe | Et | NO₂ | - CO-Ph | |
| 6-177 | CH₂SO₂Me | Me | NO₂ | - CO-Ph | |
| 6-178 | CH₂SO₂Me | Et | NO₂ | - CO-Ph | |
| 6-179 | CH₂OCH₂CH₂OMe | Me | NO₂ | - CO-Ph | |
| 6-180 | CH₂OCH₂CH₂OMe | Et | NO₂ | - CO-Ph | |
| 6-181 | Cl | Me | SO₂Me | - CH₂-CO-(4-Me-Ph) | |
| 6-182 | Cl | Et | SO₂Me | - CH₂-CO-(4-Me-Ph) | |
| 6-183 | Me | Me | SO₂Me | - CH₂-CO-(4-Me-Ph) | |
| 6-184 | Me | Et | SO₂Me | - CH₂-CO-(4-Me-Ph) | |
| 6-185 | CF₃ | Me | SO₂Me | - CH₂-CO-(4-Me-Ph) | |
| 6-186 | CF₃ | Et | SO₂Me | - CH₂-CO-(4-Me-Ph) | |
| 6-187 | OMe | Me | SO₂Me | - CH₂-CO-(4-Me-Ph) | |
| 6-188 | OMe | Et | SO₂Me | - CH₂-CO-(4-Me-Ph) | |
| 6-189 | NO₂ | Me | SO₂Me | - CH₂-CO-(4-Me-Ph) | |
| 6-190 | NO₂ | Et | SO₂Me | - CH₂-CO-(4-Me-Ph) | |
| 6-191 | SO₂Me | Me | SO₂Me | - CH₂-CO-(4-Me-Ph) | |
| 6-192 | SO₂Me | Et | SO₂Me | - CH₂-CO-(4-Me-Ph) | |
| 6-193 | CH₂OMe | Me | SO₂Me | - CH₂-CO-(4-Me-Ph) | |
| 6-194 | CH₂OMe | Et | SO₂Me | - CH₂-CO-(4-Me-Ph) | |
| 6-195 | CH₂SO₂Me | Me | SO₂Me | - CH₂-CO-(4-Me-Ph) | |
| 6-196 | CH₂SO₂Me | Et | SO₂Me | - CH₂-CO-(4-Me-Ph) | |
| 6-197 | CH₂OCH₂CH₂OMe | Me | SO₂Me | - CH₂-CO-(4-Me-Ph) | |
| 6-198 | CH₂OCH₂CH₂OMe | Et | SO₂Me | - CH₂-CO-(4-Me-Ph) | |
| 6-199 | Cl | Me | Cl | - CH₂-CO-(4-Me-Ph) | |
| 6-200 | Cl | Et | Cl | - CH₂-CO-(4-Me-Ph) | |
| 6-201 | Me | Me | Cl | - CH₂-CO-(4-Me-Ph) | |
| 6-202 | Me | Et | Cl | - CH₂-CO-(4-Me-Ph) | |
| 6-203 | CF₃ | Me | Cl | - CH₂-CO-(4-Me-Ph) | |
| 6-204 | CF₃ | Et | Cl | - CH₂-CO-(4-Me-Ph) | |
| 6-205 | OMe | Me | Cl | - CH₂-CO-(4-Me-Ph) | |
| 6-206 | OMe | Et | Cl | - CH₂-CO-(4-Me-Ph) | |
| 6-207 | NO₂ | Me | Cl | - CH₂-CO-(4-Me-Ph) | |
| 6-208 | NO₂ | Et | Cl | - CH₂-CO-(4-Me-Ph) | |
| 6-209 | SO₂Me | Me | Cl | - CH₂-CO-(4-Me-Ph) | |
| 6-210 | SO₂Me | Et | Cl | - CH₂-CO-(4-Me-Ph) | |
| 6-211 | CH₂OMe | Me | Cl | - CH2-CO-(4-Me-Ph) | |
| 6-212 | CH₂OMe | Et | Cl | - CH₂-CO-(4-Me-Ph) | |
| 6-213 | CH₂SO₂Me | Me | Cl | - CH₂-CO-(4-Me-Ph) | |
| 6-214 | CH₂SO₂Me | Et | Cl | - CH₂-CO-(4-Me-Ph) | |
| 6-215 | CH₂OCH₂CH₂oMe | Me | Cl | - CH₂-CO-(4-Me-Ph) | |
| 6-216 | CH₂OCH₂CH₂OMe | Et | Cl | - CH₂-co-(4-Me-Ph) | |
| 6-217 | Cl | Me | Br | - CH₂-CO-(4-Me-Ph) | |
| 6-218 | Cl | Et | Br | - CH₂-CO-(4-Me-Ph) | |
| 6-219 | Me | Me | Br | - CH₂-CO-(4-Me-Ph) | |
| 6-220 | Me | Et | Br | - CH₂-CO-(4-Me-Ph) | |
| 6-221 | CF₃ | Me | Br | - CH₂-CO-(4-Me-Ph) | |
| 6-222 | CF₃ | Et | Br | - CH₂-CO-(4-Me-Ph) | |
| 6-223 | OMe | Me | Br | - CH₂-CO-(4-Me-Ph) | |
| 6-224 | OMe | Et | Br | - CH₂-CO-(4-Me-Ph) | |
| 6-225 | NO₂ | Me | Br | - CH₂-CO-(4-Me-Ph) | |
| 6-226 | NO₂ | Et | Br | - CH₂-CO-(4-Me-Ph) | |
| 6-227 | SO₂Me | Me | Br | - CH₂-CO-(4-Me-Ph) | |
| 6-228 | SO₂Me | Et | Br | - CH₂-CO-(4-Me-Ph) | |
| 6-229 | CH₂OMe | Me | Br | - CH₂-CO-(4-Me-Ph) | |
| 6-230 | CH₂OMe | Et | Br | - CH₂-CO-(4-Me-Ph) | |
| 6-231 | CH₂SO₂Me | Me | Br | - CH₂-CO-(4-Me-Ph) | |
| 6-232 | CH₂SO₂Me | Et | Br | - CH₂-CO-(4-Me-Ph) | |
| 6-233 | CH₂OCH₂CH₂OMe | Me | Br | - CH₂-CO-(4-Me-Ph) | |
| 6-234 | CH₂OCH₂CH₂OMe | Et | Br | - CH₂-CO-(4-Me-Ph) | |
| 6-235 | Cl | Me | I | - CH₂-CO-(4-Me-Ph) | |
| 6-236 | Cl | Et | I | - CH₂-CO(4-Me-Ph) | |
| 6-237 | Me | Me | I | - CH₂-CO-(4-Me-Ph) | |
| 6-238 | Me | Et | I | - CH₂-CO-(4-Me-Ph) | |
| 6-239 | CF₃ | Me | I | - CH₂-CO-(4-Me-Ph) | |
| 6-240 | CF₃ | Et | I | - CH₂-CO-(4-Me-Ph) | |
| 6-241 | OMe | Me | I | - CH₂-CO-(4-Me-Ph) | |
| 6-242 | OMe | Et | I | - CH₂-CO-(4-Me-Ph) | |
| 6-243 | NO₂ | Me | I | - CH₂-CO-(4-Me-Ph) | |
| 6-244 | NO₂ | Et | I | - CH₂-CO-(4-Me-Ph) | |
| 6-245 | SO₂Me | Me | I | - CH₂-CO-(4-Me-Ph) | |
| 6-246 | SO₂Me | Et | I | - CH₂-CO-(4-Me-Ph) | |
| 6-247 | CH₂OMe | Me | I | - CH₂-CO-(4-Me-Ph) | |
| 6-248 | CH₂OMe | Et | I | - CH₂-CO-(4-Me-Ph) | |
| 6-249 | CH₂SO₂Me | Me | I | - CH₂-CO-(4-Me-Ph) | |
| 6-250 | CH₂SO₂Me | Et | I | - CH₂-CO-(4-Me-Ph) | |
| 6-251 | CH₂OCH₂CH₂OMe | Me | I | - CH₂-CO-(4-Me-Ph) | |
| 6-252 | CH₂OCH₂CH₂OMe | Et | I | - CH₂-CO-(4-Me-Ph) | |
| 6-253 | Cl | Me | NO₂ | - CH₂-CO-(4-Me-Ph) | |
| 6-254 | Cl | Et | NO₂ | - CH₂-CO-(4-Me-Ph) | |
| 6-255 | Me | Me | NO₂ | - CH₂-CO-(4-Me-Ph) | |
| 6-256 | Me | Et | NO₂ | - CH₂-CO-(4-Me-Ph) | |
| 6-257 | CF₃ | Me | NO₂ | - CH₂-CO-(4-Me-Ph) | |
| 6-258 | CF₃ | Et | NO₂ | -CH₂-CO-(4-Me-Ph) | |
| 6-259 | OMe | Me | NO₂ | - CH₂-CO-(4-Me-Ph) | |
| 6-260 | OMe | Et | NO₂ | -CH₂-CO-(4-Me-Ph) | |
| 6-261 | NO₂ | Me | NO₂ | - CH₂-CO-(4-Me-Ph) | |
| 6-262 | NO₂ | Et | NO₂ | - CH₂-CO-(4-Me-Ph) | |
| 6-263 | SO₂Me | Me | NO₂ | - CH₂-CO-(4-Me-Ph) | |
| 6-264 | SO₂Me | Et | NO₂ | - CH₂-CO-(4-Me-Ph) | |
| 6-265 | CH₂OMe | Me | NO₂ | - CH₂-CO-(4-Me-Ph) | |
| 6-266 | CH₂OMe | Et | NO₂ | - CH₂-CO-(4-Me-Ph) | |
| 6-267 | CH₂SO₂Me | Me | NO₂ | - CH₂-CO-(4-Me-Ph) | |
| 6-268 | CH₂SO₂Me | Et | NO₂ | - CH₂-CO-(4-Me-Ph) | |
| 6-269 | CH₂OCH₂CH₂OMe | Me | NO₂ | - CH₂-CO-(4-Me-Ph) | |
| 6-270 | CH₂OCH₂CH₂OMe | Et | NO₂ | - CH₂-CO-(4-Me-Ph) | |

**Tabelle 7: Erfindungsgemäße Verbindungen der allgemeinen Formel (I), worin R¹ und R² jeweils für Methyl stehen.**

| | | | | | |
|---|---|---|---|---|---|
| 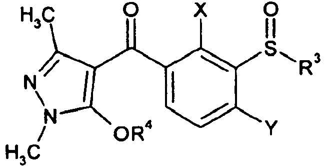 | | | | | |

| Nr. | X | R³ | Y | R⁴ | Physikalische Daten: ¹H-NMR: δ [CDCl₃] |
|---|---|---|---|---|---|
| 7-1 | Cl | Me | SO₂Me | - SO₂-n-Pr | |
| 7-2 | Cl | Et | SO₂Me | - SO₂-n-Pr | |
| 7-3 | Me | Me | SO₂Me | - SO₂-n-Pr | |
| 7-4 | Me | Et | SO₂Me | - SO₂-n-Pr | |
| 7-5 | CF₃ | Me | SO₂Me | - SO₂-n-Pr | |
| 7-6 | CF₃ | Et | SO₂Me | - SO₂-n-Pr | |
| 7-7 | OMe | Me | SO₂Me | - SO₂-n-Pr | |
| 7-8 | OMe | Et | SO₂Me | - SO₂-n-Pr | |
| 7-9 | NO₂ | Me | SO₂Me | - SO₂-n-Pr | |
| 7-10 | NO₂ | Et | SO₂Me | - SO₂-n-Pr | |
| 7-11 | SO₂Me | Me | SO₂Me | - SO₂-n-Pr | |
| 7-12 | SO₂Me | Et | SO₂Me | - SO₂-n-Pr | |
| 7-13 | CH₂OMe | Me | SO₂Me | - SO₂-n-Pr | |
| 7-14 | CH₂OMe | Et | SO₂Me | - SO₂-n-Pr | |
| 7-15 | CH₂SO₂Me | Me | SO₂Me | - SO₂-n-Pr | |
| 7-16 | CH₂SO₂Me | Et | SO₂Me | - SO₂-n-Pr | |
| 7-17 | CH₂OCH₂CH₂OMe | Me | SO₂Me | - SO₂-n-Pr | |
| 7-18 | CH₂OCH₂CH₂OMe | Et | SO₂Me | - SO₂-n-Pr | |
| 7-19 | Cl | Me | Cl | - SO₂-n-Pr | |
| 7-20 | Cl | Et | Cl | - SO₂-n-Pr | |
| 7-21 | Me | Me | Cl | - SO₂-n-Pr | |
| 7-22 | Me | Et | Cl | - SO₂-n-Pr | |
| 7-23 | CF₃ | Me | Cl | - SO₂-n-Pr | |
| 7-24 | CF₃ | Et | Cl | - SO₂-n-Pr | |
| 7-25 | OMe | Me | Cl | - SO₂-n-Pr | |
| 7-26 | OMe | Et | Cl | - SO₂-n-Pr | |
| 7-27 | NO₂ | Me | Cl | - SO₂-n-Pr | |
| 7-28 | NO₂ | Et | Cl | - SO₂-n-Pr | |
| 7-29 | SO₂Me | Me | Cl | - SO₂-n-Pr | |
| 7-30 | SO₂Me | Et | Cl | - SO₂-n-Pr | |
| 7-31 | CH₂OMe | Me | Cl | - SO₂-n-Pr | |
| 7-32 | CH₂OMe | Et | Cl | - SO₂-n-Pr | |
| 7-33 | CH₂SO₂Me | Me | Cl | - SO₂-n-Pr | |
| 7-34 | CH₂SO₂Me | Et | Cl | - SO₂-n-Pr | |
| 7-35 | CH₂OCH₂CH₂OMe | Me | Cl | - SO₂-n-Pr | |
| 7-36 | CH₂OCH₂CH₂OMe | Et | Cl | - SO₂-n-Pr | |
| 7-37 | Cl | Me | Br | - SO₂-n-Pr | |
| 7-38 | Cl | Et | Br | - SO₂-n-Pr | |
| 7-39 | Me | Me | Br | - SO₂-n-Pr | |
| 7-40 | Me | Et | Br | - SO₂-n-Pr | |
| 7-41 | CF₃ | Me | Br | - SO₂-n-Pr | |
| 7-42 | CF₃ | Et | Br | - SO₂-n-Pr | |
| 7-43 | OMe | Me | Br | - SO₂-n-Pr | |
| 7-44 | OMe | Et | Br | - SO₂n-Pr | |
| 7-45 | NO₂ | Me | Br | - SO₂-n-Pr | |
| 7-46 | NO₂ | Et | Br | - SO₂-n-Pr | |
| 7-47 | SO₂Me | Me | Br | - SO₂-n-Pr | |
| 7-48 | SO₂Me | Et | Br | - SO₂-n-Pr | |
| 7-49 | CH₂OMe | Me | Br | - SO₂-n-Pr | |
| 7-50 | CH₂OMe | Et | Br | - SO₂-n-Pr | |
| 7-51 | CH₂SO₂Me | Me | Br | - SO₂-n-Pr | |
| 7-52 | CH₂SO₂Me | Et | Br | - SO₂-n-Pr | |
| 7-53 | CH₂OCH₂CH₂OMe | Me | Br | - SO₂-n-Pr | |
| 7-54 | CH₂OCH₂CH₂OMe | Et | Br | - SO₂-n-Pr | |
| 7-55 | Cl | Me | I | - SO₂-n-Pr | |
| 7-56 | Cl | Et | I | - SO₂-n-Pr | |
| 7-57 | Me | Me | I | - SO₂-n-Pr | |
| 7-58 | Me | Et | I | - SO₂-n-Pr | |
| 7-59 | CF₃ | Me | I | - SO₂-n-Pr | |
| 7-60 | CF₃ | Et | I | - SO₂-n-Pr | |
| 7-61 | OMe | Me | I | - SO₂-n-Pr | |
| 7-62 | OMe | Et | I | - SO₂-n-Pr | |
| 7-63 | NO₂ | Me | I | - SO₂-n-Pr | |
| 7-64 | NO₂ | Et | I | - SO₂-n-Pr | |
| 7-65 | SO₂Me | Me | I | - SO₂-n-Pr | |
| 7-66 | SO₂Me | Et | I | - SO₂-n-Pr | |
| 7-67 | CH₂OMe | Me | I | - SO₂-n-Pr | |
| 7-68 | CH₂OMe | Et | I | - SO₂-n-Pr | |
| 7-69 | CH₂SO₂Me | Me | I | - SO₂-n-Pr | |
| 7-70 | CH₂SO₂Me | Et | I | - SO₂-n-Pr | |
| 7-71 | CH₂OCH₂CH₂OMe | Me | I | - SO₂-n-Pr | |
| 7-72 | CH₂OCH₂CH₂OMe | Et | I | - SO₂-n-Pr | |
| 7-73 | Cl | Me | NO₂ | - SO₂-n-Pr | |
| 7-74 | Cl | Et | NO₂ | - SO₂-n-Pr | |
| 7-75 | Me | Me | NO₂ | - SO₂-n-Pr | |
| 7-76 | Me | Et | NO₂ | - SO₂-n-Pr | |
| 7-77 | CF₃ | Me | NO₂ | - SO₂-n-Pr | |
| 7-78 | CF₃ | Et | NO₂ | - SO₂-n-Pr | |
| 7-79 | OMe | Me | NO₂ | - SO₂-n-Pr | |
| 7-80 | OMe | Et | NO₂ | - SO₂-n-Pr | |
| 7-81 | NO₂ | Me | NO₂ | - SO₂-n-Pr | |
| 7-62 | NO₂ | Et | NO₂ | - SO₂-n-Pr | |
| 7-83 | SO₂Me | Me | NO₂ | - SO₂-n-Pr | |
| 7-84 | SO₂Me | Et | NO₂ | - SO₂-n-Pr | |
| 7-85 | CH₂OMe | Me | NO₂ | - SO₂-n-Pr | |
| 7-86 | CH₂OMe | Et | NO₂ | - SO₂-n-Pr | |
| 7-87 | CH₂SO₂Me | Me | NO₂ | - SO₂-n-Pr | |
| 7-88 | CH₂SO₂Me | Et | NO₂ | - SO₂-n-Pr | |
| 7-89 | CH₂OCH₂CH₂OMe | Me | NO₂ | - SO₂-n-Pr | |
| 7-90 | CH₂OCH₂CH₂OMe | Et | NO₂ | - SO₂-n-Pr | |
| 7-91 | Cl | Me | SO₂Me | - CO-Ph | |
| 7-92 | Cl | Et | SO₂Me | - CO-Ph | |
| 7-93 | Me | Me | SO₂Me | - CO-Ph | |
| 7-94 | Me | Et | SO₂Me | - CO-Ph | |
| 7-95 | CF₃ | Me | SO₂Me | - CO-Ph | |
| 7-96 | CF₃ | Et | SO₂Me | - CO-Ph | |
| 7-97 | OMe | Me | SO₂Me | - CO-Ph | |
| 7-98 | OMe | Et | SO₂Me | - CO-Ph | |
| 7-99 | NO₂ | Me | SO₂Me | - CO-Ph | |
| 7-100 | NO₂ | Et | SO₂Me | - CO-Ph | |
| 7-101 | SO₂Me | Me | SO₂Me | - CO-Ph | |
| 7-102 | SO₂Me | Et | SO₂Me | - CO-Ph | |
| 7-103 | CH₂OMe | Me | SO₂Me | - CO-Ph | |
| 7-104 | CH₂OMe | Et | SO₂Me | - CO-Ph | |
| 7-105 | CH₂SO₂Me | Me | SO₂Me | - CO-Ph | |
| 7-106 | CH₂SO₂Me | Et | SO₂Me | - CO-Ph | |
| 7-107 | CH₂OCH₂CH₂OMe | Me | SO₂Me | - CO-Ph | |
| 7-108 | CH₂OCH₂CH₂OMe | Et | SO₂Me | - CO-PH | |
| 7-109 | Cl | Me | Cl | - CO-Ph | |
| 7-110 | Cl | Et | Cl | - CO-Ph | |
| 7-111 | Me | Me | Cl | - CO-Ph | |
| 7-112 | Me | Et | Cl | - CO-Ph | |
| 7-113 | CF₃ | Me | Cl | - CO-Ph | |
| 7-114 | CF₃ | Et | Cl | - CO-Ph | |
| 7-115 | OMe | Me | Cl | - CO-Ph | |
| 7-116 | OMe | Et | Cl | - CO-Ph | |
| 7-117 | NO₂ | Me | Cl | - CO-Ph | |
| 7-118 | NO₂ | Et | Cl | - CO-Ph | |
| 7-119 | SO₂Me | Me | Cl | - CO-Ph | |
| 7-120 | SO₂Me | Et | Cl | - CO-Ph | |
| 7-121 | CH₂OMe | Me | Cl | - CO-Ph | |
| 7-122 | CH₂OMe | Et | Cl | - CO-Ph | |
| 7-123 | CH₂SO₂Me | Me | Cl | - CO-Ph | |
| 7-124 | CH₂SO₂Me | Et | Cl | - CO-Ph | |
| 7-125 | CH₂OCH₂CH₂OMe | Me | Cl | - CO-Ph | |
| 7-126 | CH₂OCH₂CH₂OMe | Et | Cl | - CO-Ph | |
| 7-127 | Cl | Me | Br | - CO-Ph | |
| 7-128 | Cl | Et | Br | - CO-Ph | |
| 7-129 | Me | Me | Br | - CO-Ph | |
| 7-130 | Me | Et | Br | - CO-Ph | |
| 7-131 | CF₃ | Me | Br | - CO-Ph | |
| 7-132 | CF₃ | Et | Br | - CO-Ph | |
| 7-133 | OMe | Me | Br | - CO-Ph | |
| 7-134 | OMe | Et | Br | - CO-Ph | |
| 7-135 | NO₂ | Me | Br | - CO-Ph | |
| 7-136 | NO₂ | Et | Br | - CO-Ph | |
| 7-137 | SO₂Me | Me | Br | - CO-Ph | |
| 7-138 | SO₂Me | Et | Br | - CO-Ph | |
| 7-139 | CH₂OMe | Me | Br | - CO-Ph | |
| 7-140 | CH₂OMe | Et | Br | - CO-Ph | |
| 7-141 | CH₂SO₂Me | Me | Br | - CO-Ph | |
| 7-142 | CH₂SO₂Me | Et | Br | - CO-Ph | |
| 7-143 | CH₂OCH₂CH₂OMe | Me | Br | - CO-Ph | |
| 7-144 | CH₂OCH₂CH₂OMe | Et | Br | - CO-Ph | |
| 7-145 | Cl | Me | I | - CO-Ph | |
| 7-146 | Cl | Et | I | - CO-Ph | |
| 7-147 | Me | Me | I | - CO-Ph | |
| 7-148 | Me | Et | I | - CO-Ph | |
| 7-149 | CF₃ | Me | I | - CO-Ph | |
| 7-150 | CF₃ | Et | I | - CO-Ph | |
| 7-151 | OMe | Me | I | - CO-Ph | |
| 7-152 | OMe | Et | I | - CO-Ph | |
| 7-153 | NO₂ | Me | I | - CO-Ph | |
| 7-154 | NO₂ | Et | I | - CO-Ph | |
| 7-155 | SO₂Me | Me | I | - CO-Ph | |
| 7-156 | SO₂Me | Et | I | - CO-Ph | |
| 7-157 | CH₂OMe | Me | I | - CO-Ph | |
| 7-158 | CH₂OMe | Et | I | - CO-Ph | |
| 7-159 | CH₂SO₂Me | Me | I | - CO-Ph | |
| 7-160 | CH₂SO₂Me | Et | I | - CO-Ph | |
| 7-161 | CH₂OCH₂CH₂OMe | Me | I | - CO-Ph | |
| 7-162 | CH₂OCH₂CH₂OMe | Et | I | - CO-Ph | |
| 7-163 | Cl | Me | NO₂ | - CO-Ph | |
| 7-164 | Cl | Et | NO₂ | - CO-Ph | |
| 7-165 | Me | Me | NO₂ | - CO-Ph | |
| 7-166 | Me | Et | NO₂ | - CO-Ph | |
| 7-167 | CF₃ | Me | NO₂ | - CO-PH | |
| 7-168 | CF₃ | Et | NO₂ | - CO-Ph | |
| 7-169 | OMe | Me | NO₂ | - CO-Ph | |
| 7-170 | OMe | Et | NO₂ | - CO-Ph | |
| 7-171 | NO₂ | Me | NO₂ | - CO-Ph | |
| 7-172 | NO₂ | Et | NO₂ | - CO-Ph | |
| 7-173 | SO₂Me | Me | NO₂ | - CO-Ph | |
| 7-174 | SO₂Me | Et | NO₂ | - CO-Ph | |
| 7-175 | CH₂OMe | Me | NO₂ | - CO-Ph | |
| 7-176 | CH₂OMe | Et | NO₂ | - CO-Ph | |
| 7-177 | CH₂SO₂Me | Me | NO₂ | - CO-Ph | |
| 7-178 | CH₂SO₂Me | Et | NO₂ | - CO-Ph | |
| 7-179 | CH₂OCH₂CH₂OMe | Me | NO₂ | - CO-Ph | |
| 7-180 | CH₂OCH₂CH₂OMe | Et | NO₂ | - CO-Ph | |
| 7-181 | Cl | Me | SO₂Me | - CH₂-CO-(4-Me-Ph) | |
| 7-182 | Cl | Et | SO₂Me | - CH₂-CO-(4-Me-Ph) | |
| 7-183 | Me | Me | SO₂Me | - CH₂-CO-(4-Me-Ph) | |
| 7-184 | Me | Et | SO₂Me | - CH₂-CO-(4-Me-Ph) | |
| 7-185 | CF₃ | Me | SO₂Me | - CH₂-CO-(4-Me-Ph) | |
| 7-186 | CF₃ | Et | SO₂Me | - CH₂-CO-(4-Me-Ph) | |
| 7-187 | OMe | Me | SO₂Me | - CH₂-CO (4-Me-Ph) | |
| 7-188 | OMe | Et | SO₂Me | - CH₂-CO-(4-Me-Ph) | |
| 7-189 | NO₂ | Me | SO₂Me | - CH₂-CO-(4-Me-Ph) | |
| 7-190 | NO₂ | Et | SO₂Me | - CH₂-CO-(4-Me-Ph) | |
| 7-191 | SO₂Me | Me | SO₂Me | - CH₂-CO-(4-Me-Ph) | |
| 7-192 | SO₂Me | Et | SO₂Me | - CH₂-CO-(4-Me-Ph) | |
| 7-193 | CH₂OMe | Me | SO₂Me | - CH₂-CO-(4-Me-Ph) | |
| 7-194 | CH₂OMe | Et | SO₂Me | - CH₂-CO-(4-Me-Ph) | |
| 7-195 | CH₂SO₂Me | Me | SO₂Me | - CH₂-CO-(4-Me-Ph) | |
| 7-196 | CH₂SO₂Me | Et | SO₂Me | - CH₂-CO-(4-Me-Ph) | |
| 7-197 | CH₂OCH₂CH₂OMe | Me | SO₂Me | - CH₂-CO-(4-Me-Ph) | |
| 7-198 | CH₂OCH₂CH₂OMe | Et | SO₂Me | - CH₂-CO-(4-Me-Ph) | |
| 7-199 | Cl | Me | Cl | - CH₂-CO-(4-Me-Ph) | |
| 7-200 | Cl | Et | Cl | - CH₂-CO-(4-Me-Ph) | |
| 7-201 | Me | Me | Cl | - CH₂-CO-(4-Me-Ph) | |
| 7-202 | Me | Et | Cl | - CH₂-CO-(4-Me-Ph) | |
| 7-203 | CF₃ | Me | Cl | - CH₂-CO-(4-Me-Ph) | |
| 7-204 | CF₃ | Et | Cl | - CH₂-CO-(4-Me-Ph) | |
| 7-205 | OMe | Me | Cl | - CH₂-CO-(4-Me-Ph) | |
| 7-206 | OMe | Et | Cl | - CH₂-CO-(4-Me-Ph) | |
| 7-207 | NO₂ | Me | Cl | - CH₂-CO-(4-Me-Ph) | |
| 7-208 | NO₂ | Et | Cl | - CH₂-CO-(4-Me-Ph) | |
| 7-209 | SO₂Me | Me | Cl | - CH₂-CO-(4-Me-Ph) | |
| 7-210 | SO₂Me | Et | Cl | - CH₂-CO-(4-Me-Ph) | |
| 7-211 | CH₂OMe | Me | Cl | - CH₂-CO-(4-Me-Ph) | |
| 7-212 | CH₂OMe | Et | Cl | - CH₂-CO-(4-Me-Ph) | |
| 7-213 | CH₂SO₂Me | Me | Cl | - CH₂-CO-(4-Me-Ph) | |
| 7-214 | CH₂SO₂Me | Et | Cl | - CH₂-CO-(4-Me-Ph) | |
| 7-215 | CH₂OCH₂CH₂OMe | Me | Cl | - CH₂-CO-(4-Me-Ph) | |
| 7-216 | CH₂OCH₂CH₂OMe | Et | Cl | - CH₂-CO-(4-Me-Ph) | -CH₂-CO- |
| 7-217 | Cl | Me | Br | - CH₂-CO-(4-Me-Ph) | |
| 7-218 | Cl | Et | Br | -CH₂-CO-(4-Me-Ph) | |
| 7-219 | Me | Me | Br | - CH₂-CO-(4-Me-Ph) | |
| 7-220 | Me | Et | Br | - CH₂-CO-(4-Me-Ph) | |
| 7-221 | CF₃ | Me | Br | - CH₂-CO-(4-Me-Ph) | |
| 7-222 | CF₃ | Et | Br | - CH₂-CO-(4-Me-Ph) | |
| 7-223 | OMe | Me | Br | -CH₂-CO-(4-Me-Ph) | |
| 7-224 | OMe | Et | Br | -CH₂-CO-(4-Me-Ph) | |
| 7-225 | NO₂ | Me | Br | - CH₂-CO-(4-Me-Ph) | |
| 7-226 | NO₂ | Et | Br | - CH₂-CO-(4-Me-Ph) | |
| 7-227 | SO₂Me | Me | Br | - CH₂-CO-(4-Me-Ph) | |
| 7-228 | SO₂Me | Et | Br | - CH₂-CO-(4-Me-Ph) | |
| 7-229 | CH₂OMe | Me | Br | - CH₂-CO-(4-Me-Ph) | |
| 7-230 | CH₂OMe | Et | Br | - CH₂-CO-(4-Me-Ph) | |
| 7-231 | CH₂SO₂Me | Me | Br | - CH₂-CO-(4-Me-Ph) | |
| 7-232 | CH₂SO₂Me | Et | Br | - CH₂-CO-(4-Me-Ph) | |
| 7-233 | CH₂OCH₂CH₂OMe | Me | Br | - CH₂-CO-(4-Me-Ph) | |
| 7-234 | CH₂OCH₂CH₂OMe | Et | Br | - CH₂-CO-(4-Me-Ph) | |
| 7-235 | Cl | Me | I | - CH₂-CO-(4-Me-Ph) | |
| 7-236 | Cl | Et | I | - CH₂-CO-(4-Me-Ph) | |
| 7-237 | Me | Me | I | - CH₂-CO-(4-Me-Ph) | |
| 7-238 | Me | Et | I | - CH₂-CO-(4-Me-Ph) | |
| 7-239 | CF₃ | Me | I | - CH₂-CO-(4-Me-Ph) | |
| 7-240 | CF₃ | Et | I | - CH₂-CO-(4-Me-Ph) | |
| 7-241 | OMe | Me | I | - CH₂-CO-(4-Me-Ph) | |
| 7-242 | OMe | Et | I | - CH₂-CO-(4-Me-Ph) | |
| 7-243 | NO₂ | Me | I | - CH₂-CO-(4-Me-Ph) | |
| 7-244 | NO₂ | Et | I | - CH₂-CO-(4-Me-Ph) | |
| 7-245 | SO₂Me | Me | I | - CH₂-CO-(4-Me-Ph) | |
| 7-246 | SO₂Me | Et | I | - CH₂-CO-(4-Me-Ph) | |
| 7-247 | CH₂OMe | Me | I | - CH₂-CO-(4-Me-Ph) | |
| 7-248 | CH₂OMe | Et | I | - CH₂-CO-(4-Me-Ph) | |
| 7-249 | CH₂SO₂Me | Me | I | - CH₂-CO-(4-Me-Ph) | |
| 7-250 | CH₂SO₂Me | Et | I | - CH₂-CO-(4-Me-Ph) | |
| 7-251 | CH₂OCH₂CH₂oMe | Me | I | - CH₂-CO-(4-Me-Ph) | |
| 7-252 | CH₂OCH₂CH₂OMe | Et | I | - CH₂-CO-(4-Me-Ph) | |
| 7-253 | Cl | Me | NO₂ | - CH₂-CO-(4-Me-Ph) | |
| 7-254 | Cl | Et | NO₂ | - CH₂-CO-(4-Me-Ph) | |
| 7-255 | Me | Me | NO₂ | -CH₂-CO-(4-Me-Ph) | |
| 7-256 | Me | Et | NO₂ | - CH₂-CO-(4-Me-Ph) | |
| 7-257 | CF₃ | Me | NO₂ | - CH₂-CO-(4-Me-Ph) | |
| 7-258 | CF₃ | Et | NO₂ | - CH₂-CO-(4-Me-Ph) | |
| 7-259 | OMe | Me | NO₂ | -CH₂-CO-(4-Me-Ph) | |
| 7-260 | OMe | Et | NO₂ | - CH₂-CO-(4-Me-Ph) | |
| 7-261 | NO₂ | Me | NO₂ | - CH₂-CO-(4-Me-Ph) | |
| 7-262 | NO₂ | Et | NO₂ | - CH₂-CO-(4-Me-Ph) | |
| 7-263 | SO₂Me | Me | NO₂ | - CH₂-CO-(4-Me-Ph) | |
| 7-264 | SO₂Me | Et | NO₂ | - CH₂-CO-(4-Me-Ph) | |
| 7-265 | CH₂OMe | Me | NO₂ | - CH₂-CO-(4-Me-Ph) | |
| 7-266 | CH₂OMe | Et | NO₂ | - CH₂-CO-(4-Me-Ph) | |
| 7-267 | CH₂SO₂Me | Me | NO₂ | - CH₂-CO-(4-Me-Ph) | |
| 7-268 | CH₂SO₂Me | Et | NO₂ | - CH₂-CO-(4-Me-Ph) | |
| 7-269 | CH₂OCH₂CH₂OMe | Me | NO₂ | - CH₂-CO-(4-Me-Ph) | |
| 7-270 | CH₂OCH₂CH₂OMe | Et | NO₂ | - CH₂-CO-(4-Me-Ph) | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 1/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-2, 2-9, 2-10, 2-11 und 3-238 bei einer Aufwandmenge von 80 g/ha jeweils eine mindestens 90%-ige Wirkung gegen Amaranthus retroflexus und Veronica Persica.
Die Verbindungen Nr. 1-237, 1-238, 2-9, 2-10, 2-237 und 2-238 zeigen bei einer Aufwandmenge von 80 g/ha jeweils eine mindestens 90%-ige Wirkung gegen Abutilon theophrasti, Amaranthus retroflexus und Echinochloa crus galli. Die Verbindungen Nr. 1-10, 2-9 und 2-238 zeigen bei einer Aufwandmenge von 80 g/ha jeweils eine mindestens 90%-ige Wirkung gegen Abutilon theophrasti und Matricaria inodora.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Dabei zeigen beispielsweise die Verbindungen Nr. 1-10, 1-237 und 2-9 bei einer Aufwandmenge von 80 g/ha jeweils eine mindestens 90%-ige Wirkung gegen Stellaria media und Veronica Persica. Die Verbindungen Nr. 1-238, 2-10 und 2-238 zeigen bei einer Aufwandmenge von 80 g/ha jeweils eine mindestens 90%-ige Wirkung gegen Setaria viridis und Viola tricolor. Die Verbindungen Nr. 1-2, 1-237, 1-238 und 2-11 zeigen bei einer Aufwandmenge von 80 g/ha jeweils eine mindestens 90%-ige Wirkung gegen Abutilon theophrasti und Echinochloa crus galli.

## Patentansprüche

1. 4-(3-Alkylsulfinylbenzoyl)pyrazole der Formel (I) oder deren Salze 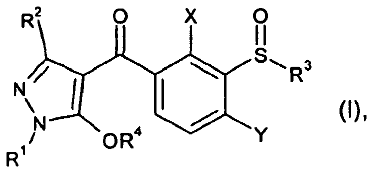 worin
R¹ bedeutet (C₁-C₄)-Alkyl,
R² bedeutet Wasserstoff oder (C₁-C₄)-Alkyl,
R³ bedeutet (C₁-C₆)-Alkyl,
R⁴ bedeutet Wasserstoff, (C₁-C₆)-Alkylsulfonyl, (C₁-C₄)-Alkoxy-(C₁-C₆)-alkylsulfonyl, oder jeweils durch m gleiche oder verschiedene Reste aus der Gruppe bestehend aus Halogen, (C₁-C₄)-Alkyl und (C₁-C₄)-Alkoxy substituiertes Phenylsulfonyl, Thiophenyl-2-sulfonyl, Benzoyl, Benzoyl-(C₁-C₆)-alkyl oder Benzyl,
X bedeutet Wasserstoff, Mercapto, Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, OR⁵, OCOR⁵, OSO₂R⁵, S(O)ₙR⁵, SO₂OR⁵, SO₂N(R⁵)₂, NR⁵SO₂R⁵, NR⁵COR⁵, (C₁-C₆)-Alkyl-S(O)ₙR⁵, (C₁-C₆)-Alkyl-OR⁵, (C₁-C₆)-Alkyl-OCOR⁵, (C₁-C₆)-Alkyl-OSO2R⁵, (C₁-C₆)-Alkyl-SO₂OR⁵, (C₁-C₆)-Alkyl-SO₂N(R⁵₎₂ oder (C₁-C₆)-Alkyl-NR⁵COR⁵;
Y bedeutet Fluor, Chlor, Brom, Iod, Nitro oder die Gruppe SO₂R⁷,
R⁵ bedeutet Wasserstoff, (C₁-C₆)-Alkyl, (C₂-C₆)-Alkenyl, (C₂-C₆)-Alkinyl, (C₃-C₆)-Cycloalkyl, Phenyl oder Phenyl-(C₁-C₆)-alkyl, wobei die sechs letztgenannten Reste durch s Reste der Gruppe Hydroxy, Mercapto, Amino, Cyano, Nitro, Rhodano, OR⁶, SR⁶, N(R⁶)₂, NOR⁶, OCOR⁶, SCOR⁶, NR⁶COR⁶, CO₂R⁶, COSR⁶, CON(R⁶)₂, (C₁)-C₄-Alkyliminooxy, (C₁-C₄)-Alkylcarbonyl, (C₁-C₄)-Alkoxy-(C₂-C₆)-alkoxycarbonyl und (C₁-C₄)-Alkylsulfonyl substituiert sind;
R⁶ bedeutet Wasserstoff, (C₁-C₆)-Alkyl, (C₂-C₆)-Alkenyl oder (C₂-C₆)-Alkinyl, R⁷ bedeutet (C₁-C₄)-Alkyl,
m bedeutet 0, 1, 2, 3, 4 oder 5,
n bedeutet 0, 1 oder 2,
s bedeutet 0, 1, 2 oder 3.

2. 4-(3-Alkylsulfinylbenzoyl)pyrazole nach Anspruch 1, worin
R¹ bedeutet Methyl, Ethyl, n-Propyl oder i-Propyl,
R² bedeutet Wasserstoff, Methyl, Ethyl, n-Propyl oder i-Propyl,
R³ bedeutet (C₁-C₄)-Alkyl,
R⁴ bedeutet Wasserstoff, (C₁-C₃)-Alkylsulfonyl, (C₁-C₂)-Alkoxy-(C₁-C₄)-alkylsulfonyl, oder jeweils durch m Methylgruppen substituiertes Phenylsulfonyl, Thiophenyl-2-sulfonyl, Benzoyl, Benzoyl-(C₁-C₆)-alkyl oder Benzyl,
X bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsuffonylmethyl,
Y bedeutet Fluor, Chlor, Brom, Iod oder die Gruppe SO₂R⁷,
R⁷ bedeutet Methyl, Ethyl, n-Propyl oder i-Propyl,
m bedeutet 0, 1, 2 oder 3.

3. 4-(3-Alkylsulfinylbenzoyl)pyrazole nach Anspruch 1 oder 2, worin
R¹ bedeutet Methyl oder Ethyl,
R² bedeutet Wasserstoff, Methyl oder Ethyl,
R³ bedeutet Methyl, Ethyl, n-Propyl oder i-Propyl,
R⁴ bedeutet Wasserstoff,
X bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Fluor, Chlor, Brom, Iod oder die Gruppe SO₂R⁷,
R⁷ bedeutet Methyl oder Ethyl.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach Anspruch 4 oder 5 auf die Pflanzen oder auf den Ort des unerwünschten Pflanzenwachstums appliziert.

7. Verwendung von Verbindungen der Formel (I) gemäß einem der Ansprüche 1 bis 3 oder von herbiziden Mitteln nach Anspruch 4 oder 5 zur Bekämpfung unerwünschter Pflanzen.

8. Verwendung nach Anspruch 7, **dadurch gekennzeichnet, daß** die Verbindungen der Formel (I) zur Bekämpfung unerwünschter Pflanzen in Kulturen von Nutzpflanzen eingesetzt werden.

9. Verwendung nach Anspruch 8, **dadurch gekennzeichnet, daß** die Nutzpflanzen transgene Nutzpflanzen sind.

10. Verbindungen der Formel (II) 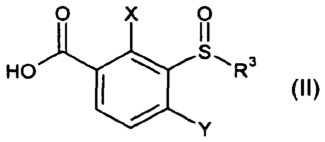 worin R³, X und Y wie in einem der Ansprüche 1 bis 3 definiert sind.

## Claims

1. A 4-(3-alkylsulfinylbenzoyl)pyrazole of the formula (I) or a salt thereof 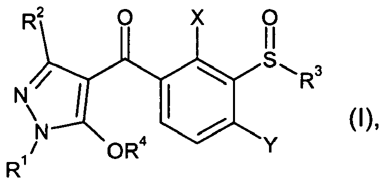 in which
R¹ is (C₁-C₄)-alkyl,
R² is hydrogen or (C₁-C₄)-alkyl,
R³ is (C₁-C₆)-alkyl,
R⁴ is hydrogen, (C₁-C₆)-alkylsulfonyl, (C₁-C₄)-alkoxy-(C₁-C₆)-alkylsulfonyl, or is phenylsulfonyl, thiophenyl-2-sulfonyl, benzoyl, benzoyl-(C₁-C₆)-alkyl or benzyl, each of which is substituted by m identical or different radicals from the group consisting of halogen, (C₁-C₄)-alkyl and (C₁-C₄)-alkoxy,
X is hydrogen, mercapto, nitro, halogen, cyano, thiocyanato, (C₁-C₆)-alkyl, (C₁-C₆)-haloalkyl, (C₂-C₆)-alkenyl, (C₂-C₆) -haloalkenyl, (C₂-C₆)-alkynyl, (C₃-C₆)-haloalkynyl, (C₃-C₆) -cycloalkyl, OR⁵, OCOR⁵, OSO₂R⁵, S(O)ₙR⁵, SO₂OR⁵, SO₂N(R⁵)₂, NR⁵SO₂R⁵, NR⁵COR⁵, (C₁-C6)-alkyl-S(O)ₙR⁵, (C₁-C₆)-alkyl-OR⁵, (C₁-C₆)-alkyl-OCOR⁵, (C₁-C₆)-alkyl-OSO2R⁵, (C₁-C₆) -alkyl-SO₂OR⁵, (C₁-C₆) -alkyl-SO₂N(R⁵)₂ or (C₁-C₆)-alkyl-NR⁵COR⁵;
Y is fluorine, chlorine, bromine, iodine, nitro or the group SO₂R⁷,
R⁵ is hydrogen, (C₁-C₆)-alkyl, (C₂-C₆)-alkenyl, (C₂-C₆)-alkynyl, (C₃-C₆)-cycloalkyl, phenyl or phenyl-(C₁-C₆)-alkyl, where the six last-mentioned radicals are substituted by s radicals from the group consisting of hydroxy, mercapto, amino, cyano, nitro, thiocyanato, OR⁶, SR⁶, N(R⁶)₂, NOR⁶, OCOR⁶, SCOR⁶, NR⁶COR⁶, CO₂R⁶, COSR⁶, CON(R⁶)₂, (C₁) -C₄-alkyliminooxy, (C₁-C₄) - alkylcarbonyl, (C₁-C₄)-alkoxy-(C₂-C₆)-alkoxycarbonyl and (C₁-C₄)-alkylsulfonyl;
R⁶ is hydrogen, (C₁-C₆)-alkyl, (C₂-C₆) -alkenyl or (C₂-C₆)-alkynyl,
R⁷ is (C₁-C₄)-alkyl,
m is 0, 1, 2, 3, 4 or 5,
n is 0, 1 or 2,
s is 0, 1, 2 or 3.

2. The 4-(3-alkylsulfinylbenzoyl)pyrazole as claimed in claim 1 in which
R¹ is methyl, ethyl, n-propyl or isopropyl,
R² is hydrogen, methyl, ethyl, n-propyl or isopropyl, R³ is (C₁-C₄)-alkyl,
R⁴ is hydrogen, (C₁-C₃)-alkylsulfonyl, (C₁-C₂)-alkoxy-(C₁-C₄)-alkylsulfonyl, or is phenylsulfonyl, thiophenyl-2-sulfonyl, benzoyl, benzoyl-(C₁-C₆)-alkyl or benzyl, each of which is substituted by m methyl groups,
X is nitro, halogen, (C₁-C₄)-alkyl, trifluoromethyl, (C₁-C₄)-alkoxy, methylsulfonyl, methoxymethyl, methoxymethoxymethyl, ethoxyethoxymethyl, ethoxymethoxymethyl, methoxyethoxymethyl, methoxypropoxymethyl, methylsulfonylmethyl, methylsulfonylethoxymethyl, methoxyethylsulfonylmethyl, methylsulfonylethylsulfonylmethyl,
Y is fluorine, chlorine, bromine, iodine or the group SO₂R⁷_{,}
R⁷ is methyl, ethyl, n-propyl or isopropyl,
m is 0, 1, 2 or 3.

3. The 4-(3-alkylsulfinylbenzoyl)pyrazole as claimed in claim 1 or 2 in which
R¹ is methyl or ethyl,
R² is hydrogen, methyl or ethyl,
R³ is methyl, ethyl, n-propyl or isopropyl,
R⁴ is hydrogen,
X is nitro, halogen, (C₁-C₄)-alkyl, trifluoromethyl, (C₁-C₄)-alkoxy, methylsulfonyl, methoxymethyl, methoxymethoxymethyl, ethoxyethoxymethyl, ethoxymethoxymethyl, methoxyethoxymethyl, methoxypropoxymethyl, methylsulfonylmethyl, methylsulfonylethoxymethyl, methoxyethylsulfonylmethyl, methylsulfonylethylsulfonylmethyl,
Y is fluorine, chlorine, bromine, iodine or the group SO₂R⁷,
R⁷ is methyl or ethyl.

4. A herbicidal composition which comprises a herbicidally active amount of at least one compound of the formula (I) as claimed in any of claims 1 to 3.

5. The herbicidal composition as claimed in claim 4 in a mixture with formulation auxiliaries.

6. A method of controlling unwanted plants, which comprises applying an effective amount of at least one compound of the formula (I) as claimed in any of claims 1 to 3 or of a herbicidal composition as claimed in claim 4 or 5 to the plants or to the location of the unwanted plant growth.

7. The use of a compound of the formula (I) as claimed in any of claims 1 to 3 or of a herbicidal composition as claimed in claim 4 or 5 for controlling unwanted plants.

8. The use as claimed in claim 7, wherein the compounds of the formula (I) are employed for controlling unwanted plants in crops of useful plants.

9. The use as claimed in claim 8, wherein the useful plants are transgenic useful plants.

10. A compound of the formula (II) 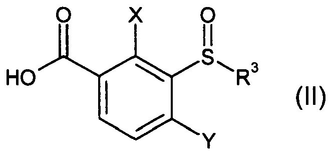 in which R³, X and Y are as defined in any of claims 1 to 3.

## Revendications

1. 4-(3-alkylsulfinylbenzoyl)pyrazoles de formule (I) ou leurs sels 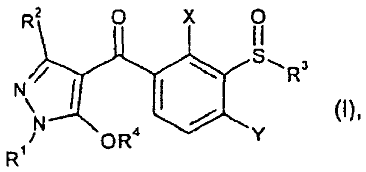 où
R¹ signifie (C₁-C₄)-alkyle,
R² signifie hydrogène ou (C₁-C₄)-alkyle,
R³ signifie (C₁-C₆)-alkyle,
R⁴ signifie hydrogène, (C₁-C₆)-alkylsulfonyle, (C₁-C₄)-alcoxy-(C₁-C₆)-alkylsulfonyle ; ou phénylsulfonyle, thiophényl-2-sulfonyle, benzoyle, benzoyl-(C₁-C₆)-alkyle ou benzyle à chaque fois substitué par m radicaux identiques ou différents du groupe constitué par halogène, (C₁-C₄)-alkyle et (C₁-C₄) - alcoxy,
X signifie hydrogène, mercapto, nitro, halogène, cyano, thiocyano, (C₁-C₆)-alkyle, (C₁-C₆)-halogénoalkyle, (C₂-C₆)-alcényle, (C₂-C₆)-halogénoalcényle, (C₂-C₆)-alcynyle, (C₃-C₆)-halogénoalcynyle, (C₃-C₆) -cycloalkyle, OR⁵, OCOR⁵, OSO₂R⁵, S(O)ₙR⁵, SO₂OR⁵, SO2N(R⁵)₂, NR⁵SO₂R⁵, NR⁵COR⁵, (C₁-C₆) -alkyl-S(O)ₙR⁵, (C₁-C₆) -alkyl-OR⁵, (C₁-C₆)-alkyl-OCOR⁵, (C₁-C₆) -alkyl-OSO₂R⁵, (C₁-C₆) -alkyl-SO₂OR⁵, (C₁-C₆) -alkyl-SO₂N(R⁵)₂ ou (C₁-C₆) -alkyl-NR⁵COR⁵;
Y signifie fluor, chlore, brome, iode, nitro ou le groupe SO₂R⁷,
R⁵ signifie hydrogène, (C₁-C₆)-alkyle, (C₂-C₆)-alcényle, (C₂-C₆)-alcynyle, (C₃-C₆) -cycloalkyle, phényle ou phényl- (C₁-C₆) -alkyle, les six derniers radicaux mentionnés étant substitués par s radicaux du groupe hydroxy, mercapto, amino, cyano, nitro, thiocyano, OR⁶, SR⁶, N(R⁶)₂, NOR⁶, OCOR⁶, SCOR⁶, NR⁶COR⁶, CO₂R⁶, COSR⁶, CON(R⁶)₂, (C₁-C₄)-alkyliminooxy, (C₁-C₄)-alkylcarbonyle, (C₁-C₄)-alcoxy- (C₂-C₆) -alcoxycarbonyle et (C₁-C₄) - alkylsulfonyle ;
R⁶ signifie hydrogène, (C₁-C₆)-alkyle, (C₂-C₆)-alcényle ou (C₂-C₆)-alcynyle,
R⁷ signifie (C₁-C₄)-alkyle,
m vaut 0, 1, 2, 3, 4 ou 5,
n vaut 0, 1 ou 2,
s vaut 0, 1, 2 ou 3.

2. 4-(3-alkylsulfinylbenzoyl)pyrazoles selon la revendication 1, où
R¹ signifie méthyle, éthyle, n-propyle ou i-propyle,
R² signifie hydrogène, méthyle, éthyle, n-propyle ou i-propyle,
R³ signifie (C₁-C₄) -alkyle,
R⁴ signifie hydrogène, (C₁-C₃)-alkylsulfonyle, (C₁-C₂)-alcoxy-(C₁-C₄)-alkylsulfonyle ; ou phénylsulfonyle, thiophényl-2-sulfonyle, benzoyle, benzoyl-(C₁-C₆)-alkyle ou benzyle à chaque fois substitué par m groupes méthyle,
X signifie nitro, halogène, (C₁-C₄) -alkyle, trifluorométhyle, (C₁-C₄)-alcoxy, méthylsulfonyle, méthoxyméthyle, méthoxyméthoxyméthyle, éthoxyéthoxyméthyle, éthoxyméthoxyméthyle, méthoxyéthoxyméthyle, méthoxypropoxyméthyle, méthylsulfonylméthyle, méthylsulfonyléthoxyméthyle, méthoxyéthylsulfonylméthyle, méthylsulfonyléthylsulfonylméthyle,
Y signifie fluor, chlore, brome, iode ou le groupe SO₂R⁷,
R⁷ signifie méthyle, éthyle, n-propyle ou i-propyle,
m vaut 0, 1, 2 ou 3.

3. 4-(3-alkylsulfinylbenzoyl)pyrazoles selon la revendication 1 ou 2, où
R¹ signifie méthyle ou éthyle,
R² signifie hydrogène, méthyle ou éthyle,
R³ signifie méthyle, éthyle, n-propyle ou i-propyle,
R⁴ signifie hydrogène,
X signifie nitro, halogène, (C₁-C₄)-alkyle, trifluorométhyle, (C₁-C₄)-alcoxy, méthylsulfonyle, méthoxyméthyle, méthoxyméthoxyméthyle, éthoxyéthoxyméthyle, éthoxyméthoxyméthyle, méthoxyéthoxyméthyle, méthoxypropoxyméthyle, méthylsulfonylméthyle, méthylsulfonyléthoxyméthyle, méthoxyéthylsulfonylméthyle, méthylsulfonyléthylsulfonylméthyle,
Y signifie fluor, chlore, brome, iode ou le groupe SO₂R⁷,
R⁷ signifie méthyle ou éthyle.

4. Agents herbicides, **caractérisés par** une teneur active en tant qu'herbicide en au moins un composé de formule (I) selon l'une quelconque des revendications 1 à 3.

5. Agents herbicides selon la revendication 4 en mélange avec des adjuvants de formulation.

6. Procédé pour lutter contre des plantes non souhaitées, **caractérisé en ce qu'**on applique une quantité active d'au moins un composé de formule (I) selon l'une quelconque des revendications 1 à 3 ou d'un agent herbicide selon la revendication 4 ou 5 sur les plantes ou sur le lieu de la croissance non souhaitée des plantes.

7. Utilisation de composés de formule (I) selon l'une quelconque des revendications 1 à 3 ou d'agents herbicides selon la revendication 4 ou 5 pour lutter contre des plantes non souhaitées.

8. Utilisation selon la revendication 7, **caractérisée en ce que** les composés de formule (I) sont utilisés pour lutter contre des plantes non souhaitées dans les cultures de plantes utiles.

9. Utilisation selon la revendication 8, **caractérisée en ce que** les plantes utiles sont des plantes utiles transgéniques.

10. Composés de formule (II) 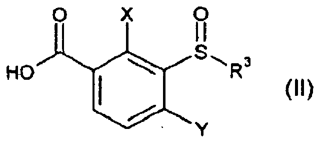 R³, X et Y étant définis comme dans l'une quelconque des revendications 1 à 3.
